(19) Europäisches Patentamt
European Patent Office
Office européen des brevets

(11) EP 3 792 365 A1

(12) EUROPEAN PATENT APPLICATION

(43) Date of publication:
17.03.2021 Bulletin 2021/11

(51) Int Cl.:
*C12Q 1/68* (2018.01) *C12Q 1/6827* (2018.01)

(21) Application number: 20203880.8

(22) Date of filing: 30.06.2017

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 01.07.2016 US 201662357847 P

(62) Document number(s) of the earlier application(s) in accordance with Art. 76 EPC:
17821368.2 / 3 510 171

(71) Applicant: Natera, Inc.
San Carlos, CA 94070 (US)

(72) Inventors:
- ZIMMERMAN, Bernhard
San Carlos, CA 94070 (US)
- BABIARZ, Joshua
San Carlos, CA 94070 (US)
- SALARI, Raheleh
San Carlos, CA 94070 (US)
- CONSTANTIN, Tudor Pompiliu
San Carlos, CA 94070 (US)
- SAKARYA, Onur
San Carlos, CA 94070 (US)
- PROSEN, Dennis
San Carlos, CA 94070 (US)
- DASHMER, Scott
San Carlos, CA 94070 (US)
- SERGEEV, Nikolay
San Carlos, CA 94070 (US)
- HILL, Matthew Micah
San Carlos, CA 94070 (US)

(74) Representative: Gibbs, Richard
Marks & Clerk LLP
Aurora
120 Bothwell Street
Glasgow G2 7JS (GB)

Remarks:
This application was filed on 26-10-2020 as a divisional application to the application mentioned under INID code 62.

# (54) COMPOSITIONS AND METHODS FOR DETECTION OF NUCLEIC ACID MUTATIONS

(57) The invention provides methods and compositions for detecting a mutation in a target gene in a sample or a fraction thereof, including in certain examples, a fraction that includes circulating tumor DNA. The methods can include a tiling PCR reaction, for example a one-sided multiplex tiling reaction. Virtually any type of mutation can be detected with the methods and compositions. In certain embodiments, gene fusions are detected. Improved PCR methods, especially for performing nested multiplex PCR reactions are provided.

EP 3 792 365 A1

SEQUENCE LISTING

<110> NATERA, INC.

<120> COMPOSITIONS AND METHODS FOR DETECTION OF NUCLEIC ACID MUTATIONS

<130> PG448807EPA

<140>
<141>

<150> 62/357,847
<151> 2016-07-01

<160> 323

<170> PatentIn version 3.5

<210> 1
<211> 22
<212> DNA
<213> Artificial Sequence

<220>
<221> source
<223> /note="Description of Artificial Sequence: Synthetic primer"

<400> 1
ggctagagga gtctgcggtg ct 22

<210> 2
<211> 23
<212> DNA
<213> Artificial Sequence

<220>
<221> source
<223> /note="Description of Artificial Sequence: Synthetic primer"

<400> 2
aggagtctgc ggtgctgtga taa 23

<210> 3
<211> 22
<212> DNA
<213> Artificial Sequence

<220>
<221> source
<223> /note="Description of Artificial Sequence: Synthetic primer"

<400> 3
gcggtgctgt gataacattc ag 22

<210> 4
<211> 26
<212> DNA

<213> Artificial Sequence

<220>
<221> source
<223> /note="Description of Artificial Sequence: Synthetic primer"

<400> 4
tgtgataaca ttcagcccct acactg 26

<210> 5
<211> 24
<212> DNA
<213> Artificial Sequence

<220>
<221> source
<223> /note="Description of Artificial Sequence: Synthetic primer"

<400> 5
ccgaatgagg gtgatgtttt tccg 24

<210> 6
<211> 20
<212> DNA
<213> Artificial Sequence

<220>
<221> source
<223> /note="Description of Artificial Sequence: Synthetic primer"

<400> 6
tttttccgcg gcacctcctt 20

<210> 7
<211> 21
<212> DNA
<213> Artificial Sequence

<220>
<221> source
<223> /note="Description of Artificial Sequence: Synthetic primer"

<400> 7
caggtcactg atggaggagg t 21

<210> 8
<211> 21
<212> DNA
<213> Artificial Sequence

<220>
<221> source
<223> /note="Description of Artificial Sequence: Synthetic primer"

<400> 8
ctgatggagg aggtcttgcc a 21

<210> 9
<211> 22
<212> DNA
<213> Artificial Sequence

<220>
<221> source
<223> /note="Description of Artificial Sequence: Synthetic primer"

<400> 9
gaggaggtct tgccagcaaa gc 22

<210> 10
<211> 24
<212> DNA
<213> Artificial Sequence

<220>
<221> source
<223> /note="Description of Artificial Sequence: Synthetic primer"

<400> 10
tcttgccagc aaagcagtag ttgg 24

<210> 11
<211> 23
<212> DNA
<213> Artificial Sequence

<220>
<221> source
<223> /note="Description of Artificial Sequence: Synthetic primer"

<400> 11
ttggggttgt agtcggtcat gat 23

<210> 12
<211> 24
<212> DNA
<213> Artificial Sequence

<220>
<221> source
<223> /note="Description of Artificial Sequence: Synthetic primer"

<400> 12
ttgtagtcgg tcatgatggt cgag 24

<210> 13
<211> 21
<212> DNA

<213> Artificial Sequence

<220>
<221> source
<223> /note="Description of Artificial Sequence: Synthetic primer"

<400> 13
cagctccatc tgcatggctt g 21

<210> 14
<211> 21
<212> DNA
<213> Artificial Sequence

<220>
<221> source
<223> /note="Description of Artificial Sequence: Synthetic primer"

<400> 14
catggcttgc agctcctggt g 21

<210> 15
<211> 20
<212> DNA
<213> Artificial Sequence

<220>
<221> source
<223> /note="Description of Artificial Sequence: Synthetic primer"

<400> 15
catggcttgc agctcctggt 20

<210> 16
<211> 19
<212> DNA
<213> Artificial Sequence

<220>
<221> source
<223> /note="Description of Artificial Sequence: Synthetic primer"

<400> 16
cctggtgctt ccggcggta 19

<210> 17
<211> 19
<212> DNA
<213> Artificial Sequence

<220>
<221> source
<223> /note="Description of Artificial Sequence: Synthetic primer"

<400> 17
ccggcggtac actgcaggt 19

<210> 18
<211> 22
<212> DNA
<213> Artificial Sequence

<220>
<221> source
<223> /note="Description of Artificial Sequence: Synthetic primer"

<400> 18
gtacactgca ggtgggtggt ca 22

<210> 19
<211> 19
<212> DNA
<213> Artificial Sequence

<220>
<221> source
<223> /note="Description of Artificial Sequence: Synthetic primer"

<400> 19
gggtggtcag ctgcaacat 19

<210> 20
<211> 18
<212> DNA
<213> Artificial Sequence

<220>
<221> source
<223> /note="Description of Artificial Sequence: Synthetic primer"

<400> 20
ctgcaacatg gcctggca 18

<210> 21
<211> 16
<212> DNA
<213> Artificial Sequence

<220>
<221> source
<223> /note="Description of Artificial Sequence: Synthetic primer"

<400> 21
ggcagcctgg cccttg 16

<210> 22
<211> 23
<212> DNA

<213> Artificial Sequence

<220>
<221> source
<223> /note="Description of Artificial Sequence: Synthetic primer"

<400> 22
tggcccttga agcactacac agg 23

<210> 23
<211> 21
<212> DNA
<213> Artificial Sequence

<220>
<221> source
<223> /note="Description of Artificial Sequence: Synthetic primer"

<400> 23
aagcactaca caggccactt c 21

<210> 24
<211> 21
<212> DNA
<213> Artificial Sequence

<220>
<221> source
<223> /note="Description of Artificial Sequence: Synthetic primer"

<400> 24
acacaggcca cttcctacag g 21

<210> 25
<211> 24
<212> DNA
<213> Artificial Sequence

<220>
<221> source
<223> /note="Description of Artificial Sequence: Synthetic primer"

<400> 25
tcctacagga agcctccctg gatc 24

<210> 26
<211> 25
<212> DNA
<213> Artificial Sequence

<220>
<221> source
<223> /note="Description of Artificial Sequence: Synthetic primer"

<400> 26
aggaagcctc cctggatctc catat 25

<210> 27
<211> 25
<212> DNA
<213> Artificial Sequence

<220>
<221> source
<223> /note="Description of Artificial Sequence: Synthetic primer"

<400> 27
atatcctccc ctgagctctg aacct 25

<210> 28
<211> 24
<212> DNA
<213> Artificial Sequence

<220>
<221> source
<223> /note="Description of Artificial Sequence: Synthetic primer"

<400> 28
cccctgagct ctgaaccttt ccat 24

<210> 29
<211> 32
<212> DNA
<213> Artificial Sequence

<220>
<221> source
<223> /note="Description of Artificial Sequence: Synthetic primer"

<400> 29
tttgtattat atagggcaga gtcatgttag tc 32

<210> 30
<211> 26
<212> DNA
<213> Artificial Sequence

<220>
<221> source
<223> /note="Description of Artificial Sequence: Synthetic primer"

<400> 30
tagggcagag tcatgttagt ctggtt 26

<210> 31
<211> 26
<212> DNA

<213> Artificial Sequence

<220>
<221> source
<223> /note="Description of Artificial Sequence: Synthetic primer"

<400> 31
ggttcctcca agaagcagac tggaga 26

<210> 32
<211> 24
<212> DNA
<213> Artificial Sequence

<220>
<221> source
<223> /note="Description of Artificial Sequence: Synthetic primer"

<400> 32
ccaagaagca gactggagat ggga 24

<210> 33
<211> 23
<212> DNA
<213> Artificial Sequence

<220>
<221> source
<223> /note="Description of Artificial Sequence: Synthetic primer"

<400> 33
aagaaatgcc catgagagga aat 23

<210> 34
<211> 22
<212> DNA
<213> Artificial Sequence

<220>
<221> source
<223> /note="Description of Artificial Sequence: Synthetic primer"

<400> 34
agaggaaatg gggagggagc ca 22

<210> 35
<211> 18
<212> DNA
<213> Artificial Sequence

<220>
<221> source
<223> /note="Description of Artificial Sequence: Synthetic primer"

<400> 35
ggagggagcc agggaagg 18

<210> 36
<211> 21
<212> DNA
<213> Artificial Sequence

<220>
<221> source
<223> /note="Description of Artificial Sequence: Synthetic primer"

<400> 36
ccagggaagg ctgggtgaac c 21

<210> 37
<211> 20
<212> DNA
<213> Artificial Sequence

<220>
<221> source
<223> /note="Description of Artificial Sequence: Synthetic primer"

<400> 37
tgggtgaacc agcagactgt 20

<210> 38
<211> 22
<212> DNA
<213> Artificial Sequence

<220>
<221> source
<223> /note="Description of Artificial Sequence: Synthetic primer"

<400> 38
aaccagcaga ctgtgttgca ag 22

<210> 39
<211> 19
<212> DNA
<213> Artificial Sequence

<220>
<221> source
<223> /note="Description of Artificial Sequence: Synthetic primer"

<400> 39
ggagggaggg aaggttggg 19

<210> 40
<211> 21
<212> DNA

<213> Artificial Sequence

<220>
<221> source
<223> /note="Description of Artificial Sequence: Synthetic primer"

<400> 40
gggaaggttg ggtggaagca c 21

<210> 41
<211> 17
<212> DNA
<213> Artificial Sequence

<220>
<221> source
<223> /note="Description of Artificial Sequence: Synthetic primer"

<400> 41
caccctgggt gccatgg 17

<210> 42
<211> 19
<212> DNA
<213> Artificial Sequence

<220>
<221> source
<223> /note="Description of Artificial Sequence: Synthetic primer"

<400> 42
gggtgccatg gagcctaag 19

<210> 43
<211> 24
<212> DNA
<213> Artificial Sequence

<220>
<221> source
<223> /note="Description of Artificial Sequence: Synthetic primer"

<400> 43
gggttctgga gccaaagtca gtca 24

<210> 44
<211> 26
<212> DNA
<213> Artificial Sequence

<220>
<221> source
<223> /note="Description of Artificial Sequence: Synthetic primer"

<400> 44
agccaaagtc agtcatcaga ggagtc 26

<210> 45
<211> 20
<212> DNA
<213> Artificial Sequence

<220>
<221> source
<223> /note="Description of Artificial Sequence: Synthetic primer"

<400> 45
tgtgctcagc cattgggtag 20

<210> 46
<211> 21
<212> DNA
<213> Artificial Sequence

<220>
<221> source
<223> /note="Description of Artificial Sequence: Synthetic primer"

<400> 46
agccattggg tagggcagct t 21

<210> 47
<211> 22
<212> DNA
<213> Artificial Sequence

<220>
<221> source
<223> /note="Description of Artificial Sequence: Synthetic primer"

<400> 47
tagggcagct tcagtgcaat ca 22

<210> 48
<211> 24
<212> DNA
<213> Artificial Sequence

<220>
<221> source
<223> /note="Description of Artificial Sequence: Synthetic primer"

<400> 48
agtgcaatca cagcagtgga tttg 24

<210> 49
<211> 26
<212> DNA

<213> Artificial Sequence

<220>
<221> source
<223> /note="Description of Artificial Sequence: Synthetic primer"

<400> 49
gtgcaatcac agcagtggat ttgagg 26

<210> 50
<211> 20
<212> DNA
<213> Artificial Sequence

<220>
<221> source
<223> /note="Description of Artificial Sequence: Synthetic primer"

<400> 50
gtggatttga gggtgcagct 20

<210> 51
<211> 29
<212> DNA
<213> Artificial Sequence

<220>
<221> source
<223> /note="Description of Artificial Sequence: Synthetic primer"

<400> 51
gatcttggtc agttgtgttt cctatagtt 29

<210> 52
<211> 29
<212> DNA
<213> Artificial Sequence

<220>
<221> source
<223> /note="Description of Artificial Sequence: Synthetic primer"

<400> 52
ggtcagttgt gtttcctata gttggagaa 29

<210> 53
<211> 19
<212> DNA
<213> Artificial Sequence

<220>
<221> source
<223> /note="Description of Artificial Sequence: Synthetic primer"

<400> 53
ggagaactgc caagccaca 19

<210> 54
<211> 19
<212> DNA
<213> Artificial Sequence

<220>
<221> source
<223> /note="Description of Artificial Sequence: Synthetic primer"

<400> 54
gccaagccac agagttgga 19

<210> 55
<211> 24
<212> DNA
<213> Artificial Sequence

<220>
<221> source
<223> /note="Description of Artificial Sequence: Synthetic primer"

<400> 55
tctgaattgg tgtctgggga tctg 24

<210> 56
<211> 22
<212> DNA
<213> Artificial Sequence

<220>
<221> source
<223> /note="Description of Artificial Sequence: Synthetic primer"

<400> 56
tggtgtctgg ggatctgtgc tc 22

<210> 57
<211> 20
<212> DNA
<213> Artificial Sequence

<220>
<221> source
<223> /note="Description of Artificial Sequence: Synthetic primer"

<400> 57
aagctgagcc atgaggacca 20

<210> 58
<211> 21
<212> DNA

<213> Artificial Sequence

<220>
<221> source
<223> /note="Description of Artificial Sequence: Synthetic primer"

<400> 58
agccatgagg accaggtcac a 21

<210> 59
<211> 21
<212> DNA
<213> Artificial Sequence

<220>
<221> source
<223> /note="Description of Artificial Sequence: Synthetic primer"

<400> 59
aggacctctt tggactgcag t 21

<210> 60
<211> 23
<212> DNA
<213> Artificial Sequence

<220>
<221> source
<223> /note="Description of Artificial Sequence: Synthetic primer"

<400> 60
ctttggactg cagtttccct ctc 23

<210> 61
<211> 22
<212> DNA
<213> Artificial Sequence

<220>
<221> source
<223> /note="Description of Artificial Sequence: Synthetic primer"

<400> 61
taggcaggga tggtaactcc tg 22

<210> 62
<211> 23
<212> DNA
<213> Artificial Sequence

<220>
<221> source
<223> /note="Description of Artificial Sequence: Synthetic primer"

<400> 62
gggatggtaa ctcctgccct gtt 23

<210> 63
<211> 18
<212> DNA
<213> Artificial Sequence

<220>
<221> source
<223> /note="Description of Artificial Sequence: Synthetic primer"

<400> 63
gccactcccc accctcta 18

<210> 64
<211> 26
<212> DNA
<213> Artificial Sequence

<220>
<221> source
<223> /note="Description of Artificial Sequence: Synthetic primer"

<400> 64
ccctctaggg ttgtcaatga aatgaa 26

<210> 65
<211> 28
<212> DNA
<213> Artificial Sequence

<220>
<221> source
<223> /note="Description of Artificial Sequence: Synthetic primer"

<400> 65
caaaattgtg attcagtggg tagattct 28

<210> 66
<211> 28
<212> DNA
<213> Artificial Sequence

<220>
<221> source
<223> /note="Description of Artificial Sequence: Synthetic primer"

<400> 66
tgtgattcag tgggtagatt ctgtgtgt 28

<210> 67
<211> 20
<212> DNA

<213> Artificial Sequence

<220>
<221> source
<223> /note="Description of Artificial Sequence: Synthetic primer"

<400> 67
caacacatgg gccagggcaa 20

<210> 68
<211> 21
<212> DNA
<213> Artificial Sequence

<220>
<221> source
<223> /note="Description of Artificial Sequence: Synthetic primer"

<400> 68
tgggccaggg caaatgagtc a 21

<210> 69
<211> 20
<212> DNA
<213> Artificial Sequence

<220>
<221> source
<223> /note="Description of Artificial Sequence: Synthetic primer"

<400> 69
tgagtcaccc gctatgtgct 20

<210> 70
<211> 22
<212> DNA
<213> Artificial Sequence

<220>
<221> source
<223> /note="Description of Artificial Sequence: Synthetic primer"

<400> 70
acccgctatg tgctcagttc cc 22

<210> 71
<211> 24
<212> DNA
<213> Artificial Sequence

<220>
<221> source
<223> /note="Description of Artificial Sequence: Synthetic primer"

<400> 71
tccctcctct atgcaatgga ccga 24

<210> 72
<211> 25
<212> DNA
<213> Artificial Sequence

<220>
<221> source
<223> /note="Description of Artificial Sequence: Synthetic primer"

<400> 72
ctatgcaatg gaccgaccgt gatca 25

<210> 73
<211> 22
<212> DNA
<213> Artificial Sequence

<220>
<221> source
<223> /note="Description of Artificial Sequence: Synthetic primer"

<400> 73
tggaccgacc gtgatcagat ta 22

<210> 74
<211> 29
<212> DNA
<213> Artificial Sequence

<220>
<221> source
<223> /note="Description of Artificial Sequence: Synthetic primer"

<400> 74
accgtgatca gattagggtt acctgagga 29

<210> 75
<211> 26
<212> DNA
<213> Artificial Sequence

<220>
<221> source
<223> /note="Description of Artificial Sequence: Synthetic primer"

<400> 75
gtgtaaattg ccgagcacgt agtaac 26

<210> 76
<211> 22
<212> DNA

<213> Artificial Sequence

<220>
<221> source
<223> /note="Description of Artificial Sequence: Synthetic primer"

<400> 76
ccgagcacgt agtaaccatg ca 22

<210> 77
<211> 25
<212> DNA
<213> Artificial Sequence

<220>
<221> source
<223> /note="Description of Artificial Sequence: Synthetic primer"

<400> 77
acgtagtaac catgcaacaa gtgtt 25

<210> 78
<211> 31
<212> DNA
<213> Artificial Sequence

<220>
<221> source
<223> /note="Description of Artificial Sequence: Synthetic primer"

<400> 78
accatgcaac aagtgttagc tcctattatc c 31

<210> 79
<211> 20
<212> DNA
<213> Artificial Sequence

<220>
<221> source
<223> /note="Description of Artificial Sequence: Synthetic primer"

<400> 79
atggggacac agtgtgtgct 20

<210> 80
<211> 22
<212> DNA
<213> Artificial Sequence

<220>
<221> source
<223> /note="Description of Artificial Sequence: Synthetic primer"

<400> 80
agtgtgtgct gccatctccc tt 22

<210> 81
<211> 21
<212> DNA
<213> Artificial Sequence

<220>
<221> source
<223> /note="Description of Artificial Sequence: Synthetic
primer"

<400> 81
gctgccatct cccttctacc g 21

<210> 82
<211> 22
<212> DNA
<213> Artificial Sequence

<220>
<221> source
<223> /note="Description of Artificial Sequence: Synthetic
primer"

<400> 82
ctcccttcta ccggcagatc cc 22

<210> 83
<211> 20
<212> DNA
<213> Artificial Sequence

<220>
<221> source
<223> /note="Description of Artificial Sequence: Synthetic
primer"

<400> 83
accggcagat ccctttgcct 20

<210> 84
<211> 17
<212> DNA
<213> Artificial Sequence

<220>
<221> source
<223> /note="Description of Artificial Sequence: Synthetic
primer"

<400> 84
aggggcctgg cctgcga 17

<210> 85
<211> 18
<212> DNA

<213> Artificial Sequence

<220>
<221> source
<223> /note="Description of Artificial Sequence: Synthetic primer"

<400> 85
gcctgcgagg gctctcaa 18

<210> 86
<211> 23
<212> DNA
<213> Artificial Sequence

<220>
<221> source
<223> /note="Description of Artificial Sequence: Synthetic primer"

<400> 86
ctctcaagag cctttccctc tgc 23

<210> 87
<211> 23
<212> DNA
<213> Artificial Sequence

<220>
<221> source
<223> /note="Description of Artificial Sequence: Synthetic primer"

<400> 87
caagagcctt tccctctgcc ctt 23

<210> 88
<211> 20
<212> DNA
<213> Artificial Sequence

<220>
<221> source
<223> /note="Description of Artificial Sequence: Synthetic primer"

<400> 88
tccctctgcc cttttcaagc 20

<210> 89
<211> 20
<212> DNA
<213> Artificial Sequence

<220>
<221> source
<223> /note="Description of Artificial Sequence: Synthetic primer"

<400> 89
agcctctgcc catctgtcct 20

<210> 90
<211> 22
<212> DNA
<213> Artificial Sequence

<220>
<221> source
<223> /note="Description of Artificial Sequence: Synthetic primer"

<400> 90
tctgtcctgg gcatgtctct gc 22

<210> 91
<211> 21
<212> DNA
<213> Artificial Sequence

<220>
<221> source
<223> /note="Description of Artificial Sequence: Synthetic primer"

<400> 91
agcagtaaga gctggttggg a 21

<210> 92
<211> 22
<212> DNA
<213> Artificial Sequence

<220>
<221> source
<223> /note="Description of Artificial Sequence: Synthetic primer"

<400> 92
agctggttgg gaccacactg ag 22

<210> 93
<211> 20
<212> DNA
<213> Artificial Sequence

<220>
<221> source
<223> /note="Description of Artificial Sequence: Synthetic primer"

<400> 93
cctgcaggtc agctcacctt 20

<210> 94
<211> 21
<212> DNA

<213> Artificial Sequence

<220>
<221> source
<223> /note="Description of Artificial Sequence: Synthetic primer"

<400> 94
tcagctcacc ttggctcaca g 21

<210> 95
<211> 26
<212> DNA
<213> Artificial Sequence

<220>
<221> source
<223> /note="Description of Artificial Sequence: Synthetic primer"

<400> 95
agaaaccgat tttcctatct ctctgc 26

<210> 96
<211> 25
<212> DNA
<213> Artificial Sequence

<220>
<221> source
<223> /note="Description of Artificial Sequence: Synthetic primer"

<400> 96
tttcctatct ctctgcctgg agggt 25

<210> 97
<211> 19
<212> DNA
<213> Artificial Sequence

<220>
<221> source
<223> /note="Description of Artificial Sequence: Synthetic primer"

<400> 97
tggtggaggg ctggtttgg 19

<210> 98
<211> 21
<212> DNA
<213> Artificial Sequence

<220>
<221> source
<223> /note="Description of Artificial Sequence: Synthetic primer"

<400> 98
gggctggttt ggggaagagt g 21

<210> 99
<211> 23
<212> DNA
<213> Artificial Sequence

<220>
<221> source
<223> /note="Description of Artificial Sequence: Synthetic primer"

<400> 99
tggtttgggg aagagtgggc tag 23

<210> 100
<211> 24
<212> DNA
<213> Artificial Sequence

<220>
<221> source
<223> /note="Description of Artificial Sequence: Synthetic primer"

<400> 100
ggggaagagt gggctagtgc atta 24

<210> 101
<211> 25
<212> DNA
<213> Artificial Sequence

<220>
<221> source
<223> /note="Description of Artificial Sequence: Synthetic primer"

<400> 101
agtgggctag tgcattacat agggt 25

<210> 102
<211> 27
<212> DNA
<213> Artificial Sequence

<220>
<221> source
<223> /note="Description of Artificial Sequence: Synthetic primer"

<400> 102
tagtgcatta catagggtgg gagccaa 27

<210> 103
<211> 19
<212> DNA

<213> Artificial Sequence

<220>
<221> source
<223> /note="Description of Artificial Sequence: Synthetic primer"

<400> 103
ctgcgccggt ggaagcatg 19

<210> 104
<211> 22
<212> DNA
<213> Artificial Sequence

<220>
<221> source
<223> /note="Description of Artificial Sequence: Synthetic primer"

<400> 104
cggtggaagc atgtgggagc ta 22

<210> 105
<211> 22
<212> DNA
<213> Artificial Sequence

<220>
<221> source
<223> /note="Description of Artificial Sequence: Synthetic primer"

<400> 105
gaagcatgtg ggagctagaa gt 22

<210> 106
<211> 25
<212> DNA
<213> Artificial Sequence

<220>
<221> source
<223> /note="Description of Artificial Sequence: Synthetic primer"

<400> 106
tgtgggagct agaagtgacg tctag 25

<210> 107
<211> 19
<212> DNA
<213> Artificial Sequence

<220>
<221> source
<223> /note="Description of Artificial Sequence: Synthetic primer"

<400> 107
ctaggggtgg gggcgagct 19

<210> 108
<211> 19
<212> DNA
<213> Artificial Sequence

<220>
<221> source
<223> /note="Description of Artificial Sequence: Synthetic primer"

<400> 108
gtgggggcga gctttcacc 19

<210> 109
<211> 22
<212> DNA
<213> Artificial Sequence

<220>
<221> source
<223> /note="Description of Artificial Sequence: Synthetic primer"

<400> 109
aaggagctcc ccaccccctg at 22

<210> 110
<211> 20
<212> DNA
<213> Artificial Sequence

<220>
<221> source
<223> /note="Description of Artificial Sequence: Synthetic primer"

<400> 110
ctccccaccc cctgatcagc 20

<210> 111
<211> 21
<212> DNA
<213> Artificial Sequence

<220>
<221> source
<223> /note="Description of Artificial Sequence: Synthetic primer"

<400> 111
ggatacacac ggggctgagg t 21

<210> 112
<211> 20
<212> DNA

<213> Artificial Sequence

<220>
<221> source
<223> /note="Description of Artificial Sequence: Synthetic primer"

<400> 112
acggggctga ggtgcagaat 20

<210> 113
<211> 23
<212> DNA
<213> Artificial Sequence

<220>
<221> source
<223> /note="Description of Artificial Sequence: Synthetic primer"

<400> 113
aggtgcagaa tcaggggctc ctc 23

<210> 114
<211> 22
<212> DNA
<213> Artificial Sequence

<220>
<221> source
<223> /note="Description of Artificial Sequence: Synthetic primer"

<400> 114
agaatcaggg gctcctcagg ga 22

<210> 115
<211> 22
<212> DNA
<213> Artificial Sequence

<220>
<221> source
<223> /note="Description of Artificial Sequence: Synthetic primer"

<400> 115
gaaggttggg agcttccgtt tt 22

<210> 116
<211> 22
<212> DNA
<213> Artificial Sequence

<220>
<221> source
<223> /note="Description of Artificial Sequence: Synthetic primer"

<400> 116
tgggagcttc cgttttggct tg 22

<210> 117
<211> 21
<212> DNA
<213> Artificial Sequence

<220>
<221> source
<223> /note="Description of Artificial Sequence: Synthetic primer"

<400> 117
ctgggctgcc ctaatcacca c 21

<210> 118
<211> 21
<212> DNA
<213> Artificial Sequence

<220>
<221> source
<223> /note="Description of Artificial Sequence: Synthetic primer"

<400> 118
ccctaatcac caccccaccc a 21

<210> 119
<211> 25
<212> DNA
<213> Artificial Sequence

<220>
<221> source
<223> /note="Description of Artificial Sequence: Synthetic primer"

<400> 119
caattccagg gactagcata acgaa 25

<210> 120
<211> 24
<212> DNA
<213> Artificial Sequence

<220>
<221> source
<223> /note="Description of Artificial Sequence: Synthetic primer"

<400> 120
agggactagc ataacgaagt gaca 24

<210> 121
<211> 25
<212> DNA

<213> Artificial Sequence

<220>
<221> source
<223> /note="Description of Artificial Sequence: Synthetic primer"

<400> 121
ggcacagcct gagacactat tcagt 25

<210> 122
<211> 26
<212> DNA
<213> Artificial Sequence

<220>
<221> source
<223> /note="Description of Artificial Sequence: Synthetic primer"

<400> 122
cctgagacac tattcagtcc tgcctt 26

<210> 123
<211> 21
<212> DNA
<213> Artificial Sequence

<220>
<221> source
<223> /note="Description of Artificial Sequence: Synthetic primer"

<400> 123
cttgggagtc cctggggctc t 21

<210> 124
<211> 19
<212> DNA
<213> Artificial Sequence

<220>
<221> source
<223> /note="Description of Artificial Sequence: Synthetic primer"

<400> 124
agtccctggg gctctgtgc 19

<210> 125
<211> 22
<212> DNA
<213> Artificial Sequence

<220>
<221> source
<223> /note="Description of Artificial Sequence: Synthetic primer"

<400> 125
atcatgatgc cggagaaagc ca 22

<210> 126
<211> 21
<212> DNA
<213> Artificial Sequence

<220>
<221> source
<223> /note="Description of Artificial Sequence: Synthetic primer"

<400> 126
tgccggagaa agccaggacc a 21

<210> 127
<211> 17
<212> DNA
<213> Artificial Sequence

<220>
<221> source
<223> /note="Description of Artificial Sequence: Synthetic primer"

<400> 127
gaccagggcg gccacga 17

<210> 128
<211> 20
<212> DNA
<213> Artificial Sequence

<220>
<221> source
<223> /note="Description of Artificial Sequence: Synthetic primer"

<400> 128
ccacgagggc agaggtcacc 20

<210> 129
<211> 19
<212> DNA
<213> Artificial Sequence

<220>
<221> source
<223> /note="Description of Artificial Sequence: Synthetic primer"

<400> 129
cgagggcaga ggtcaccac 19

<210> 130
<211> 22
<212> DNA

<213> Artificial Sequence

<220>
<221> source
<223> /note="Description of Artificial Sequence: Synthetic primer"

<400> 130
ggtcaccaca gagaggatca gc 22

<210> 131
<211> 18
<212> DNA
<213> Artificial Sequence

<220>
<221> source
<223> /note="Description of Artificial Sequence: Synthetic primer"

<400> 131
tgtggctccg gggtgggt 18

<210> 132
<211> 23
<212> DNA
<213> Artificial Sequence

<220>
<221> source
<223> /note="Description of Artificial Sequence: Synthetic primer"

<400> 132
cggggtgggt gacactggaa gac 23

<210> 133
<211> 26
<212> DNA
<213> Artificial Sequence

<220>
<221> source
<223> /note="Description of Artificial Sequence: Synthetic primer"

<400> 133
ggggtattga caaccacacc aggtct 26

<210> 134
<211> 25
<212> DNA
<213> Artificial Sequence

<220>
<221> source
<223> /note="Description of Artificial Sequence: Synthetic primer"

<400> 134
ttgacaacca caccaggtct ccttt 25

<210> 135
<211> 25
<212> DNA
<213> Artificial Sequence

<220>
<221> source
<223> /note="Description of Artificial Sequence: Synthetic primer"

<400> 135
ccacaccagg tctcctttga gttgg 25

<210> 136
<211> 24
<212> DNA
<213> Artificial Sequence

<220>
<221> source
<223> /note="Description of Artificial Sequence: Synthetic primer"

<400> 136
caggtctcct ttgagttggt ccca 24

<210> 137
<211> 24
<212> DNA
<213> Artificial Sequence

<220>
<221> source
<223> /note="Description of Artificial Sequence: Synthetic primer"

<400> 137
aaataacctc ccccactgag acaa 24

<210> 138
<211> 25
<212> DNA
<213> Artificial Sequence

<220>
<221> source
<223> /note="Description of Artificial Sequence: Synthetic primer"

<400> 138
cctcccccac tgagacaaaa actac 25

<210> 139
<211> 28
<212> DNA

<213> Artificial Sequence

<220>
<221> source
<223> /note="Description of Artificial Sequence: Synthetic primer"

<400> 139
ccactgagac aaaaactact tgctcctt 28

<210> 140
<211> 29
<212> DNA
<213> Artificial Sequence

<220>
<221> source
<223> /note="Description of Artificial Sequence: Synthetic primer"

<400> 140
agacaaaaac tacttgctcc ttcccatcg 29

<210> 141
<211> 24
<212> DNA
<213> Artificial Sequence

<220>
<221> source
<223> /note="Description of Artificial Sequence: Synthetic primer"

<400> 141
tccttcccat cgctggagac cttg 24

<210> 142
<211> 24
<212> DNA
<213> Artificial Sequence

<220>
<221> source
<223> /note="Description of Artificial Sequence: Synthetic primer"

<400> 142
ccatcgctgg agaccttgtc acac 24

<210> 143
<211> 23
<212> DNA
<213> Artificial Sequence

<220>
<221> source
<223> /note="Description of Artificial Sequence: Synthetic primer"

<400> 143
agcaggacca gactcatccc gat 23

<210> 144
<211> 25
<212> DNA
<213> Artificial Sequence

<220>
<221> source
<223> /note="Description of Artificial Sequence: Synthetic primer"

<400> 144
accagactca tcccgatttg acagg 25

<210> 145
<211> 28
<212> DNA
<213> Artificial Sequence

<220>
<221> source
<223> /note="Description of Artificial Sequence: Synthetic primer"

<400> 145
agcttttggg tgaagggaaa tttaacag 28

<210> 146
<211> 28
<212> DNA
<213> Artificial Sequence

<220>
<221> source
<223> /note="Description of Artificial Sequence: Synthetic primer"

<400> 146
tgggtgaagg gaaatttaac agggatga 28

<210> 147
<211> 26
<212> DNA
<213> Artificial Sequence

<220>
<221> source
<223> /note="Description of Artificial Sequence: Synthetic primer"

<400> 147
acagggatga atatcagtgg cgggat 26

<210> 148
<211> 23
<212> DNA

<213> Artificial Sequence

<220>
<221> source
<223> /note="Description of Artificial Sequence: Synthetic primer"

<400> 148
ttggtttgaa acatgagctg cac 23

<210> 149
<211> 22
<212> DNA
<213> Artificial Sequence

<220>
<221> source
<223> /note="Description of Artificial Sequence: Synthetic primer"

<400> 149
gagatgtggg ggccgcaggt ga 22

<210> 150
<211> 21
<212> DNA
<213> Artificial Sequence

<220>
<221> source
<223> /note="Description of Artificial Sequence: Synthetic primer"

<400> 150
cgcaggtgac caaacaacag g 21

<210> 151
<211> 23
<212> DNA
<213> Artificial Sequence

<220>
<221> source
<223> /note="Description of Artificial Sequence: Synthetic primer"

<400> 151
accaaacaac agggtgcgct gta 23

<210> 152
<211> 26
<212> DNA
<213> Artificial Sequence

<220>
<221> source
<223> /note="Description of Artificial Sequence: Synthetic primer"

<400> 152
caacagggtg cgctgtaagt aacacc 26

<210> 153
<211> 26
<212> DNA
<213> Artificial Sequence

<220>
<221> source
<223> /note="Description of Artificial Sequence: Synthetic primer"

<400> 153
ctgtaagtaa caccctctga ggctaa 26

<210> 154
<211> 28
<212> DNA
<213> Artificial Sequence

<220>
<221> source
<223> /note="Description of Artificial Sequence: Synthetic primer"

<400> 154
gtaacaccct ctgaggctaa gttacttc 28

<210> 155
<211> 22
<212> DNA
<213> Artificial Sequence

<220>
<221> source
<223> /note="Description of Artificial Sequence: Synthetic primer"

<400> 155
ctcacagtgg ggaggggtcc tg 22

<210> 156
<211> 20
<212> DNA
<213> Artificial Sequence

<220>
<221> source
<223> /note="Description of Artificial Sequence: Synthetic primer"

<400> 156
gaggggtcct gtgccctctg 20

<210> 157
<211> 19
<212> DNA

<213> Artificial Sequence

<220>
<221> source
<223> /note="Description of Artificial Sequence: Synthetic primer"

<400> 157
ctctgctggc cagaccaca 19

<210> 158
<211> 24
<212> DNA
<213> Artificial Sequence

<220>
<221> source
<223> /note="Description of Artificial Sequence: Synthetic primer"

<400> 158
tggccagacc acacggaggt atcg 24

<210> 159
<211> 25
<212> DNA
<213> Artificial Sequence

<220>
<221> source
<223> /note="Description of Artificial Sequence: Synthetic primer"

<400> 159
gaccacacgg aggtatcgtg cttgg 25

<210> 160
<211> 23
<212> DNA
<213> Artificial Sequence

<220>
<221> source
<223> /note="Description of Artificial Sequence: Synthetic primer"

<400> 160
ggaggtatcg tgcttggtac tgg 23

<210> 161
<211> 23
<212> DNA
<213> Artificial Sequence

<220>
<221> source
<223> /note="Description of Artificial Sequence: Synthetic primer"

<400> 161
acacatgcag aagagggaca ctg 23

<210> 162
<211> 18
<212> DNA
<213> Artificial Sequence

<220>
<221> source
<223> /note="Description of Artificial Sequence: Synthetic primer"

<400> 162
actggtgggg cgggggaa 18

<210> 163
<211> 20
<212> DNA
<213> Artificial Sequence

<220>
<221> source
<223> /note="Description of Artificial Sequence: Synthetic primer"

<400> 163
gtggggcggg ggaagagaca 20

<210> 164
<211> 22
<212> DNA
<213> Artificial Sequence

<220>
<221> source
<223> /note="Description of Artificial Sequence: Synthetic primer"

<400> 164
cgggggaaga gacacaaaac tg 22

<210> 165
<211> 24
<212> DNA
<213> Artificial Sequence

<220>
<221> source
<223> /note="Description of Artificial Sequence: Synthetic primer"

<400> 165
tgaactgctg caaacaaatg ggtt 24

<210> 166
<211> 32
<212> DNA

<213> Artificial Sequence

<220>
<221> source
<223> /note="Description of Artificial Sequence: Synthetic primer"

<400> 166
gctgcaaaca aatgggttat ttagtctgaa ga 32

<210> 167
<211> 23
<212> DNA
<213> Artificial Sequence

<220>
<221> source
<223> /note="Description of Artificial Sequence: Synthetic primer"

<400> 167
acaacagtga aactccgcct caa 23

<210> 168
<211> 22
<212> DNA
<213> Artificial Sequence

<220>
<221> source
<223> /note="Description of Artificial Sequence: Synthetic primer"

<400> 168
aggaggagaa tgggagcact gt 22

<210> 169
<211> 23
<212> DNA
<213> Artificial Sequence

<220>
<221> source
<223> /note="Description of Artificial Sequence: Synthetic primer"

<400> 169
gagaatggga gcactgtctt caa 23

<210> 170
<211> 24
<212> DNA
<213> Artificial Sequence

<220>
<221> source
<223> /note="Description of Artificial Sequence: Synthetic primer"

<400> 170
actgtcttca aacactgcac atgc 24

<210> 171
<211> 24
<212> DNA
<213> Artificial Sequence

<220>
<221> source
<223> /note="Description of Artificial Sequence: Synthetic primer"

<400> 171
cttcaaacac tgcacatgca cagc 24

<210> 172
<211> 25
<212> DNA
<213> Artificial Sequence

<220>
<221> source
<223> /note="Description of Artificial Sequence: Synthetic primer"

<400> 172
acatgcacag ctgctgctgt aagga 25

<210> 173
<211> 25
<212> DNA
<213> Artificial Sequence

<220>
<221> source
<223> /note="Description of Artificial Sequence: Synthetic primer"

<400> 173
aaacctagga gaggaaggtt aaggc 25

<210> 174
<211> 25
<212> DNA
<213> Artificial Sequence

<220>
<221> source
<223> /note="Description of Artificial Sequence: Synthetic primer"

<400> 174
ggagaggaag gttaaggcaa caggt 25

<210> 175
<211> 23
<212> DNA

<213> Artificial Sequence

<220>
<221> source
<223> /note="Description of Artificial Sequence: Synthetic primer"

<400> 175
gttaaggcaa caggtcccca gct 23

<210> 176
<211> 21
<212> DNA
<213> Artificial Sequence

<220>
<221> source
<223> /note="Description of Artificial Sequence: Synthetic primer"

<400> 176
gcaacaggtc cccagctctg a 21

<210> 177
<211> 22
<212> DNA
<213> Artificial Sequence

<220>
<221> source
<223> /note="Description of Artificial Sequence: Synthetic primer"

<400> 177
cagctctgaa actgcccaag gg 22

<210> 178
<211> 21
<212> DNA
<213> Artificial Sequence

<220>
<221> source
<223> /note="Description of Artificial Sequence: Synthetic primer"

<400> 178
aaactgccca agggaacaga g 21

<210> 179
<211> 22
<212> DNA
<213> Artificial Sequence

<220>
<221> source
<223> /note="Description of Artificial Sequence: Synthetic primer"

<400> 179
aagatccctg tcactgggca tg 22

<210> 180
<211> 24
<212> DNA
<213> Artificial Sequence

<220>
<221> source
<223> /note="Description of Artificial Sequence: Synthetic primer"

<400> 180
ctgtcactgg gcatgtttaa gtgg 24

<210> 181
<211> 25
<212> DNA
<213> Artificial Sequence

<220>
<221> source
<223> /note="Description of Artificial Sequence: Synthetic primer"

<400> 181
cactgggcat gtttaagtgg aggca 25

<210> 182
<211> 24
<212> DNA
<213> Artificial Sequence

<220>
<221> source
<223> /note="Description of Artificial Sequence: Synthetic primer"

<400> 182
catgtttaag tggaggcagg atgg 24

<210> 183
<211> 18
<212> DNA
<213> Artificial Sequence

<220>
<221> source
<223> /note="Description of Artificial Sequence: Synthetic primer"

<400> 183
caggatggcc ccttggtg 18

<210> 184
<211> 20
<212> DNA

<213> Artificial Sequence

<220>
<221> source
<223> /note="Description of Artificial Sequence: Synthetic primer"

<400> 184
ccttggtggg ggtggtagag 20

<210> 185
<211> 25
<212> DNA
<213> Artificial Sequence

<220>
<221> source
<223> /note="Description of Artificial Sequence: Synthetic primer"

<400> 185
gagccatgac ccacctttca cacag 25

<210> 186
<211> 23
<212> DNA
<213> Artificial Sequence

<220>
<221> source
<223> /note="Description of Artificial Sequence: Synthetic primer"

<400> 186
gacccacctt tcacacagtg gtc 23

<210> 187
<211> 22
<212> DNA
<213> Artificial Sequence

<220>
<221> source
<223> /note="Description of Artificial Sequence: Synthetic primer"

<400> 187
agtggtcaga gcactcgagc tg 22

<210> 188
<211> 20
<212> DNA
<213> Artificial Sequence

<220>
<221> source
<223> /note="Description of Artificial Sequence: Synthetic primer"

<400> 188
agagcactcg agctgtggca 20

<210> 189
<211> 25
<212> DNA
<213> Artificial Sequence

<220>
<221> source
<223> /note="Description of Artificial Sequence: Synthetic primer"

<400> 189
aggtagggga gggacagaaa gttta 25

<210> 190
<211> 26
<212> DNA
<213> Artificial Sequence

<220>
<221> source
<223> /note="Description of Artificial Sequence: Synthetic primer"

<400> 190
ggagggacag aaagtttaca aaaccg 26

<210> 191
<211> 29
<212> DNA
<213> Artificial Sequence

<220>
<221> source
<223> /note="Description of Artificial Sequence: Synthetic primer"

<400> 191
cagaaagttt acaaaaccga atccagggt 29

<210> 192
<211> 25
<212> DNA
<213> Artificial Sequence

<220>
<221> source
<223> /note="Description of Artificial Sequence: Synthetic primer"

<400> 192
acaaaaccga atccagggtg ttctg 25

<210> 193
<211> 26
<212> DNA

<213> Artificial Sequence

<220>
<221> source
<223> /note="Description of Artificial Sequence: Synthetic primer"

<400> 193
aacccagaaa ccatttgtgg tcatgg 26

<210> 194
<211> 25
<212> DNA
<213> Artificial Sequence

<220>
<221> source
<223> /note="Description of Artificial Sequence: Synthetic primer"

<400> 194
aaaccatttg tggtcatggg ccaaa 25

<210> 195
<211> 18
<212> DNA
<213> Artificial Sequence

<220>
<221> source
<223> /note="Description of Artificial Sequence: Synthetic primer"

<400> 195
cctggccctg ccccctta 18

<210> 196
<211> 21
<212> DNA
<213> Artificial Sequence

<220>
<221> source
<223> /note="Description of Artificial Sequence: Synthetic primer"

<400> 196
cctgccccct taccaatgca g 21

<210> 197
<211> 24
<212> DNA
<213> Artificial Sequence

<220>
<221> source
<223> /note="Description of Artificial Sequence: Synthetic primer"

<400> 197
cttaccaatg caggagacgc catc 24

<210> 198
<211> 21
<212> DNA
<213> Artificial Sequence

<220>
<221> source
<223> /note="Description of Artificial Sequence: Synthetic primer"

<400> 198
tgcaggagac gccatcctca g 21

<210> 199
<211> 26
<212> DNA
<213> Artificial Sequence

<220>
<221> source
<223> /note="Description of Artificial Sequence: Synthetic primer"

<400> 199
cgtggtcaca gaagcagatg accttg 26

<210> 200
<211> 26
<212> DNA
<213> Artificial Sequence

<220>
<221> source
<223> /note="Description of Artificial Sequence: Synthetic primer"

<400> 200
cacagaagca gatgaccttg tggctt 26

<210> 201
<211> 22
<212> DNA
<213> Artificial Sequence

<220>
<221> source
<223> /note="Description of Artificial Sequence: Synthetic primer"

<400> 201
gtggctttca gggtccatgt ga 22

<210> 202
<211> 25
<212> DNA

<213> Artificial Sequence

<220>
<221> source
<223> /note="Description of Artificial Sequence: Synthetic primer"

<400> 202
caggtccat gtgacattcg tctac 25

<210> 203
<211> 29
<212> DNA
<213> Artificial Sequence

<220>
<221> source
<223> /note="Description of Artificial Sequence: Synthetic primer"

<400> 203
tccatgtgac attcgtctac ctcacagtg 29

<210> 204
<211> 25
<212> DNA
<213> Artificial Sequence

<220>
<221> source
<223> /note="Description of Artificial Sequence: Synthetic primer"

<400> 204
cattcgtcta cctcacagtg actgc 25

<210> 205
<211> 31
<212> DNA
<213> Artificial Sequence

<220>
<221> source
<223> /note="Description of Artificial Sequence: Synthetic primer"

<400> 205
taatgcttaa tattcacttc cccgtggcct t 31

<210> 206
<211> 27
<212> DNA
<213> Artificial Sequence

<220>
<221> source
<223> /note="Description of Artificial Sequence: Synthetic primer"

<400> 206
taatattcac ttccccgtgg ccttcca 27

<210> 207
<211> 24
<212> DNA
<213> Artificial Sequence

<220>
<221> source
<223> /note="Description of Artificial Sequence: Synthetic primer"

<400> 207
ccgtggcctt ccatcactag tgac 24

<210> 208
<211> 24
<212> DNA
<213> Artificial Sequence

<220>
<221> source
<223> /note="Description of Artificial Sequence: Synthetic primer"

<400> 208
tccatcacta gtgacaagga ggga 24

<210> 209
<211> 24
<212> DNA
<213> Artificial Sequence

<220>
<221> source
<223> /note="Description of Artificial Sequence: Synthetic primer"

<400> 209
gtgacaagga gggagggtca gtct 24

<210> 210
<211> 20
<212> DNA
<213> Artificial Sequence

<220>
<221> source
<223> /note="Description of Artificial Sequence: Synthetic primer"

<400> 210
gtcagtcttg ggccgagcct 20

<210> 211
<211> 16
<212> DNA

<213> Artificial Sequence

<220>
<221> source
<223> /note="Description of Artificial Sequence: Synthetic primer"

<400> 211
tgggccgagc ctgcct 16

<210> 212
<211> 18
<212> DNA
<213> Artificial Sequence

<220>
<221> source
<223> /note="Description of Artificial Sequence: Synthetic primer"

<400> 212
gagcctgcct ccccactc 18

<210> 213
<211> 25
<212> DNA
<213> Artificial Sequence

<220>
<221> source
<223> /note="Description of Artificial Sequence: Synthetic primer"

<400> 213
ccactcccag cctcagtact atgtc 25

<210> 214
<211> 26
<212> DNA
<213> Artificial Sequence

<220>
<221> source
<223> /note="Description of Artificial Sequence: Synthetic primer"

<400> 214
cagcctcagt actatgtctc caggtg 26

<210> 215
<211> 19
<212> DNA
<213> Artificial Sequence

<220>
<221> source
<223> /note="Description of Artificial Sequence: Synthetic primer"

<400> 215
ccaggtggtc actgtgggt 19

<210> 216
<211> 21
<212> DNA
<213> Artificial Sequence

<220>
<221> source
<223> /note="Description of Artificial Sequence: Synthetic primer"

<400> 216
gtcactgtgg gtgctctggt g 21

<210> 217
<211> 21
<212> DNA
<213> Artificial Sequence

<220>
<221> source
<223> /note="Description of Artificial Sequence: Synthetic primer"

<400> 217
actgtgggtg ctctggtggt c 21

<210> 218
<211> 21
<212> DNA
<213> Artificial Sequence

<220>
<221> source
<223> /note="Description of Artificial Sequence: Synthetic primer"

<400> 218
tgctctggtg gtccctgttc c 21

<210> 219
<211> 25
<212> DNA
<213> Artificial Sequence

<220>
<221> source
<223> /note="Description of Artificial Sequence: Synthetic primer"

<400> 219
gtccctgttc ctaggtccca tagcc 25

<210> 220
<211> 26
<212> DNA

<213> Artificial Sequence

<220>
<221> source
<223> /note="Description of Artificial Sequence: Synthetic primer"

<400> 220
ttcctaggtc ccatagccac tggaag 26

<210> 221
<211> 26
<212> DNA
<213> Artificial Sequence

<220>
<221> source
<223> /note="Description of Artificial Sequence: Synthetic primer"

<400> 221
aattttccat ccatcccagg tcacat 26

<210> 222
<211> 25
<212> DNA
<213> Artificial Sequence

<220>
<221> source
<223> /note="Description of Artificial Sequence: Synthetic primer"

<400> 222
ccatccatcc caggtcacat ttgag 25

<210> 223
<211> 25
<212> DNA
<213> Artificial Sequence

<220>
<221> source
<223> /note="Description of Artificial Sequence: Synthetic primer"

<400> 223
tggtataaac catgactgtc ttggg 25

<210> 224
<211> 24
<212> DNA
<213> Artificial Sequence

<220>
<221> source
<223> /note="Description of Artificial Sequence: Synthetic primer"

<400> 224
aaaccatgac tgtcttgggc aatg 24

<210> 225
<211> 24
<212> DNA
<213> Artificial Sequence

<220>
<221> source
<223> /note="Description of Artificial Sequence: Synthetic primer"

<400> 225
actgtcttgg gcaatgcgga attc 24

<210> 226
<211> 23
<212> DNA
<213> Artificial Sequence

<220>
<221> source
<223> /note="Description of Artificial Sequence: Synthetic primer"

<400> 226
tgggcaatgc ggaattcata ggc 23

<210> 227
<211> 25
<212> DNA
<213> Artificial Sequence

<220>
<221> source
<223> /note="Description of Artificial Sequence: Synthetic primer"

<400> 227
tactgttgcc cacccttttgc ctagg 25

<210> 228
<211> 25
<212> DNA
<213> Artificial Sequence

<220>
<221> source
<223> /note="Description of Artificial Sequence: Synthetic primer"

<400> 228
ccaccctttg cctaggtgct ccata 25

<210> 229
<211> 23
<212> DNA

<213> Artificial Sequence

<220>
<221> source
<223> /note="Description of Artificial Sequence: Synthetic primer"

<400> 229
tgcctaggtg ctccataatg atg 23

<210> 230
<211> 25
<212> DNA
<213> Artificial Sequence

<220>
<221> source
<223> /note="Description of Artificial Sequence: Synthetic primer"

<400> 230
gtgctccata atgatggcca aagct 25

<210> 231
<211> 19
<212> DNA
<213> Artificial Sequence

<220>
<221> source
<223> /note="Description of Artificial Sequence: Synthetic primer"

<400> 231
gccagacctc gcagctcag 19

<210> 232
<211> 21
<212> DNA
<213> Artificial Sequence

<220>
<221> source
<223> /note="Description of Artificial Sequence: Synthetic primer"

<400> 232
cctcgcagct cagccaactc t 21

<210> 233
<211> 24
<212> DNA
<213> Artificial Sequence

<220>
<221> source
<223> /note="Description of Artificial Sequence: Synthetic primer"

<400> 233
tataagcact gtcacccctt cctt 24

<210> 234
<211> 21
<212> DNA
<213> Artificial Sequence

<220>
<221> source
<223> /note="Description of Artificial Sequence: Synthetic primer"

<400> 234
cactgtcacc ccttccttgg c 21

<210> 235
<211> 31
<212> DNA
<213> Artificial Sequence

<220>
<221> source
<223> /note="Description of Artificial Sequence: Synthetic primer"

<400> 235
agaaaaagta ttggaacgaa gcttaccaaa t 31

<210> 236
<211> 27
<212> DNA
<213> Artificial Sequence

<220>
<221> source
<223> /note="Description of Artificial Sequence: Synthetic primer"

<400> 236
ttggaacgaa gcttaccaaa tgttagc 27

<210> 237
<211> 31
<212> DNA
<213> Artificial Sequence

<220>
<221> source
<223> /note="Description of Artificial Sequence: Synthetic primer"

<400> 237
caataggatt gtcatctgta ctggactatt g 31

<210> 238
<211> 30
<212> DNA

<213> Artificial Sequence

<220>
<221> source
<223> /note="Description of Artificial Sequence: Synthetic primer"

<400> 238
tgtcatctgt actggactat tggctcagaa 30

<210> 239
<211> 25
<212> DNA
<213> Artificial Sequence

<220>
<221> source
<223> /note="Description of Artificial Sequence: Synthetic primer"

<400> 239
atgttctcac cacctcgctt cactt 25

<210> 240
<211> 25
<212> DNA
<213> Artificial Sequence

<220>
<221> source
<223> /note="Description of Artificial Sequence: Synthetic primer"

<400> 240
accacctcgc ttcactttta aaggc 25

<210> 241
<211> 26
<212> DNA
<213> Artificial Sequence

<220>
<221> source
<223> /note="Description of Artificial Sequence: Synthetic primer"

<400> 241
ttcactttta aaggcccaat gtaagc 26

<210> 242
<211> 21
<212> DNA
<213> Artificial Sequence

<220>
<221> source
<223> /note="Description of Artificial Sequence: Synthetic primer"

<400> 242
aaggcccaat gtaagcccac a 21

<210> 243
<211> 30
<212> DNA
<213> Artificial Sequence

<220>
<221> source
<223> /note="Description of Artificial Sequence: Synthetic primer"

<400> 243
ttcatatctg aacttgaaag actaattgcg 30

<210> 244
<211> 29
<212> DNA
<213> Artificial Sequence

<220>
<221> source
<223> /note="Description of Artificial Sequence: Synthetic primer"

<400> 244
tctgaacttg aaagactaat tgcgcctct 29

<210> 245
<211> 31
<212> DNA
<213> Artificial Sequence

<220>
<221> source
<223> /note="Description of Artificial Sequence: Synthetic primer"

<400> 245
tttaacacat atcttgctat ttgtcttccc a 31

<210> 246
<211> 30
<212> DNA
<213> Artificial Sequence

<220>
<221> source
<223> /note="Description of Artificial Sequence: Synthetic primer"

<400> 246
catatcttgc tatttgtctt cccacacagg 30

<210> 247
<211> 26
<212> DNA

<213> Artificial Sequence

<220>
<221> source
<223> /note="Description of Artificial Sequence: Synthetic primer"

<400> 247
aagctcctga ggtcagcagt tgtatt 26

<210> 248
<211> 28
<212> DNA
<213> Artificial Sequence

<220>
<221> source
<223> /note="Description of Artificial Sequence: Synthetic primer"

<400> 248
ctgaggtcag cagttgtatt ttacaggt 28

<210> 249
<211> 26
<212> DNA
<213> Artificial Sequence

<220>
<221> source
<223> /note="Description of Artificial Sequence: Synthetic primer"

<400> 249
aatcttttct catgcacaca cacgct 26

<210> 250
<211> 23
<212> DNA
<213> Artificial Sequence

<220>
<221> source
<223> /note="Description of Artificial Sequence: Synthetic primer"

<400> 250
ttctcatgca cacacacgct ctt 23

<210> 251
<211> 24
<212> DNA
<213> Artificial Sequence

<220>
<221> source
<223> /note="Description of Artificial Sequence: Synthetic primer"

<400> 251
agtaaaacca catgcactgc tgct 24

<210> 252
<211> 24
<212> DNA
<213> Artificial Sequence

<220>
<221> source
<223> /note="Description of Artificial Sequence: Synthetic primer"

<400> 252
accacatgca ctgctgctaa aagc 24

<210> 253
<211> 26
<212> DNA
<213> Artificial Sequence

<220>
<221> source
<223> /note="Description of Artificial Sequence: Synthetic primer"

<400> 253
gctaaaagca aagaaccgaa ttagct 26

<210> 254
<211> 30
<212> DNA
<213> Artificial Sequence

<220>
<221> source
<223> /note="Description of Artificial Sequence: Synthetic primer"

<400> 254
agcaaagaac cgaattagct tagctagact 30

<210> 255
<211> 24
<212> DNA
<213> Artificial Sequence

<220>
<221> source
<223> /note="Description of Artificial Sequence: Synthetic primer"

<400> 255
gtaggaaagc agacaagcag tcaa 24

<210> 256
<211> 22
<212> DNA

<213> Artificial Sequence

<220>
<221> source
<223> /note="Description of Artificial Sequence: Synthetic primer"

<400> 256
aagcagacaa gcagtcaagc ct 22

<210> 257
<211> 25
<212> DNA
<213> Artificial Sequence

<220>
<221> source
<223> /note="Description of Artificial Sequence: Synthetic primer"

<400> 257
gacaagcagt caagcctata tgcca 25

<210> 258
<211> 25
<212> DNA
<213> Artificial Sequence

<220>
<221> source
<223> /note="Description of Artificial Sequence: Synthetic primer"

<400> 258
agtcaagcct atatgccaac tgcca 25

<210> 259
<211> 30
<212> DNA
<213> Artificial Sequence

<220>
<221> source
<223> /note="Description of Artificial Sequence: Synthetic primer"

<400> 259
ctatatgcca actgccaaat atactgcagt 30

<210> 260
<211> 26
<212> DNA
<213> Artificial Sequence

<220>
<221> source
<223> /note="Description of Artificial Sequence: Synthetic primer"

<400> 260
ccaactgcca aatatactgc agtcat 26

<210> 261
<211> 21
<212> DNA
<213> Artificial Sequence

<220>
<221> source
<223> /note="Description of Artificial Sequence: Synthetic primer"

<400> 261
gtaagtctgg aggggctggc t 21

<210> 262
<211> 20
<212> DNA
<213> Artificial Sequence

<220>
<221> source
<223> /note="Description of Artificial Sequence: Synthetic primer"

<400> 262
tggaggggct ggcttagtgg 20

<210> 263
<211> 18
<212> DNA
<213> Artificial Sequence

<220>
<221> source
<223> /note="Description of Artificial Sequence: Synthetic primer"

<400> 263
ggctggctta gtggcaca 18

<210> 264
<211> 24
<212> DNA
<213> Artificial Sequence

<220>
<221> source
<223> /note="Description of Artificial Sequence: Synthetic primer"

<400> 264
tagtggcaca aacatcagct gtgc 24

<210> 265
<211> 26
<212> DNA

<213> Artificial Sequence

<220>
<221> source
<223> /note="Description of Artificial Sequence: Synthetic primer"

<400> 265
atacttagac tctctggaac cacagg 26

<210> 266
<211> 25
<212> DNA
<213> Artificial Sequence

<220>
<221> source
<223> /note="Description of Artificial Sequence: Synthetic primer"

<400> 266
agactctctg gaaccacagg ttaaa 25

<210> 267
<211> 28
<212> DNA
<213> Artificial Sequence

<220>
<221> source
<223> /note="Description of Artificial Sequence: Synthetic primer"

<400> 267
gaaccacagg ttaaaatcct ggcagggt 28

<210> 268
<211> 26
<212> DNA
<213> Artificial Sequence

<220>
<221> source
<223> /note="Description of Artificial Sequence: Synthetic primer"

<400> 268
ggttaaaatc ctggcagggt cagctg 26

<210> 269
<211> 26
<212> DNA
<213> Artificial Sequence

<220>
<221> source
<223> /note="Description of Artificial Sequence: Synthetic primer"

<400> 269
tttagctttc caagtttgct gaagga 26

<210> 270
<211> 26
<212> DNA
<213> Artificial Sequence

<220>
<221> source
<223> /note="Description of Artificial Sequence: Synthetic primer"

<400> 270
tttccaagtt tgctgaagga gtttca 26

<210> 271
<211> 27
<212> DNA
<213> Artificial Sequence

<220>
<221> source
<223> /note="Description of Artificial Sequence: Synthetic primer"

<400> 271
acagtaaagt gttggctgtc tttatcc 27

<210> 272
<211> 26
<212> DNA
<213> Artificial Sequence

<220>
<221> source
<223> /note="Description of Artificial Sequence: Synthetic primer"

<400> 272
aagtgttggc tgtctttatc ctgaga 26

<210> 273
<211> 26
<212> DNA
<213> Artificial Sequence

<220>
<221> source
<223> /note="Description of Artificial Sequence: Synthetic primer"

<400> 273
aactttccct tgtaacttac ccctac 26

<210> 274
<211> 25
<212> DNA

<213> Artificial Sequence

<220>
<221> source
<223> /note="Description of Artificial Sequence: Synthetic primer"

<400> 274
ccccttgtaac ttacccctac ttgtg 25

<210> 275
<211> 30
<212> DNA
<213> Artificial Sequence

<220>
<221> source
<223> /note="Description of Artificial Sequence: Synthetic primer"

<400> 275
tgttggggac tctcaatatt agcaatagat 30

<210> 276
<211> 33
<212> DNA
<213> Artificial Sequence

<220>
<221> source
<223> /note="Description of Artificial Sequence: Synthetic primer"

<400> 276
ggactctcaa tattagcaat agataggggt cca 33

<210> 277
<211> 22
<212> DNA
<213> Artificial Sequence

<220>
<221> source
<223> /note="Description of Artificial Sequence: Synthetic primer"

<400> 277
aaactcggac agtttgggga at 22

<210> 278
<211> 25
<212> DNA
<213> Artificial Sequence

<220>
<221> source
<223> /note="Description of Artificial Sequence: Synthetic primer"

<400> 278
ggacagtttg gggaatgaaa gaaag 25

<210> 279
<211> 25
<212> DNA
<213> Artificial Sequence

<220>
<221> source
<223> /note="Description of Artificial Sequence: Synthetic primer"

<400> 279
agatgtctga aagcaagagt cgatc 25

<210> 280
<211> 24
<212> DNA
<213> Artificial Sequence

<220>
<221> source
<223> /note="Description of Artificial Sequence: Synthetic primer"

<400> 280
ctgaaagcaa gagtcgatcc caca 24

<210> 281
<211> 20
<212> DNA
<213> Artificial Sequence

<220>
<221> source
<223> /note="Description of Artificial Sequence: Synthetic primer"

<400> 281
agtcgatccc acaagccaga 20

<210> 282
<211> 26
<212> DNA
<213> Artificial Sequence

<220>
<221> source
<223> /note="Description of Artificial Sequence: Synthetic primer"

<400> 282
tcccacaagc cagaaatgga tcttct 26

<210> 283
<211> 25
<212> DNA

<213> Artificial Sequence

<220>
<221> source
<223> /note="Description of Artificial Sequence: Synthetic primer"

<400> 283
ccaaaccagc ctgtctgctt agaaa 25

<210> 284
<211> 29
<212> DNA
<213> Artificial Sequence

<220>
<221> source
<223> /note="Description of Artificial Sequence: Synthetic primer"

<400> 284
cctgtctgct tagaaaccaa aactatccc 29

<210> 285
<211> 31
<212> DNA
<213> Artificial Sequence

<220>
<221> source
<223> /note="Description of Artificial Sequence: Synthetic primer"

<400> 285
gaaaccaaaa ctatcccaat caaagattgt c 31

<210> 286
<211> 30
<212> DNA
<213> Artificial Sequence

<220>
<221> source
<223> /note="Description of Artificial Sequence: Synthetic primer"

<400> 286
aaaactatcc caatcaaaga ttgtcactgg 30

<210> 287
<211> 33
<212> DNA
<213> Artificial Sequence

<220>
<221> source
<223> /note="Description of Artificial Sequence: Synthetic primer"

<400> 287
aaaacatttg tatgcagcca atcagaaata aat 33

<210> 288
<211> 31
<212> DNA
<213> Artificial Sequence

<220>
<221> source
<223> /note="Description of Artificial Sequence: Synthetic primer"

<400> 288
ttgtatgcag ccaatcagaa ataaatactg t 31

<210> 289
<211> 28
<212> DNA
<213> Artificial Sequence

<220>
<221> source
<223> /note="Description of Artificial Sequence: Synthetic primer"

<400> 289
ctattatact taccaaaggt cagtggga 28

<210> 290
<211> 24
<212> DNA
<213> Artificial Sequence

<220>
<221> source
<223> /note="Description of Artificial Sequence: Synthetic primer"

<400> 290
acttaccaaa ggtcagtggg attg 24

<210> 291
<211> 27
<212> DNA
<213> Artificial Sequence

<220>
<221> source
<223> /note="Description of Artificial Sequence: Synthetic primer"

<400> 291
atagataaaa gctaagttgc cccagct 27

<210> 292
<211> 26
<212> DNA

<213> Artificial Sequence

<220>
<221> source
<223> /note="Description of Artificial Sequence: Synthetic primer"

<400> 292
aaaagctaag ttgccccagc tctacc 26

<210> 293
<211> 30
<212> DNA
<213> Artificial Sequence

<220>
<221> source
<223> /note="Description of Artificial Sequence: Synthetic primer"

<400> 293
ctctacctaa gcacacagag taatatagca 30

<210> 294
<211> 29
<212> DNA
<213> Artificial Sequence

<220>
<221> source
<223> /note="Description of Artificial Sequence: Synthetic primer"

<400> 294
gcacacagag taatatagca gagctagct 29

<210> 295
<211> 33
<212> DNA
<213> Artificial Sequence

<220>
<221> source
<223> /note="Description of Artificial Sequence: Synthetic primer"

<400> 295
aatatagcag agctagctac tactggattt ttg 33

<210> 296
<211> 29
<212> DNA
<213> Artificial Sequence

<220>
<221> source
<223> /note="Description of Artificial Sequence: Synthetic primer"

<400> 296
gagctagcta ctactggatt tttgcaagc 29

<210> 297
<211> 20
<212> DNA
<213> Artificial Sequence

<220>
<221> source
<223> /note="Description of Artificial Sequence: Synthetic primer tag sequence"

<400> 297
acacgacgct cttccgatct 20

<210> 298
<211> 21
<212> DNA
<213> Artificial Sequence

<220>
<221> source
<223> /note="Description of Artificial Sequence: Synthetic primer tag sequence"

<400> 298
agacgtgtgc tcttccgatc t 21

<210> 299
<211> 304
<212> DNA
<213> Artificial Sequence

<220>
<221> source
<223> /note="Description of Artificial Sequence: Synthetic gene fusion spike polynucleotide"

<400> 299
tggttaggga aacagggcag gagttaccat ccctgcctac agagagggaa actgcagtcc 60

aaagaggtcc tgtgacctgg tcctcatggc tcagcttgta agtaacaaga ggcggaatta 120

gagcacagat ccccagacac caattcagat cctaggaagt ctcagttttt agagtattta 180

ctatcagtgt tctttttttt tctgacttct tgctgcttga gttttataat gtctaataaa 240

ttgtatttta gctgtggagg aagatgcaga gtcagaagat gaagaggagg aggatgtgaa 300

actc 304

<210> 300
<211> 304
<212> DNA
<213> Artificial Sequence

<220>
<221> source

<223> /note="Description of Artificial Sequence: Synthetic gene fusion spike polynucleotide"

<400> 300
aaagttcctt ttcccatgtg ctcttttttt tttttttttt aaatagaata gaagtctcag 60

tttttagagt atttactatc agtgttcttt ttttttctga ctctcagttt ttagagtcat 120

ttactatcag tgttcttttt tttctgaccc ctgggccagc tgcaccctca aatccactgc 180

tgtgattgca ctgaagctgc cctacccaat ggctgagcac agcagaaata ctaaggcagg 240

cccaattcct gggagtcatg ggactcctct gatgactgac tttggctcca gaacccctta 300

gggc 304

<210> 301
<211> 304
<212> DNA
<213> Artificial Sequence

<220>
<221> source
<223> /note="Description of Artificial Sequence: Synthetic gene fusion spike polynucleotide"

<400> 301
agtgttttgg tttctcccac agtattctga aaaggaggac aaatatgaag aaagaaatta 60

aacttctgtc tgacaaactg aaagaggctg agacccgtgc tgaatttgca gagagaacgg 120

ttgcaaaact ggaaaagaca attgatgacc tggaagtgta ccgccggaag caccaggagc 180

tgcaagccat gcagatggag ctgcagagcc ctgagtacaa gctgagcaag ctccgcacct 240

cgaccatcat gaccgactac aaaccccaac tactgctttg ctggcaagac ctcctccatc 300

agtg 304

<210> 302
<211> 304
<212> DNA
<213> Artificial Sequence

<220>
<221> source
<223> /note="Description of Artificial Sequence: Synthetic gene fusion spike polynucleotide"

<400> 302
aagccaggca gtgtaggggc ttggtggtgg ccatcgaacc tgacctccac ctctatccgt 60

attaggtctt tgagagctgg atgcaccatt ggctcctgtt tgaaatgagc aggcactcct 120

tggagcaaaa gcccactgac gctccaccga aagatgattt ttggatacca gaaacaagtt 180

tcatacttta ctattatagt tggaatattt ctggttgtta caatcccact gacctttggt 240

aagtataata gaatttttaa aataggcaac aaactgttta cttaatcata cctgattgat 300

ttat 304

<210> 303
<211> 62
<212> DNA
<213> Artificial Sequence

<220>
<221> source
<223> /note="Description of Artificial Sequence: Synthetic gene fusion spike oligonucleotide"

<400> 303
ctgcagacaa gcataaagat gtcatcatca accaagtgta ccgccggaag caccaggagc 60

tg 62

<210> 304
<211> 54
<212> DNA
<213> Artificial Sequence

<220>
<221> source
<223> /note="Description of Artificial Sequence: Synthetic gene fusion spike oligonucleotide"

<400> 304
atgtcaactc gcgaaaaaaa cagccaagtg taccgccgga agcaccagga gctg 54

<210> 305
<211> 19
<212> DNA
<213> Artificial Sequence

<220>
<221> source
<223> /note="Description of Artificial Sequence: Synthetic primer"

<400> 305
caaaagccca ctgacgctc 19

<210> 306
<211> 34
<212> DNA
<213> Artificial Sequence

<220>
<221> source
<223> /note="Description of Artificial Sequence: Synthetic primer"

<400> 306
ttaagtaaac agtttgttgc ctattttaaa aatt 34

<210> 307
<211> 20
<212> DNA

<213> Artificial Sequence

<220>
<221> source
<223> /note="Description of Artificial Sequence: Synthetic primer"

<400> 307
gcagagagaa cggttgcaaa 20

<210> 308
<211> 21
<212> DNA
<213> Artificial Sequence

<220>
<221> source
<223> /note="Description of Artificial Sequence: Synthetic primer"

<400> 308
ggggtttgta gtcggtcatg a 21

<210> 309
<211> 160
<212> DNA
<213> Artificial Sequence

<220>
<221> source
<223> /note="Description of Artificial Sequence: Synthetic gene fusion spike polynucleotide"

<400> 309
caaaagccca ctgacgctcc accgaaagat gatttttgga taccagaaac aagtttcata 60

ctttactatt atagttggaa tatttctggt tgttacaatc ccactgacct ttggtaagta 120

taatagaatt tttaaaatag gcaacaaact gtttacttaa 160

<210> 310
<211> 160
<212> DNA
<213> Artificial Sequence

<220>
<221> source
<223> /note="Description of Artificial Sequence: Synthetic gene fusion spike polynucleotide"

<400> 310
gcagagagaa cggttgcaaa actggaaaag acaattgatg acctggaagt gtaccgccgg 60

aagcaccagg agctgcaagc catgcagatg gagctgcaga gccctgagta caagctgagc 120

aagctccgca cctcgaccat catgaccgac tacaaacccc 160

<210> 311
<211> 284

<212> DNA
<213> Artificial Sequence

<220>
<221> source
<223> /note="Description of Artificial Sequence: Synthetic
template polynucleotide"

<400> 311
acacgacgct cttccgattc tgtcatccaa atactccaca cgcaaatttc cttccactcg 60

gataagatgc tgaggagggg ccagacctaa gagcaatcag tgaggaatca gaggcctggg 120

gaccctgggc aaccagccct gtcgtctctc cagccccagc tgctcaccat cgctatctga 180

gcagcgctca tggtgggggc agcgcctcac aacctccgtc atgtgctgtg actgcttgta 240

gatggccatg agatcggaag agcacacgtc tgaactccag tcac 284

<210> 312
<211> 43
<212> DNA
<213> Artificial Sequence

<220>
<221> source
<223> /note="Description of Artificial Sequence: Synthetic
primer tag sequence"

<400> 312
acacgacgct cttccgattc tgtcatccaa atactccaca cgc 43

<210> 313
<211> 40
<212> DNA
<213> Artificial Sequence

<220>
<221> source
<223> /note="Description of Artificial Sequence: Synthetic
primer tag sequence"

<400> 313
acacgacgct cttccgatct cttccactcg gataagatgc 40

<210> 314
<211> 36
<212> DNA
<213> Artificial Sequence

<220>
<221> source
<223> /note="Description of Artificial Sequence: Synthetic
primer tag sequence"

<400> 314
acacgacgct cttccgatgg gccagaccta agagca 36

<210> 315

<211> 40
<212> DNA
<213> Artificial Sequence

<220>
<221> source
<223> /note="Description of Artificial Sequence: Synthetic primer tag sequence"

<400> 315
acacgacgct cttccgattc agtgaggaat cagaggcctg 40

<210> 316
<211> 39
<212> DNA
<213> Artificial Sequence

<220>
<221> source
<223> /note="Description of Artificial Sequence: Synthetic primer tag sequence"

<400> 316
acacgacgct cttccgatct cctgggcaac cagccctgt 39

<210> 317
<211> 38
<212> DNA
<213> Artificial Sequence

<220>
<221> source
<223> /note="Description of Artificial Sequence: Synthetic primer"

<400> 317
acacgacgct cttccgatca gctgctcacc atcgctat 38

<210> 318
<211> 35
<212> DNA
<213> Artificial Sequence

<220>
<221> source
<223> /note="Description of Artificial Sequence: Synthetic primer tag sequence"

<400> 318
acacgacgct cttccgatga gcagcgctca tggtg 35

<210> 319
<211> 39
<212> DNA
<213> Artificial Sequence

<220>
<221> source
<223> /note="Description of Artificial Sequence: Synthetic

primer tag sequence"

<400> 319
acacgacgct cttccgatct cagcgcctca caacctccg 39

<210> 320
<211> 45
<212> DNA
<213> Artificial Sequence

<220>
<221> source
<223> /note="Description of Artificial Sequence: Synthetic primer tag sequence"

<400> 320
agacgtgtgc tcttccgatc tcatggccat ctacaagcag tcaca 45

<210> 321
<211> 43
<212> DNA
<213> Artificial Sequence

<220>
<221> source
<223> /note="Description of Artificial Sequence: Synthetic primer tag sequence"

<400> 321
agacgtgtgc tcttccgatc taccatgagc gctgctcaga tag 43

<210> 322
<211> 39
<212> DNA
<213> Artificial Sequence

<220>
<221> source
<223> /note="Description of Artificial Sequence: Synthetic primer tag sequence"

<400> 322
agacgtgtgc tcttccgatc tgatggtgag cagctgggg 39

<210> 323
<211> 34
<212> DNA
<213> Artificial Sequence

<220>
<221> source
<223> /note="Description of Artificial Sequence: Synthetic primer tag sequence"

<400> 323
gtgactggag ttcagacgtg tgctcttccg atct 34

## Description

### CROSS-REFERENCE TO RELATED APPLICATIONS

This application claims the benefit of U.S. Provisional Application Serial No. 62/357,847, filed July 1, 2016, which is hereby incorporated by reference in its entirety.

### SEQUENCE LISTING

The instant application contains a Sequence Listing which has been submitted electronically in ASCII format and is hereby incorporated by reference in its entirety. Said ASCII copy, created on June 29, 2017, is named N_017_WO_01_SL.TXT and is 83,468 bytes in size.

### FIELD OF THE INVENTION

The disclosed inventions relate generally to methods for detecting nucleic acid mutations and fusions using amplification methods such as the polymerase chain reaction (PCR).

### BACKGROUND OF THE INVENTION

Detection of mutations associated with disease, including cancers whether prior to diagnosis, in making a diagnosis, for disease staging or to monitor treatment efficacy has traditionally relied or solid tumor biopsy samples. Such sampling is highly invasive and not without risk of potentially contributing to metastasis or surgical complications. Mutations determinative for disease or developmental abnormalities can be recognized as a chromosomal translocation, an interstitial deletion, a single nucleotide variation (SNV), an inversion, a single nucleotide polymorphism (SNP), an insertion, a deletion, a substitution, and combinations thereof. Chromosomal translocations or gene fusions can be associated with genes know to be involved in a variety of cancers including AKT1, ALK, BRAF, EGFR, HER2, KRAS, MEK1, MET, NRAS, PIK3CA, RET, and ROS1 and others.

Gene fusions are some of the main driver events in certain cancers, such as lung cancer. Gene fusions are usually detected by mRNA-Seq in tumor biopsies, but that approach cannot be applied to fusion detection in plasma. The ability to detect mutations using a simple blood draw can avoid highly invasive medical procedures and potential complications, including scaring. The disclosed invention takes advantage of the ability to detect mutations in cell-free DNA samples such as serum or plasma found in blood.

### SUMMARY OF THE INVENTION

The invention provides methods and compositions for detecting a mutation in a target gene in a sample or a fraction thereof, including, in certain examples, a fraction that includes circulating tumor DNA. The methods can include a tiling PCR reaction, for example a one-sided multiplex tiling reaction. Virtually any type of mutation can be detected with the methods and compositions. In certain embodiments, gene fusions are detected. Improved PCR methods, especially for performing nested multiplex PCR reactions are provided.

Provided herein in one embodiment is a method for detecting a mutation in a target gene in a sample or fraction thereof, for example a cell-free fraction, such as a plasma fraction, that includes circulating tumor DNA, from a mammal. The method includes performing a multiplex PCR reaction using a tiled series of primers on DNA from the sample, and in illustrative embodiments, performing nested, multiplex PCR reactions first using a tiled series of outer primers to form outer primer target amplicons, and then using a tiled series of inner primers to form inner primer target amplicons from the outer primer target amplicons. The inner primer target amplicons are then subjected to nucleic acid sequencing, such as high-throughput nucleic acid sequencing, to detect the mutation. In illustrative embodiments, the mutation is a gene fusion.

Provided herein in another embodiment is a method for detecting a mutation in a target gene in a sample or a fraction thereof from a mammal. The method includes the following: forming an outer primer reaction mixture by combining a polymerase, deoxynucleoside triphosphates, nucleic acid fragments from a nucleic acid library generated from the sample, a series of forward target-specific outer primers and a plus strand reverse outer universal primer, where the nucleic acid fragments include a reverse outer universal primer binding site, where the series of forward target-specific outer primers includes 5 to 250 primers that bind to a tiled series of target specific outer primer binding sites spaced apart on the target gene by between 10 and 100 nucleotides; subjecting the outer primer reaction mixture to outer primer amplification conditions to generate outer primer target amplicons generated using primer pairs comprising one of the primers of the series of forward target-specific outer primers and the reverse outer universal primer; and analyzing the nucleic acid sequence of at least a portion of the outer primer target amplicons, thereby detecting a mutation in the target gene.

The method can further include before the analyzing step: forming an inner primer amplification reaction mixture by combining the outer primer target amplicons, a polymerase, deoxynucleoside triphosphates, a reverse inner universal primer and a series of forward target-specifics inner primers comprising 5 to 250 primers that bind to a tiled series of target-specific inner primer binding sites spaced apart on the target gene by between 10 and 100 nucleotides and each found on at least one outer primer target amplicon, configured to prime an extension reaction in the same direction as the series of outer target-specific primers; and subjecting the inner primer reaction mixture to inner primer amplification conditions to generate inner primer target amplicons generated using primer pairs comprising one of the forward target-specific inner primers and the reverse inner universal primer, where the amplicons whose nucleic acid sequences are analyzed include the inner primer target amplicons.

The analyzing step can include determining the nucleic acid sequence of at least a portion of the amplicons using massively parallel sequencing. The tiled series of target-specific outer and/or inner primer binding sites can be spaced apart on the target gene by between 10 and 75 nucleotides or 15 and 50 nucleotides, for example.

In yet another embodiment for detecting a mutation in a target gene in a sample or a fraction thereof from a mammal, the method includes the following steps: forming an inner primer reaction mixture by combining a nucleic acid sample, which can include nucleic acid fragments from a library constructed from a sample or a fraction thereof, especially a cell-free fraction thereof, or in nested PCR methods can be outer primer target amplicons, as well as a polymerase, nucleotides, such as deoxynucleoside triphosphates, a reverse inner universal primer and a series of forward target-specific inner primers comprising 5 to 1000, 5 to 500, or 5 to 250 primers that bind to a tiled series of target-specific inner primer binding sites spaced apart on the target gene by between 10 and 100 nucleotides and optionally each found on at least one outer primer target amplicon, optionally configured to prime an extension reaction in the same direction as the series of target-specific outer primers; and subjecting the inner primer reaction mixture to inner primer amplification conditions to generate inner primer target amplicons generated using primer pairs comprising one of the forward target-specific inner primers and the reverse inner universal primer, and analyzing the nucleic acid sequence of at least a portion of the inner primer target amplicons, thereby detecting a mutation in the target gene. Optionally the method can include before forming the inner primer reaction mixture, generating a series of outer primer amplicons according to the following steps: forming an outer primer reaction mixture by combining a polymerase, nucleotides, such as deoxynucleoside triphosphates, nucleic acid fragments from a nucleic acid library generated from the sample, a series of forward target-specific outer primers and a plus strand reverse outer universal primer, wherein the nucleic acid fragments comprise a reverse outer universal primer binding site, wherein the series of forward outer target-specific primers comprises 5 to 250 primers that bind to a tiled series of outer target primer binding sites spaced apart on the target gene by between 10 and 100 nucleotides; and subjecting the outer primer reaction mixture to outer primer amplification conditions to generate outer primer target amplicons generated using primer pairs comprising one of the primers of the series of forward target-specific outer primers and the reverse outer universal primer.

The target-specific inner primer binding sites, in one exemplary embodiment, overlap the target outer primer binding sites by between 5 and 20 nucleotides. In yet another embodiment the overlap can be 0 1, 2, 3, 4, 5, 6, 7, 8, 9, 10, 11, 12, 13, 14, or 15 nucleotides on the low end of the range, and 2, 3, 4, 5, 6, 7, 8, 9, 10, 11, 12, 13, 14, 15, 20, or 25 nucleotides on the high end of the range The reverse outer universal primer can include the same nucleotide sequence as the reverse inner universal primer. The tiled series of target-specific outer primer binding sites and the target-specific inner primer binding sites can be located on a target region of each of 1 to 100 target genes.

In yet another embodiment of the method at least 10%, 20%, 25%, 50%, 75%, 80%, 90%, 95%, 96%, 97%, 98%, 99%, and 100% of the outer primer target amplicons have overlapping sequences with at least one other of the outer primer target amplicon where the target region includes between 500 and 10,000 nucleotides and wherein the target region includes known mutations associated with a disease. The method can include outer primer target amplicons that have overlapping sequences covering at least one target region on each of 1 to 100 target genes, or 5 to 50 target genes, where each target region includes between 500 and 10,000 nucleotides, and where the target regions include known mutations associated with a disease. Each of at least 50% of the outer primer target amplicons and at least one of the inner primer target amplicons can have overlapping sequences.

The method can further include: forming a minus strand, outer primer reaction mixture by combining a polymerase, deoxynucleoside triphosphates, nucleic acid fragments from the nucleic acid library generated from the sample, a series of minus strand, forward target-specific outer primers and a minus strand, reverse outer universal primer, where the nucleic acid fragments include a minus strand, reverse outer universal primer binding site, where the series of minus strand, forward target-specific outer primers includes 5 to 250 primers that bind to a tiled series of minus strand, forward target-specific outer primer binding sites spaced apart on the target gene by between 10 and 100 nucleotides, wherein the minus strand forward target-specific outer primer binding sites are located on the minus strand of the strand targeted by the target-specific outer primer binding sites; subjecting the minus strand reaction mixture to amplification conditions to generate minus strand, target outer amplicons generated using primer pairs comprising one of the primers of the series of minus strand, forward target-specific outer primers and the minus strand, reverse outer universal primer; and analyzing the nucleic acid sequence of at least a portion of the minus strand, target outer amplicons, thereby detecting a mutation in the target gene.

The method can yet further include before the analyzing: forming a minus strand, inner primer amplification reaction mixture by combining the minus strand, outer primer target amplicons, a polymerase, deoxynucleoside triphosphates, a minus strand, reverse inner universal primer and a series of forward minus strand, target-specific inner primers comprising 5 to 250 primers that bind to a tiled series of minus strand, target-specific inner primer binding sites spaced apart on the target gene by between 10 and 100 nucleotides and each found on at least one minus strand, outer primer target amplicon, configured to prime an extension reaction in the same direction as the series of minus strand, target-specific outer primers; and subjecting the minus strand reaction mixture to minus strand, target-specific inner primer amplification conditions to form minus strand, inner primer target amplicons generated using primer pairs comprising one of the minus strand, forward target-specific inner primers and the minus strand, inner universal primer, where the amplicons whose nucleic acid sequences are analyzed include the minus strand, inner primer target amplicons. The minus strand, outer primer amplification conditions can be identical to the outer primer amplification conditions and the minus strand, inner primer amplification conditions can be identical to the inner primer amplification conditions. The method where the disease associated with the mutations is cancer.

In one embodiment of the method the presence of at least 10, 20, 25, 30, 40, 50 and 100 contiguous nucleic acids from the target gene and at least 10, 20, 25, 30, 40, 50 and 100 contiguous nucleotides from a region of the genome of the mammal not found on the target gene on the outer primer target amplicon and/or the inner primer target amplicon is indicative of a gene fusion comprising the target gene. The series of forward plus strand, target-specific outer primers includes at least one primer that binds to a target primer binding site that is between 25 and 150 nucleotides from a known fusion breakpoint for the target gene, and where the outer primer target amplicons include amplicons that are at least 150 nucleotides long.

The method detects a gene fusion from at least one, or at least two, fusion partner gene selected from the group consisting of AKT1, ALK, BRAF, EGFR, HER2, KRAS, MEK1, MET, NRAS, PIK3CA, RET, and ROS1 and where the series of target-specific outer primers includes at least one primer that binds to a target primer binding site that is between 25 and 150 nucleotides from a known fusion breakpoint for each of the target genes, and where the outer primer target amplicons include amplicons that are at least 150 nucleotides long. The gene fusion includes a chromosomal translocation from a fusion partner gene selected from the group consisting of AKT1, ALK, BRAF, EGFR, HER2, KRAS, MEK1, MET, NRAS, PIK3CA, RET, and ROS1.

The series of forward target-specific outer primers and the series of forward target-specific inner primers of the method each include at least one primer that binds to a target primer binding site that is a target distance from a known fusion breakpoint for the target gene, and where the outer primer target amplicons include at least one amplicon that is as long as the target distance. The target gene is selected from AKT1, ALK, BRAF, EGFR, HER2, KRAS, MEK1, MET, NRAS, PIK3CA, RET, and ROS1. The target gene can include at least two fusion partner genes selected from the group consisting of AKT1, ALK, BRAF, EGFR, HER2, KRAS, MEK1, MET, NRAS, PIK3CA, RET, and ROS1, and the series of target-specific outer primers and the series of target-specific inner primers each include between 5 and 250 primers and each binds to at least one target region on one of the at least two fusion partner genes, and where at least one primer binds to a target binding sequence that is a target distance from a known fusion breakpoint for each of the at least two fusion partner genes, and where the outer primer target amplicons for each of the at least two fusion partner genes include at least one amplicon that is as long as the target distance.

The series of target-specific outer primers and the series of target-specific inner primers can each include at least one primer that binds to a target binding sequence that: is between 25 and 150 nucleotides from a known fusion breakpoint for each of the target genes, and where the outer primer target amplicons include amplicons that are at least 150 nucleotides long that span a known genetic fusion breakpoint; is between 25 and 100 nucleotides from a known fusion breakpoint for each of the target genes, and where the outer primer target amplicons include amplicons that are at least 100 nucleotides long that span a known genetic fusion breakpoint; or is between 25 and 50 nucleotides from a known fusion breakpoint for each of the target genes, and where the outer primer target amplicons include amplicons that are at least 50 nucleotides long that span a known genetic fusion breakpoint.

The target-specific outer primer amplification conditions of the method include at least 5 PCR cycles having a target-specific outer primer annealing step of between 30 and 120 minutes or between 60 and 90 minutes, at between 58C and 72C.

The method can include two sets of target-specific outer primer amplification conditions where a first set of between 2 and 10 PCR cycles with an outer primer annealing step of between 30 and 120 minutes at between 58C and 65C and a second set of between 5 and 50 PCR cycles with a target-specific outer primer annealing step of between 30 and 120 minutes at between 68C and 72C. The highest Tm of the set of target-specific outer primers can be 2 to 10 degrees below the annealing temperature. The annealing can be performed in a combined annealing/extension step.

Provided is a further embodiment of the method for detecting a mutation in a target gene in a sample, or a fraction thereof from a mammal, where the target-specific outer primer amplification conditions include at least 5 PCR cycles having a target-specific outer primer annealing step of between 30 and 120 minutes, or between 60 and 90 minutes long, at between 58C and 72C. The target-specific outer primer amplification conditions can include a first set of between 2 and 10 PCR cycles with a target-specific outer primer annealing step of between 30 and 120 minutes at between 58C and 65C and a second set of between 5 and 50 PCR cycles with a target-specific outer primer annealing step of between 30 and 120 minutes at between 68C and 72C. The highest Tm of 50%, 75%, 90%, 95% or all of target-specific outer primers can be between 1, 2, 3, 4, 5, 6, 7, 8, 9, 10, 11, 12, 13, 14, 15, or 20 degrees C on the low end of the range and 2, 3, 4, 5, 6, 7, 8, 9, 10, 11, 12, 13, 14, 15, 20, or 25 degrees C on the high end of the range, below the annealing temperature used for the amplification (e.g. PCR) reaction. The highest Tm of the set of target-specific outer primers can be 2 to 10 degrees below the annealing temperature. The series of target-specific outer primers includes at least one primer that binds to a target binding sequence that is between 25 and 150 nucleotides from a known fusion breakpoint for the target gene and the annealing can be performed in a combined annealing/extension step.

Provided in another embodiment is a method for amplifying a target nucleic acid region *in vitro.* The method can include the following: forming a reaction mixture by combining a polymerase, deoxynucleoside triphosphates, nucleic acid fragments from a library, a first pool of a plurality of target-specific primers and a first reverse universal primer, where the nucleic acid fragments of the library include a universal reverse primer binding site, and where the plurality of target-specific primers includes 5 to 250 primers that are capable of binding to a tiled series of primer binding sites that are spaced apart on the target nucleic acid region by between 10 and 50 nucleotides; and subjecting the reaction mixture to amplification conditions to form amplicons of 100 to 200 nucleotides in length, where the amplification conditions include an annealing step of between 30 and 120 minutes at between 58C and 72C, thereby amplifying the target nucleic acid region. The method of target-specific primer amplification can include the at least 5 PCR cycles having a target-specific outer primer annealing step of between 60 and 90 minutes at between 58C and 72C.

The method can further include target-specific primer amplification conditions where a first set of between 2 and 10 PCR cycles with a target-specific outer primer annealing step of between 30 and 120 minutes at between 58C and 65C and a second set of between 5 and 50 PCR cycles with a target-specific outer primer annealing step of between 30 and 120 minutes at between 68C and 72C. The highest Tm of 50%, 75%, 90%, 95% or all of target-specific outer primers can be between 1, 2, 3, 4, 5, 6, 7, 8, 9, 10, 11, 12, 13, 14, 15, or 20 degrees C on the low end of the range and 2, 3, 4, 5, 6, 7, 8, 9, 10, 11, 12, 13, 14, 15, 20, or 25 degrees C on the high end of the range, below the annealing temperature used for the amplification (e.g. PCR) reaction. The highest Tm of the set of target-specific primers can be 2 to 10 degrees below the annealing temperature. The annealing can be performed in a combined annealing/extension step.

Provided in a further embodiment is a method for detecting a fusion involving a target gene in a sample or a fraction thereof from a mammal. The method includes: subjecting nucleic acids in the sample to a one-sided PCR tiling reaction across a target region of the target gene to generate outer target amplicons, where the tiling reaction is performed using a reverse outer universal primer and 5 to 250 forward target-specific outer primers that bind to a tiled series of outer target primer binding sites spaced apart on the target region of the target gene by between 10 and 100 nucleotides; and analyzing the nucleic acid sequence of at least a portion of the target amplicons, thereby detecting a mutation in the target gene. The method further includes performing a second one-sided PCR tiling reaction by amplifying the outer target amplicons using a reverse inner universal primer and a series of forward target-specific inner primers comprising 5 to 250 primers that bind to a tiled series of target inner primer binding sites spaced apart on the target region of the target gene by between 10 and 100 nucleotides and each found on at least one outer primer target amplicon, to generate inner forward target amplicons, where the forward target-specific inner primers are configured to prime an extension reaction in the same direction as the series of outer target-specific primers, and where the target amplicons whose nucleic acid sequences are analyzed include the inner forward target amplicons.

The target-specific inner primer binding sites of the method can overlap the target-specific outer primer binding sites by between 5 and 20 nucleotides. The target region includes a region of the target gene known to be involved in gene fusions. The tiled series of target-specific outer primer binding sites can be spaced apart on the target region by between 10 and 75, or 15 and 50, nucleotides. The tiled series of target-specific outer primer binding sites and the target-specific inner primer binding sites is selected on a target region of each of 2 to 50 target genes.

Other features and advantages of the disclosed inventions will be apparent from the following detailed description and from the claims.

## BRIEF DESCRIPTION OF THE DRAWINGS

FIG. 1: Graphical representation of gene fusion spikes, 160 bp, across a gene fusion.
FIG. 2: Graphical representation of artificially synthesized 160 bp gene fusion spikes wherein the gene fusion lies between the "partner" first gene and the "target" second gene with different portions of each gene.

FIG. 3: Graphical representation of target specific primers tiled in consecutive 30 bp windows grouped in order to select inner + outer primers for pooling in a One-Sided nested multiplex PCR method.

FIG. 4: Graphical representation of primer design pools for each outer plus strand, inner plus strand, outer minus strand and inner minus strand primer sets for a selected Tiling Target.

FIG. 5: Graphical representation of data analysis starting with reading amplified reads for the inner primers when using a One-Sided nested multiplex PCR method with target specific tiled primers.

FIGS. 6A-6B: Diagrams of PCR methods with target specific tiled primers are depicted. FIGS. 6A-6B illustrates a One-Sided nested multiplex PCR method with target specific primers in which the initially amplified outer primer amplicon (FIG. 6A, PCR No. 1) is the template for the second round of Nested PCR with the inner primer (FIG. 6B, PCR No. 2).

FIGS. 7A-7B: Illustrate an experimental workflow for a One-Sided nested multiplex PCR method with target specific tiled primers from library preparation and a first amplification round (FIG. 7A, PCR No. 1), a second amplification round (PCR No. 2) through NGS sequencing and sequencing analysis (FIG. 7B).

FIGS. 8A-8C: Graphical representation of the NGS sequencing depth of read (DOR) for the sequenced TP53 gene amplicons resulting from One-Sided nested multiplex PCR methods with target specific tiled primers. FIG. 8A illustrates DOR for amplicons sequenced that were generated using Plus strand target specific PCR primer pools. FIG. 8B illustrates DOR for amplicons sequenced that were generated using Minus strand target specific PCR primer pools. FIG. 8C illustrates the combined DOR for amplicons sequenced that were generated using both the Plus and Minus strand target specific PCR primers pools.

FIGS. 9A-9B: Two possible methods for detecting gene fusions are illustrated. FIG. 9A illustrates the One-Sided Nested Multiplex PCR method (Star 1 and Star 2) for a TPM4-ALK1 and the Two-Sided, one step multiplex PCR method (One Star) of a CD74 (partner gene) and ROS1 (target gene). FIG. 9B illustrates target specific tiled primers tiled across the ALK1 gene region where a fusion can occur.

FIGS. 10A-10C: Sequencing data of three gene fusion spikes is illustrated. FIG. 10A depicts wildtype ALK sequence read of the amplicon resulting from One-Sided nested multiplex PCR on the top track and the sequenced TPM4-ALK9 breakpoint sequenced from the One-Sided nested multiplex PCR derived amplicon on the lower track. FIG. 10B depicts wildtype ALK sequenced amplicon from One-Sided nested multiplex PCR on the top track and the sequenced NPM1-ALK9 breakpoint sequenced form the One-Sided nested multiplex PCR derived amplicon on the lower track. FIG. 10C depicts wildtype CD74 PCR amplified by the Two-Sided, one step multiplex PCR method with target specific tiled primers on the lower track sequencing read (no amplification and so no sequencing product) and the sequenced CD74-ROS1_13 breakpoint amplified by the Two-Sided, one step multiplex PCR method on the upper track sequencing read.

FIG. 11: Flow chart of analysis for detection of fusions or SNVs.

FIG. 12: Schematic of primer competition for wild type ALK amplification. In black, ALK sequence, Blue EML4 Sequence, Red Primers.

FIGS. 13A-13H: Table of exemplary primers for the STAR 1 (148 forward target-specific outer primers) and STAR 2 (148 forward, target-specific inner primers) for PCR amplification of ALK, chromosome 2, and ROS1, chromosome 6, target region (SEQ ID Nos. 1-296. Column heading are: Name (name of primer); Specific ("True" is unique sequence to the gene, "False" is not unique (provided for outer primer only as all inner primers are "True")); bp (base pair no); Start (start of the nucleotide primer binding sequence on the gene); Tm (bound primer melting temperature); SEQ ID NO. (sequence listing ID number of the primer); and Distance (Distance between the start of the outer primer and the start of the inner primer).

FIG. 14: Graphical representation showing the spikes design of four different gene fusion pairs, all spikes with same breakpoints but different proportion of target and partner genes.

FIG. 15: Graphical representation showing the two different approaches for detecting gene fusions, Star1-Star2 and OneStar.

FIG. 16: Graphical representation of the location of 4 of the forward primers, as well as their respective amplicons with respect to a gene-fusion breakpoint of ALK:TPM4.

FIG. 17: Graphical representation of the relative location of forward inner primers 2, 3, and 4 with respect to the template fusion spike molecules.

FIG. 18A: Graphical representation of tiling multiple targets of various lengths with a series of forward target specific primers. Length of target insert, without adapters, is indicated within the parenthesis.

FIG. 18B: Graph with a 1 Stage Annealing cycles spectra of tagged primer fluorescence vs amplicon length.

FIG. 18C: Graph with a 2 Stage Annealing cycles spectra of tagged primer fluorescence vs amplicon length.

FIG. 19A: Graphical representation of the percent product produced by the ammplification of 8F9+5R4_RSQ Template, a 117bp target insert, with a series of primers using 30, 60 and 90 minute annealing cycles.

FIGS. 19B: Graphical representation of the percent product produced by the ammplification of 8F9+5R4_RSQ Template, a target 121bp target insert, with a series of primers using 30, 60 and 90 minute annealing cycles.

FIG. 19C: Graphical representation of the percent product produced by the ammplification of 8F9+5R4_RSQ Template, a 121bp target insert, with a series of primers using a 90 minute annealing cycle and two different master mix compositions.

FIG. 19D: Graphical representation of the percent product produced by the ammplification of 8F9+5R4_RSQ Template; a 232bp target insert, using a series of primers with a 90 minute, 60 minute and 30 minute annealing cycle.

The above-identified figures are provided by way of representation and not limitation.

## DETAILED DESCRIPTION OF THE INVENTION

Provided herein in one illustrative embodiment is a strategy for mutation detection in circulating nucleic acids that utilizes multiplex PCR. The method in illustrative embodiments, can be used to scan a known cancer-related gene for known or unknown mutations and/or it can be used to detect gene fusions. The multiplex PCR is performed with primers that bind to a tiled series of binding sites on a target region of a target gene (i.e. the primers are tiled across the gene). The target region can be a region where a mutation is suspected, believed or known to occur. The multiplex PCR is typically followed by sequencing and bioinformatics analysis. For example, PCR primers can be tiled across an entire region where a cancer-related gene fusion is known to occur from prior analysis. In this approach, the bioinformatics analysis can identify sequence reads that map to two genes (the target gene and the fusion partner), thereby detecting a gene fusion event. In illustrative embodiments, methods of this embodiment of the invention are PCR methods that utilize one-sided primer tiling, especially nested, one-sided primer tiling. Improvements to such one-sided tiling multiplex PCR methods are provided that provider larger amplicons with higher yield and more specificity.

Accordingly, a method according to one embodiment of the invention is provided for detecting a mutation in a target gene in a sample or a fraction thereof from a mammal. In certain illustrative embodiments, the mutation is a gene fusion. The method can include the following steps: forming a one-sided multiplex PCR tiling reaction mixture for amplifying a nucleic acid library generated from a sample or a fragment thereof. In illustrative embodiments, the one-sided multiplex PCR amplification, is a nested, one-sided multiplex PCR amplification. The one-sided multiplex PCR reaction uses a series of forward primers that bind to a tiled series of binding sites on a target region of a target gene. In illustrative embodiments, the target gene is a cancer-related gene, such as a gene known to be a gene fusion partner in a fusion event that is a cancer driver. The reaction mixture is subjected to amplification conditions and the nucleic acid sequence of at least a portion of the amplicons generated are analyzed to determine their nucleic acid sequence.

In a more specific example, a method of this embodiment for detecting a mutation in a target gene can include the following steps: forming an outer primer reaction mixture by combining a polymerase, deoxynucleoside triphosphates, nucleic acid fragments from a nucleic acid library generated from the sample, a series of forward target-specific outer primers and a first strand reverse outer universal primer, wherein the nucleic acid fragments comprise a reverse outer universal primer binding site, wherein the series of forward target-specific outer primers comprises 5 to 250 primers that bind to a tiled series of outer target primer binding sites spaced apart on the target gene by between 10 and 100 nucleotides; subjecting the outer primer reaction mixture to outer primer amplification conditions to generate outer primer target amplicons generated using primer pairs comprising one of the primers of the series of forward target-specific outer primers and the reverse outer universal primer; and analyzing the nucleic acid sequence of at least a portion of the outer primer target amplicons, thereby detecting a mutation in the target gene.

In certain embodiments, methods provided herein are methods for detecting a gene fusion, especially a gene fusion associated with cancer. Such fusions can include at least 1, 2, 3, 4, 5, 6, 7, 8, 9, 10, or all of the following fusion partner genes: AKT1, ALK, BRAF, EGFR, HER2, KRAS, MEK1, MET, NRAS, PIK3CA, RET, and ROS1. Primers used in methods provided here for detecting fusions, can include a series of between 3, 4, 5, 6, 7, 8, 9, 10, 11, 12, 13, 14, 15, 20, 25, 50, 75, 100, 125, 150, 200 or 250, 500, 1000, 5000, 10,000, 20,000, 25,000, 50,0000, 60,000, or 75,000 primers on the low end of the range and can include a series of between 5, 6, 7, 8, 9, 10, 11, 12, 13, 14, 15, 20, 25, 50, 75, 100, 125, 150, 200 or 250, 500, 1000, 5000, 10,000, 20,000, 25,000, 50,0000, 60,000, 75,000, or 100,000 primers on the high end of the range, wherein between 1, 2, 3, 4, 5, 6, 7, 8, 9, 10, 15, 20, 25, 50, 75, 100, 150, 200, 250, 300, 400, 500, 750, 1000, 2500, 5000, or 10,000 of the primers on the low end of the range and 2, 3, 4, 5, 6, 7, 8, 9, 10, 15, 20, 25, 50, 75, 100, 150, 200, 250, 300, 400, 500, 750, 1000, 2500, 5000, 10,000 or 25,000 of the primers on the high end of the range, bind to a target binding sequence that is between 25 and 150 nucleotides from a known fusion breakpoint for each of the target genes, and wherein the amplicons produced by the method includes amplicons that are on average between 25 and 200 nucleotides in length, in certain embodiments between 50 and 150 nucleotides in length. In illustrative embodiments, the gene fusion includes a chromosomal translocation from a fusion partner gene selected from the following: AKT1, ALK, BRAF, EGFR, HER2, KRAS, MEK1, MET, NRAS, PIK3CA, RET, and ROS1. In some embodiments, methods provided herein that include improved PCR reaction mixture and cycling conditions, and One-Sided nested multiplex PCR using tiled primers including any of the illustrative primer site spacings provided herein, are specifically designed to detect gene fusions.

In methods provided herein for detection fusions, a target region can be for example, between 0.5 kb and 10 kb for a target gene and in certain embodiments, between 0.5kb and 5kb for a target gene. As disclosed in Example 1, a target region for detecting fusion by mapping public database (e.g. COSMIC) fusion transcripts to genomic coordinates (i.e. translocations), but preferably uses exon boundaries and reported fusions. Using this approach, a target region to be tiled would require tiling < 3.6 kb of sequence for each of three exemplary targets: ALK, ROS1 and RET. Table 2 of Example 1 sets out specific, exemplary target regions for known fusion targets ALK, ROS1, and RET.

A sample analyzed in methods of the present invention, in certain illustrative embodiments, is a blood sample, or a fraction thereof. Methods provided herein, in certain embodiments, are *in vitro* methods. Methods provided herein, in certain embodiments, are specially adapted for amplifying DNA fragments, especially tumor DNA fragments that are found in circulating tumor DNA (ctDNA). Such fragments are typically about 160 nucleotides in length.

It is known in the art that cell-free nucleic acid (cfNA), e.g cfDNA, can be released into the circulation via various forms of cell death such as apoptosis, necrosis, autophagy and necroptosis. The cfDNA, is fragmented and the size distribution of the fragments varies from 150-350 bp to > 10000 bp. (see Kalnina et al. World J Gastroenterol. 2015 Nov 7; 21(41): 11636-11653). For example the size distributions of plasma DNA fragments in hepatocellular carcinoma (HCC) patients spanned a range of 100-220 bp in length with a peak in count frequency at about 166bp and the highest tumor DNA concentration in fragments of 150-180 bp in length (see: Jiang et al. Proc Natl Acad Sci USA 112:E1317-E1325).

In an illustrative embodiment the circulating tumor DNA (ctDNA) is isolated from blood using EDTA-2Na tube after removal of cellular debris and platelets by centrifugation. The plasma samples can be stored at -80oC until the DNA is extracted using, for example, QIAamp DNA Mini Kit (Qiagen, Hilden, Germany), (e.g. Hamakawa et al., Br J Cancer. 2015; 112:352-356). Hamakava et al. reported median concentration of extracted cell free DNA of all samples 43.1 ng per ml plasma (range 9.5-1338 ng ml/) and a mutant fraction range of 0.001-77.8%, with a median of 0.90%.

In certain illustrative embodiments the sample is a tumor. Methods are known in the art for isolating nucleic acid from a tumor and for creating a nucleic acid library from such a DNA sample given the teachings here. Furthermore, given the teachings herein, a skilled artisan will recognize how to create a nucleic acid library appropriate for the methods herein from other samples such as other liquid samples where the DNA is free floating in addition to ctDNA samples.

Methods of the present invention in certain embodiments, typically include a step of generating and amplifying a nucleic acid library from the sample (i.e. library preparation). The nucleic acids from the sample during the library preparation step can have ligation adapters, often referred to as library tags or ligation adaptor tags (LTs), appended, where the ligation adapters contain a universal priming sequence, followed by a universal amplification. In an embodiment, this may be done using a standard protocol designed to create sequencing libraries after fragmentation. In an embodiment, the DNA sample can be blunt ended, and then an A can be added at the 3' end. A Y-adaptor with a T-overhang can be added and ligated. In some embodiments, other sticky ends can be used other than an A or T overhang. In some embodiments, other adaptors can be added, for example looped ligation adaptors. In some embodiments, the adaptors may have tag designed for PCR amplification.

Primer tails can improve the detection of fragmented DNA from universally tagged libraries. If the library tag and the primer-tails contain a homologous sequence, hybridization can be improved (for example, melting temperature (Tm) is lowered) and primers can be extended if only a portion of the primer target sequence is in the sample DNA fragment. In some embodiments, 13 or more target specific base pairs may be used. In some embodiments, 10 to 12 target specific base pairs may be used. In some embodiments, 8 to 9 target specific base pairs may be used. In some embodiments, 6 to 7 target specific base pairs may be used.

Since illustrative embodiments of the methods provided herein utilize a one-sided multiplex PCR approach, during library preparation one or more universal primer binding sites (e.g. reverse outer universal primer binding sites, reverse inner universal primer binding sites) are typically included on adapters ligated to nucleic acid fragments of the library. Furthermore, sequencing primer binding sites for subsequence nucleic acid sequence determination can be added during the library preparation step, or any subsequent step, as will be recognized by a skilled artisan. Additionally, unique or semi-unique identifiers (UIDs) can be added to isolated nucleic acids from the sample during a library preparation step.

Many kits and methods are known in the art for generation of libraries of nucleic acids that include universal primer binding sites for subsequent amplification, for example clonal amplification, and for subsequence sequencing. To help facilitate ligation of adapters library preparation and amplification can include end repair and adenylation (i.e. A-tailing). Kits especially adapted for preparing libraries from small nucleic acid fragments, especially circulating free DNA, can be useful for practicing methods provided herein. For example, the NEXTflex Cell Free kits available from Bio Scientific (Austin, TX) or the Natera Library Prep Kit (further discussed in example 9, Natera, San Carlos, CA). However, such kits would typically be modified to include adaptors that are customized for the amplification and sequencing steps of the methods provided herein. Adaptor ligation can be performed using commercially available kits such as the ligation kit found in the Agilent SureSelect kit (Agilent, CA).

Accordingly, as a result of library preparation, a nucleic acid library is generated that includes nucleic acid fragments that have a reverse outer universal primer binding site and optionally a reverse inner universal primer binding site for nested embodiments, as discussed herein. Such universal primer binding sites are recognized and typically complementary to universal primers, which are included in the reaction mixtures of illustrative embodiments of methods provided herein. The Examples provided herein, illustrate the use of universal primer binding sites and universal primers.

A series of primers used for the present invention, for example reverse or forward inner or outer target-specific primers in certain embodiments include between 5, 10, 15, 20, 25, 50, 100, 125, 150, 250, 500, 1000, 2500, 5000, 10,000, 20,000, 25,000, or 50,000 on the low end of the range and 15, 20, 25, 50, 100, 125, 150, 250, 500, 1000, 2500, 5000, 10,000, 20,000, 25,000, 50,000, 60,000, 75,000, or 100,000 primers on the upper end of the range, that each bind to one of a series of outer target primer binding sites that are tiled across a target region of a target gene. In the present invention, when a series of primers are tiled across a target gene region each primer of the series binds to a different binding site of the series of primer binding sites, wherein the primer binding sites within a series are typically spaced apart by between 1 and 100 nucleotides and are capable of priming a series of primer extension reactions on a nucleic acid strand in the same 5' to 3' direction wherein a primer extension reaction product from a first primer of a series overlaps the region of the target gene that is bound by at least one next primer in the series.

The primer binding sites in a series can include at least 2 primer binding sites that are spaced apart by between 10, 15, 20, 25, 30, 40, 50, 60, 70, 75, 80, 90, 100, 125, 150, 175, or 200 nucleotides on the low end of the range, and 10, 15, 20, 25, 30, 40, 50, 60, 70, 75, 80, 90, 100, 125, 150, 175, 200, or 250 nucleotides on the high end of the range. In certain embodiments, the primer binding sites in a series includes at least 2, 3, 4, 5, 6, 7, 8, 9, 10, 15, 20, 25, 30, 40, 50, 100, 125, 150, 175, 200, 250, 500, 1000, 1500, 10000, 1500, 2000, 2500, 3000, 4000, 5000, 10,000, 15,000, 20,000, 25,000, or 50,000 primers and primer binding sites on the low end, and 3, 4, 5, 6, 7, 8, 9, 10, 15, 20, 25, 30, 40, 50, 100, 125, 150, 175, 200, 250, 500, 1000, 1500, 10000, 1500, 2000, 2500, 3000, 4000, 5000, 10,000, 15,000, 20,000, 25,000, 50,000, 60,000, 70,000, 75,000 or 100,000 primers and primer binding sites on the high end of the range. In certain illustrative embodiments, the series of primer binding sites span an entire target region of a gene of interest and are spaced apart by between 2, 3, 4, 5, 6, 7, 8, 9, 10, 15, 20, 25, 30, 40, 50, 75, 100, 125, 150, 175, 200, or 250 nucleotides on the low end and between 3, 4, 5, 6, 7, 8, 9, 10, 15, 20, 25, 30, 40, 50, 75, 100, 125, 150, 175, 200, 250, or 500 on the high end.

Such primer binding site spacing can be chosen in certain illustrative examples, based on the expected amplicon sizes produced by the series of primers that bind the tiled binding sites and/or based on the amplification conditions used for the tiling PCR. For example, the tiling primer binding site spacing can be between 10%, 20%, 25%, 30%, 40%, 50%, 60%, 70%, 75%, 80%, 85%, or 90% of the expected, empirical, or actual average amplicon length, on the low end of the range, and 20%, 25%, 30%, 40%, 50%, 60%, 70%, 75%, 80%, 85%, 90%, 95% or 100% on the high end of the range. In certain illustrative embodiments, the tiling primer binding site spacing is at between 25% and 90% of the average actual amplicon length of amplicons generated during a method of the invention provided herein. In another illustrative embodiment, the tiling primer binding site spacing is at between 25% and 50% of the average actual amplicon length of amplicons generated during a method of the invention provided herein. In yet another illustrative embodiment, the tiling primer binding site spacing is at between 50% and 90% of the actual average amplicon length of amplicons generated during a method of the invention provided herein. In another embodiment provided herein, the tiling primer binding site spacing is less than the average length of amplicons generated during a method provided herein.

Thus, in methods provided herein for detecting gene fusions, the above primer ranges will help to assure that an amplicon spans a fusion breakpoint by a distance that is less than or equal to the high end of the range provided. For example, in certain illustrative embodiments for fusion detection, a primer binding site will be within a distance no greater than the average amplicon length from a fusion breakpoint. In other illustrative embodiments for fusion detection, a primer binding site will be within a distance no greater than 75% of the average amplicon length from a fusion breakpoint. The spacing or distance between primer binding sites when discussed herein, is based on the distance between the 3' end of a first primer binding site and the 5' end of a second primer binding site that is bound by a primer that primes in the same direction as, and downstream from a primer that binds the first primer binding site.

In certain illustrative examples, the primer binding sites are spaced apart on the target region of the target gene by between 25 and 200 nucleotides. In certain illustrative examples, the primer binding sites are spaced apart on the target region of the target gene by between 25 and 150 nucleotides. In certain illustrative examples, the primer binding sites are spaced apart on the target region of the target gene by between 10 and 100 nucleotides. In other illustrative examples, the tiled series of target-specific outer primer binding sites are spaced apart on the target gene by between 10 and 75 nucleotides. In other illustrative methods, the tiled series of target-specific outer primer binding sites are spaced apart on the target region of the target gene by between 15 and 50 nucleotides. The primer binding sites discussed in this section related to primer spacing can be any of the target-specific primer binding sites of methods of the invention. For example, the spacing discussed can be for the target-specific outer or inner primer binding sites in either the plus or minus strand.

A method provided herein, in illustrative embodiments, is a One-Sided nested multiplex PCR method, also referred to herein as a One-Sided nested multiplex PCR method. As such, the method typically includes an amplification reaction that uses nested primers (i.e. an inner primer as a member of a set of inner primers and an outer primer as a member of a set of outer primers).

Example 3 herein provides details regarding an approach to designing tiled primers for use in methods provided herein. The primers bind a tiled series of primer binding sites spaced across a target region of a target gene (i.e. gene of interest). As exemplified, primers can be designed for plus and/or minus strands of a target gene region with melting temperature (Tm) optimums of between 55C and 65C, for example 58C and 61C (FIGS 4-6). Primer designed with relaxed (deltaG -6, deltaG-5, deltaG-4) or strict (deltaG -3) primer sets can be designed. The relaxed set will typically have more windows covered with primers but can also contain potentially harmful primers that cause primer-dimers. Primers can be ordered from any company supplying primers, such as IDT (Integrated DNA Technologies, Inc., San Diego, CA). The primers can be designed with or without tags. For example, outer primers can be designed without a tag and inner primers can be designed with a tag, such as, but not limited to, ACACGACGCTCTTCCGATCT (SEQ ID NO: 297).

Primer designs can be generated with Primer3 (Untergrasser A, Cutcutache I, Koressaar T, Ye J, Faircloth BC, Remm M, Rozen SG (2012) "Primer3 - new capabilities and interfaces." Nucleic Acids Research 40(15):e115 and Koressaar T, Remm M (2007) "Enhancements and modifications of primer design program Primer3." Bioinformatics 23(10): 1289-91) source code available at primer3.sourceforge.net). Primer specificity can be evaluated by BLAST and added to existing primer design pipeline.

Plus (+) strand primers can be generated for selected target regions. Target region sequences can be targeted in windows every 20-50 bp. Each primer design window can be 20-40 bp long from the window start. Primers can be searched in two consecutive windows for pairing nested Outer and Inner primers. Outer primers can be designed that target the right most, 5' (or leftmost on minus strand) coordinate of each region using Primer3. The rationale for using windows is that an inner primer will be selected from every second window, and a matching outer primer (following rules described below) will be selected either from the same or previous (3') window but not farther away. Primers can be generated using RunPrimer3.java with one_sided=true option. This mode of the program generates only one set of primers without generating a paired minus primer.

Primer specificities can be determined using the BLASTn program from the ncbi-blast-2.2.29+ package. The task option "blastn-short" can be used to map the primers against hg19 human genome. Primer designs can be determined as "specific" if the primer has less than 100 hits to the genome and the top hit is the target complementary primer binding region of the genome and is at least two scores higher than other hits (score is defined by BLASTn program). This can be done in order to have a unique hit to the genome and to not have many other hits throughout the genome.

Primers can be grouped on each consecutive window to inner + outer pairs (see e.g., FIG. 5) with the following rules:

a) There is an Outer/Inner primer pair every tiled window (30 bp window illustrated (see e.g., FIG. 3)
b) From every second window, a specific inner primer can be tried based on output order by Primer3.
c) A primer can be skipped if it overlaps >50% with any other inner primer that was already selected.
d) An outer primer can be attempted to be identified such that:

  a. Outer primers from the current and previous window (the one from inner primer) are tried to find a primer such that:

    1. The first base of the primer is before the first base of the inner primer (or after for minus primers)
    2. The part of the inner primer that doesn't overlap with the outer primer is between 5 and 20 bases
    3. The Outer primer is specific
    4. Primers are tested in the order given by Primer3 output

  b. If (i) fails, try same as (i) except Outer primer was non-specific
  c. If (ii) fails, try same as (i) except distance was 3 to 40 bases
  d. If (iii) fails, try same as (i) except distance was 3 to 40 bases, and Outer primer was non-specific
  e. If (iv) fails, try same as (i) except distance was 40 to 100 bases
  f. If (v) fails, try same as (i) except distance was 40 to 100 bases, and Outer primer was non-specific

e) None or minimal interactions with other primers (was tested separately for Inner and Outer primers)
f) Inner primers have no interactions with the plus strand tag sequence ACACGACGCTCTTCCGATCT" (SEQ ID NO: 297)
g) Outer primers have no interactions with the minus strand tag sequence AGACGTGTGCTCTTCCGATCT (SEQ ID NO: 298)
h) The final selected primers can be visualized in IGV (Robinson et al., Integrative Genomics Viewer. Nature Biotechnology 29, 24-26 (2011) and UCSC browser (Sugnet et al., The human genome browser at UCSC. Genome Res. 2002 Jun;12(6):996-1006) using bed files and coverage maps for validation.

Primer sets with relaxed and strict deltaG thresholds (-6 vs -3) can be designed for each of 58 and 61 Tm settings (including plus/minus strand and inner/outer primers, e.g., 4 pools per design). The final set of selected primers can be assessed to see their coverage of each target region on each strand, and on the combination of each strand (termed as "both"). Acceptable primer sets are then used in methods provide herein, for nested multiplex PCR.

Example 4 herein provides details regarding an approach for identifying target regions and designing tiled primers for use in methods for detection of mutations in cancer-related genes, such as genes known to have various mutations that are cancer driver mutations, such as the TP53 gene. Primer design parameters and an illustrative example of settings for those parameters are provided in Example 4 Tables 9-11.

As discussed herein, for nested one-sided PCR methods provided herein, inner and outer primers are used. Accordingly, in a specific embodiment, a method of the present invention further includes before the analyzing, forming an inner primer amplification reaction mixture by combining the outer primer target amplicons, a polymerase, nucleotides such as deoxynucleoside triphosphates, a reverse inner universal primer and a series of forward target-specifics inner primers comprising 5 to 250 primers that bind to a tiled series of target-specific inner primer binding sites spaced apart on the target gene by between 10 and 100 nucleotides and each found on at least one outer primer target amplicon, configured to prime an extension reaction in the same direction as the series of outer target-specific primers; and subjecting the inner primer reaction mixture to inner primer amplification conditions to generate inner primer target amplicons generated using primer pairs comprising one of the forward target-specific inner primers and the reverse inner universal primer, wherein the amplicons whose nucleic acid sequences are analyzed comprise the inner primer target amplicons. In certain embodiments, the target-specific inner primer binding sites overlap a matched target-specific outer primer binding sites by between 0, 1, 2, 3, 4, 5, 6, 7, 8, 9, 10, 11, 12, 13, 14, or 15 nucleotides on the low end of the range, and 2, 3, 4, 5, 6, 7, 8, 9, 10, 11, 12, 13, 14, 15, 20, or 25 nucleotides on the high end of the range. In one illustrative embodiment, target-specific inner primer binding sites overlap at least one target-specific outer primer binding site by between 5 and 20 nucleotides. In yet another illustrative embodiment the target-specific inner primer binding sites do not overlap the outer primer binding sites. For one-sided methods, the universal primer on the opposite side of the PCR amplicon can be the same or different for the PCR reaction with the inner primers versus the PCR reaction with the outer primers.

Methods of the present invention, in certain embodiments, include forming an amplification reaction mixture. Any of the reaction mixtures provided herein, themselves forming in illustrative embodiments, a separate aspect of the invention. A reaction mixture of the present invention typically is formed by combining a polymerase, nucleotides such as deoxynucleoside triphosphates, nucleic acid fragments from a nucleic acid library generated from a sample, especially a cell-free fraction of blood comprising circulating tumor DNA, and a series of primers. The series of primers can include a plus and/or minus strand forward target-specific outer primers and a plus and/or a minus strand reverse outer universal primer wherein the nucleic acid fragments comprise a reverse outer universal primer binding site, wherein the series of forward outer target-specific primers comprises 5 to 250 primers that bind to a tiled series of outer target primer binding sites spaced apart on the target gene by between 10 and 100 nucleotides and each target region comprises between 500 and 10,000 nucleotides. In yet further exemplary composition the series of primers can include a plus and/or minus strand forward target-specific inner primers and a plus and/or a minus strand reverse inner universal primer wherein the nucleic acid fragments comprise a reverse inner universal primer binding site, wherein the series of forward inner target-specific primers comprises 5 to 250 primers that bind to a tiled series of outer target primer binding sites spaced apart on the target gene by between 10 and 100 nucleotides and each target region comprises between 500 and 10,000 nucleotides. The compositions can include nucleic acid fragments directly derived from a ctDNA sample, that cross a gene fusion breakpoint.

An amplification reaction mixture useful for the present invention includes components known in the art for nucleic acid amplification, especially for PCR amplification. For example, the reaction mixture typically includes deoxynucleoside triphosphates, a polymerase, and magnesium. Polymerases that are useful for the present invention can include any polymerase that can be used in an amplification reaction especially those that are useful in PCR reactions. In certain embodiments, hot start Taq polymerases are especially useful. Amplification reaction mixtures useful for practicing the methods provided herein, such as K23 and AmpliTaq Gold master mix (Life Technologies, Carlsbad, CA), are provided as non-limiting examples in the Examples section provided herein. More details regarding PCR reaction mixtures are found in a further section herein.

Amplification (e.g. temperature cycling) conditions for PCR are well known in the art. The methods provided herein can include any PCR cycling conditions that result in amplification of target nucleic acids such as target nucleic acids from a library. Non-limiting exemplary cycling conditions are provided in the Examples section herein. More details regarding PCR cycling conditions are found in a further section herein.

An illustrative embodiment of the method of fusion detection provided herein applies a one-sided nested multiplex amplification of the ctDNA libraries using an exemplary Star1 and Star2 protocol. The Star1 PCR program is: 95C 10 min; 15x [95C 30 sec, 63C 10 min, 72C 2 min]; 72C 7 min, 4C hold. The Star2 PCR program is: 95C 10 min; 15x [95C 30 sec, 63C 10 min, 72C 2 min]; 72C 7 min, 4C hold.

An illustrative embodiment of the methods of the present invention utilize an extended annealing and/or extension and/or combined annealing/extension time after an initial denaturation step (e.g. 95C for 5 to 15 minutes) and cycling parameters that include a denaturing step (e.g. 95C for 15 to 120 seconds) the extended annealing step of between 30 and 240 minutes and optionally an extension step of between 70 and 75C (e.g. 72C) for 30 to 240 seconds. The annealing step is a step in a PCR cycle after a denaturation step and before an optional extension step. Optionally, the PCR has multiple stages (i.e multiple different sets of cycling parameters), for example the PCR can be a 2-stage PCR as demonstrated in Example 12 provided herein. Accordingly, in one embodiment provided herein is a method of the invention, wherein the amplification conditions, such as the target-specific outer primer amplification conditions, include at least 2, 3, 4, 5, 6, 7, 8, 9, 10, 11, 12, 13, 14, 15, 20, 25, or 30 PCR cycles having an annealing step of between 30, 35, 40, 45, 50, 55 or 60 minutes on the low end of the range and 35, 40, 45, 50, 55, 60, 120, 180, or 240 minutes on the high end of the range, at a temperature between 55, 56, 57, 58, 59, 60, 61, 62, 63, 64, or 65C on the low end of the range, and 60, 61, 62, 63, 64, 65, or 70C on the high end of the range. In an illustrative embodiment, the annealing step is between 30 and 120 minutes at between 58C and 72C. In related embodiments, the annealing step is between 60 and 90 minutes long at between 58C and 65C.

In related embodiments, the amplification conditions comprise a first set of between 2, 3, 4, 5, 6, 7, 8, 9, 10, 11, 12, 13, 14, or 15 cycles on the low end of the range and 3, 4, 5, 6, 7, 8, 9, 10, 11, 12, 13, 14, 15, 20, 25 or 30 cycles on the high end of the range, and a second set of between 2, 3, 4, 5, 6, 7, 8, 9, 10, 11, 12, 13, 14, 15, 20, or 25 cycles on the low end of the range and 3, 4, 5, 6, 7, 8, 9, 10, 11, 12, 13, 14, 15, 20, 25, 30, 35, 40, 50, or 60 cycles on the high end of the range. In an illustrative embodiment, the amplification conditions comprise 2 and 10 PCR cycles with an annealing step, such as a target-specific outer primer annealing step, of between 30 and 120 minutes at between 40 and 60C, such as between 58C and 65C and a second set of between 5 and 50 PCR cycles with a target-specific outer primer annealing step of between 30 and 120 minutes at between 55 and 75C, such as between 58C and 72C. In another embodiment, the highest Tm of 50%, 75%, 90%, 95% or all of primers of the set of target-specific and/or a universal primer, is between 1, 2, 3, 4, 5, 6, 7, 8, 9, 10, 11, 12, 13, 14, 15, or 20 degrees C on the low end of the range and 2, 3, 4, 5, 6, 7, 8, 9, 10, 11, 12, 13, 14, 15, 20, or 25 degrees C on the high end of the range, below the annealing temperature used for the amplification (e.g. PCR) reaction. In an illustrative embodiment, the Tm of at least 50% of the primers of the set of primers is 2 to 10 degrees below the annealing temperature used for the PCR reaction.

In these embodiments with an extended annealing or extension step, the extended step can also be a combined annealing/extension step. In some embodiments provided herein, embodiments that include any of the primer binding site spacing provided herein, are combined with embodiments that include any of the extended annealing and/or extension conditions provided herein.

One additional surprising result provided in Example 12 herein, is that a higher ionic strength PCR master mix (K23) produced significantly higher percent yields as compared to a commercial AmpliTaq Gold Master Mix (Life Technologies, Carlsbad, CA), and had greater selectivity with fewer side products due to amplification by shorter primers. Accordingly, provided herein in certain embodiments is a IX PCR reaction mixture wherein the ionic strength final concentration is between 75 and 1000 mM, 100 and 800 mM, 150 and 600 mM, and 200 and 400 mM..

There are many workflows that are possible when conducting PCR; some workflows typical to the methods disclosed herein are provided herein. The steps outlined herein are not meant to exclude other possible steps nor does it imply that any of the steps described herein are required for the method to work properly. A large number of parameter variations or other modifications are known in the literature, and may be made without affecting the essence of the invention.

In some embodiments, methods provided herein can be used to scan a target gene for mutations by performing tiled multiplex PCR across a target region known to be mutated in mammalian diseases, such as cancer. Accordingly, in certain embodiments, provided herein is a method for detecting a mutation in a target gene in a sample or a fraction thereof from a mammal, wherein the outer primer target amplicons optionally having overlapping sequences span a target region of the target gene, wherein the target region can include an entire gene, all the exons of a gene, or any fraction thereof. For example, between 0, 0.1, 0.25, 0.5, and 1.0k on the low end of the range, and 1.0, 2.5, 5, and 10k nucleotides in length on the high end of the range. The target region can include known mutations associated with a disease. Provided herein are a series of primers that are effective for tiling across all exons of the human p53 gene. For methods of scanning a target gene for mutations provided herein, the PCR method can be one-side (target-specific primers on one side (forward or reverse) and universal primer on the other side) or two-side (i.e. target specific primers on both sides). Example 4 provided herein, illustrates an example of such a method for detecting mutations of the TP53 gene. For example, for TP53, target regions can be found within exons 5 through 8, which contain the majority of its mutations in ovarian cancer (See Table 4). As illustrated, to assure complete tiling, primer target coverage can be tested with various read lengths (e.g. 50 bp, 75 bp, 100 bp, 125 bp, 150 bp, 175 bp, or 200 bp) excluding the length of the primers. As exemplified in Tables 4-8, ideally when considering both strands there is 100% coverage of a Target Region of a Target gene.

In certain examples of this embodiment, the outer primer target amplicons have overlapping sequences covering between 1, 2, 3, 4, 5, 6, 7, 8, 9, or 10 target region on the low end of the range, and 2, 3, 4, 5, 6, 7, 8, 9, 10, 15, 20, or 25 target regions on the high end of the range, on each of 1, 2, 3, 4, 5, 6, 7, 8, 9, 10, 15, 20, 25, 50 and 75 target genes on the low end of the range, and 2, 3, 4, 5, 6, 7, 8, 9, 10, 15, 20, 25, 50, 75, and 100 target genes on the high end of the range. In one illustrative embodiment, the outer primer target amplicons have overlapping sequences covering between 2 and 5 target regions on between 2 and 5 target genes. In another illustrative embodiment, the outer primer target amplicons have overlapping sequences covering 1 or 2 target regions on between 2 and 10 target genes.

In certain examples of this embodiment, the outer primer target amplicons and the inner primer target amplicons have overlapping sequences covering between 1, 2, 3, 4, 5, 6, 7, 8, 9, or 10 target regions on the low end of the range, and 2, 3, 4, 5, 6, 7, 8, 9, 10, 15, 20, or 25 target regions on the high end of the range, on each of 1, 2, 3, 4, 5, 6, 7, 8, 9, 10, 15, 20, 25, 50 and 75 target genes on the low end of the range, and 2, 3, 4, 5, 6, 7, 8, 9, 10, 15, 20, 25, 50, 75, and 100 target genes on the high end of the range. In one illustrative embodiment, the outer primer target amplicons and the inner primer target amplicons have overlapping sequences covering between 2 and 5 target regions on between 2 and 5 target genes. In another illustrative embodiment, the outer primer target amplicons have overlapping sequences covering 1 or 2 target regions on between 2 and 10 target genes.

In certain embodiments of the methods provided herein, a method for tiling PCR is performed on both strands in opposite directions. Accordingly, in one embodiment, the method further includes, in addition to forming a plus strand outer primer reaction mixture and subject that to plus strand amplification conditions, forming a minus strand, outer primer reaction mixture, and in some embodiments an minus strand, inner primer reaction mixture, and subjecting this/these minus strand reaction mixture(s) to amplification conditions (i.e. amplifying the target nucleic acid fragments), and analyzing the nucleic acid sequence of at least a portion of the minus strand, outer primer target amplicons, and in certain embodiments minus strand inner primer target amplicons. As will be understood, the teachings herein for the plus strand reaction mixture, amplification conditions, and sequence analysis apply to the minus strand just as they apply to the plus strand.

In certain embodiments of the method provided herein, at least a portion and in illustrative examples the entire sequence of an amplicon, such as an inner primer target amplicon for methods that include nested PCR reactions, is determined. Methods for determining the sequence of an amplicon are known in the art. Any of the sequencing methods known in the art, e.g. Sanger sequencing, can be used for such sequence determination. In illustrative embodiments high throughput next-generation sequencing techniques (also referred to herein as massively parallel sequencing techniques) such as, but not limited to, those employed in MYSEQ (Illumina), HISEQ (Illumina, San Diego CA), ION TORRENT (Life Technologies, Carlsbad, CA), GENOME ANALYZER ILX (Illumina), GS FLEX+ (ROCHE 454), can be used for sequencing the amplicons produced by the methods provided herein.

High throughput genetic sequencers are amenable to the use of barcoding (i.e., sample tagging with distinctive nucleic acid sequences) so as to identify specific samples from individuals thereby permitting the simultaneous analysis of multiple samples in a single run of the DNA sequencer. The number of times a given region of the genome in a library preparation (or other nucleic preparation of interest) is sequenced (number of reads) will be proportional to the number of copies of that sequence in the genome of interest (or expression level in the case of cDNA containing preparations). Biases in amplification efficiency can be taken into account in such quantitative determination.

**Analytics**

During performance of the methods provided herein, nucleic acid sequencing data is generated for amplicons created by the tiled multiplex PCR. Algorithm design tools are available that can be used and/or adapted to analyze this data to determine within certain confidence limits, whether a mutation, including a gene fusion, is present in a target gene, as illustrated in the examples herein.

FIG. 11 provides an exemplary workflow for the analysis of sequencing data resulting from either one-sided nested multiplex PCR methods with target specific tiled primers or two-sided, one step multiplex PCR method with target specific tiled primers. Sequencing data, optionally for a plus and minus strand, can be analyzed using Fastq and the paired end reads can be assembled. Unique identifiers can be used in quality control to confirm the accuracy of sequencing reads of the same amplicon. Sequencing Reads can be demultiplexed using an in-house tool, assembled and mapped to a reference genome, such as the hg19 genome, using the Burrows-Wheeler alignment software, Bwa mem function (BWA, Burrows-Wheeler Alignment Software (see Li H. and Durbin R. (2010) Fast and accurate long-read alignment with Burrows-Wheeler Transform. Bioinformatics, Epub. [PMID: 20080505]).

QC metrics can be utilized to improve the quality of the analysis. Tiling amplification statistics QC can be performed by analyzing total reads, number of mapped reads, number of mapped reads on target, and number of reads counted. In specific non-limiting examples, reads having a certain number, (e.g. 2, 3, 4, 5, 6, 7, 8, 9, or 10) or more mismatches to the reference human genome can be discarded. Furthermore, a mapping quality score, as known in the art, can be utilized and reads with a mapping quality score of less than a certain cutoff (e.g. 25, 20 (1 in 200 mapped incorrectly), 15, or 10) can be discarded. Then a depth of reads can be calculated and statistics thereof can be calculated.

Reads that pass QC analysis are then analyzed as shown in Figure 11, to detect fusions and/or to detect SNVs. As shown in FIG. 11, a different analytical flow can be followed depending on whether the method is analyzing the data to detect fusions or SNVs. For fusion detection Bwa mem mode reports supplementary alignments as alignments of reads that have a primary alignment that explain the mapped portion of the primary alignment. There can be multiple supplementary alignments for each primary alignment. By building a linkage map of the primary-supplementary alignment pairs, the breakpoints in the data can be discovered. Breakpoints can be detected as sequences linked too far from each other to be explained by a local mutation. They may either be gene fusions or artifacts.

Certain illustrative embodiments, utilize paired-end bridge analysis, where paired end reads are mapped before they are assembled. For this analysis sequencing reads can be mapped in paired-end mode. If sequencing reads are found to map on one fusion gene and its sequencing mate maps confidently on the fusion partner then the sequence read can be counted as evidence of a detected fusion bridge. The bridge maps can be produced for the target regions and reported in a similar manner to supplementary read analysis. The counts of bridge reads versus breakpoint reads can be compared and analyzed for one barcode first and then metrics can be built to report them for all the samples. Thus, detection of breakpoints can be verified.

In one specific example of analysis to detect fusions, after BWA maps sequencing reads for a sample to a reference human genome, some of the reads can map to two or more different locations in the genome (as discussed below for "supplementary read analysis"). These are initial seeding fusion calls, which may be true fusion calls, or may be false positives. For example, the reads may map to two homologs of a gene mapping to different locations of the genome, and not to two different genes of a fusion event. To help to differentiate these possibilities, the algorithm can create a new reference sequence that is a modified version of the original reference genome that now includes the possible fusion event, building a donor, acceptor, fusion sequence template for each call. Even reads that initially did not show a fusion alignment can be run through the analysis again using the modified version of the reference genome that includes the possible fusion event. Some reads that initially did not show an alignment to fusion partners, may now show an alignment when they are mapped to the putative fusion sequence. If a sufficient number of reads whether or not from the same initial nucleic acid fragment (as a number or percentage of reads) in a sample, map to a particular fusion event, then the fusion can be reported. For example, if at least 2, 3, 4, 5, 6, 7, 8, 9, 10, 11, 12, 13, 14, 15, 16, 17, 18, 19, 20, 25, 50, or 100 reads from a sample having 1,000, 2,000, 2,500, 5,000, 10,000, 20,000, or 25,000 nucleic acid fragments, map to a fusion, whether or not from the same initial nucleic acid fragment, then a fusion can be reported.

Accordingly, the present invention includes methods for detecting gene fusions in a sample from a mammal, that include the following: performing PCR on nucleic acid fragments from the sample for a target region known to be a site for a gene fusion, to generate amplicons; sequencing the amplicons to generate sequence information about the nucleic acid fragments; initially mapping the sequence information to a reference genome to determine whether any nucleic acid fragments appear to cross a fusion junction indicative of an apparent gene fusion; remapping the sequence information to a fusion genome that comprises the apparent gene fusion; wherein a number of nucleic fragments that map to the apparent gene fusion in the fusion genome that is above a cutoff value is indicative of a gene fusion.

In one specific example of analysis for SNV detection, the SNV branch of the flow diagram shown in FIG. 11 can be followed. First, a tiling count is performed of the number of times an SNV is detected at a position for each sequencing read that is derived from the same starting nucleic acid fragment in the sample. In order to help facilitate this, an amplification reaction can be performed after ligating unique identifiers (UIDs) to nucleic acid fragment from a sample. Thus, analysis can be performed by identifying and counting UIDs and fragment ends (since there can be more nucleic acid fragments in the sample than "UIDs"). An SNV can be called, for example, if a certain percentage (5, 10, 20, 25, 30, 40, 50, 60, 70, 75, 80, 90, 95, or 99%) of reads for a given nucleic acid from the sample is exceeded. This is represented by the Tiling Count program in FIG. 11. For non-limiting example, if 10% of the reads of a given nucleic acid fragment from the initial sample, reveal an SNV, then an SNV can be called for that starting nucleic acid fragment.

Next a Tiling Pileup analysis can be performed for a given amplicon, to determine whether a cutoff is exceeded for an absolute number or a percentage of amplicons that report the SNV for the same position. If at least a certain number or a certain percentage of amplicons that span a particular position report an SNV (the cutoff is exceeded) at that position, then an SNV call is made for that position. For example, if at least 2, 3, 4, 5, 6, 7, 8, 9. or 10 amplicons that span a target position report an SNV at that position, then the SNV is called for that position.

## Target Genes

Target genes of the present invention in exemplary embodiments, are cancer-related genes. However, a skilled artisan will understand that the methods provided herein can be used to detect similar mutations on any other gene(s). A cancer-related gene refers to a gene associated with an altered risk for a cancer or an altered prognosis for a cancer.

Exemplary cancer-related genes that promote cancer include oncogenes; genes that enhance cell proliferation, invasion, or metastasis; genes that inhibit apoptosis; and pro-angiogenesis genes. Cancer-related genes that inhibit cancer include, but are not limited to, tumor suppressor genes; genes that inhibit cell proliferation, invasion, or metastasis; genes that promote apoptosis; and anti-angiogenesis genes.

An embodiment of the mutation detection method begins with the selection of the region of the gene that becomes the target. The region with known mutations and fusion points and the artificially synthesized gene fusions, referred to as fusion spikes, are used to develop the methods of gene fusion detection as well as serve as fingerprints of gene fusion for diagnostic purposes. COSMIC (Catalog of Somatic Mutations in Cancer, Sanger Institute at www.sanger.ac.uk) database of fusion transcripts to genomic coordinates (i.e., translocations) can be used to select a target region (ie. A range of a sequence) for each reported fusion based on exon boundaries. Fusion partners are identified that contributed at least 1% to the total number of observed fusions for that gene.

The method of the present invention in exemplary embodiments, detects a gene fusion from 1, 2, 3, 4, 5, 6, 7, 8, 9, 10 or all fusion partner genes selected from the following: AKT1, ALK, BRAF, EGFR, HER2, KRAS, MEK1, MET, NRAS, PIK3CA, RET, and ROS1.

In addition to gene fusion detection, methods provided herein can be used to detect virtually any type of mutation, especially mutations known to be associated with cancer. Exemplary polymorphisms or mutations can be in one or more of the following genes: TP53, PTEN, PIK3CA, APC, EGFR, NRAS, NF2, FBXW7, ERBBs, ATAD5, KRAS, BRAF, VEGF, EGFR, HER2, ALK, p53, BRCA, BRCA1, BRCA2, SETD2, LRP1B, PBRM, SPTA1, DNMT3A, ARID1A, GRIN2A, TRRAP, STAG2, EPHA3/5/7, POLE, SYNE1, C20orf80, CSMD1, CTNNB1, ERBB2. FBXW7, KIT, MUC4, ATM, CDH1, DDX11, DDX12, DSPP, EPPK1, FAM186A, GNAS, HRNR, KRTAP4-11, MAP2K4, MLL3, NRAS, RBI, SMAD4, TTN, ABCC9, ACVR1B, ADAM29, ADAMTS19, AGAP10, AKT1, AMBN, AMPD2, ANKRD30A, ANKRD40, APOBR, AR, BIRC6, BMP2, BRAT1, BTNL8, C12orf4, C1QTNF7, C20orf186, CAPRIN2, CBWD1, CCDC30, CCDC93, CD5L, CDC27, CDC42BPA, CDH9, CDKN2A, CHD8, CHEK2, CHRNA9, CIZ1, CLSPN, CNTN6, COL14A1, CREBBP, CROCC, CTSF, CYP1A2, DCLK1, DHDDS, DHX32, DKK2, DLEC1, DNAH14, DNAH5, DNAH9, DNASE1L3, DUSP16, DYNC2H1, ECT2, EFHB, RRN3P2, TRIM49B, TUBB8P5, EPHA7, ERBB3, ERCC6, FAM21A, FAM21C, FCGBP, FGFR2, FLG2, FLT1, FOLR2, FRYL, FSCB, GAB1, GABRA4, GABRP, GH2, GOLGA6L1, GPHB5, GPR32, GPX5, GTF3C3, HECW1, HIST1H3B, HLA-A, HRAS, HS3ST1, HS6ST1, HSPD1, IDH1, JAK2, KDM5B, KIAA0528, KRT15, KRT38, KRTAP21-1, KRTAP4-5, KRTAP4-7, KRTAP5-4, KRTAP5-5, LAMA4, LATS1, LMF1, LPAR4, LPPR4, LRRFIP1, LUM, LYST, MAP2K1, MARCH1, MARCO, MB21D2, MEGF10, MMP16, MORC1, MRE11A, MTMR3, MUC12, MUC17, MUC2, MUC20, NBPF10, NBPF20, NEK1, NFE2L2, NLRP4, NOTCH2, NRK, NUP93, OBSCN, OR11H1, OR2B11, OR2M4, OR4Q3, OR5D13, OR812, OXSM, PIK3R1, PPP2R5C, PRAME, PRF1, PRG4, PRPF19, PTH2, PTPRC, PTPRJ, RAC1, RAD50, RBM12, RGPD3, RGS22, ROR1, RP11-671M22.1, RP13-996F3.4, RP1L1, RSBN1L, RYR3, SAMD3, SCN3A, SEC31A, SF1, SF3B1, SLC25A2, SLC44A1, SLC4A11, SMAD2, SPTA1, ST6GAL2, STK11, SZT2, TAF1L, TAX1BP1, TBP, TGFBI, TIF1, TMEM14B, TMEM74, TPTE, TRAPPC8, TRPS1, TXNDC6, USP32, UTP20, VASN, VPS72, WASH3P, WWTR1, XPO1, ZFHX4, ZMIZ1, ZNF167, ZNF436, ZNF492, ZNF598, ZRSR2, ABL1, AKT2, AKT3, ARAF, ARFRP1, ARID2, ASXL1, ATR, ATRX, AURKA, AUR, AXL, BAP1, BARD1, BCL2, BCL2L2, BCL6, BCOR, BCORL1, BLM, BRIP1, BTK, CARD11, CBFB, CBL, CCND1, CCND2, CCND3, CCNE1, CD79A, CD79B, CDC73, CDK12, CDK4, CDK6, CDK8, CDKN1B, CDKN2B, CDKN2C, CEBPA, CHEK1, CIC, CRKL, CRLF2, CSF1R, CTCF, CTNNA1, DAXX, DDR2, DOT1L, EMSY (C11orf30), EP300, EPHA3, EPHA5, EPHB1, ERBB4, ERG, ESR1, EZH2, FAM123B (WTX), FAM46C, FANCA, FANCC, FANCD2, FANCE, FANCF, FANCG, FANCL, FGF10, FGF14, FGF19, FGF23, FGF3, FGF4, FGF6, FGFR1, FGFR2, FGFR3, FGFR4, FLT3, FLT4, FOXL2, GATA1, GATA2, GATA3, GID4 (C17orf39), GNA11, GNA13, GNAQ, GNAS, GPR124, GSK3B, HGF, IDH1, IDH2, IGF1R, IKBKE, IKZF1, IL7R, INHBA, IRF4, IRS2, JAK1, JAK3, JUN, KAT6A (MYST3), KDM5A, KDM5C, KDM6A, KDR, KEAP1, KLHL6, MAP2K2, MAP2K4, MAP3K1, MCL1, MDM2, MDM4, MED12, MEF2B, MEN1, MET, MITF, MLH1, MLL, MLL2, MPL, MSH2, MSH6, MTOR, MUTYH, MYC, MYCL1, MYCN, MYD88, NF1, NFKBIA, NKX2-1, NOTCH1, NPM1, NRAS, NTRK1, NTRK2, NTRK3, PAK3, PALB2, PAX5, PBRM1, PDGFRA, PDGFRB, PDK1, PIK3CG, PIK3R2, PPP2R1A, PRDM1, PRKAR1A, PRKDC, PTCH1, PTPN11, RAD51, RAF1, RARA, RET, RICTOR, RNF43, RPTOR, RUNX1, SMARCA4, SMARCB1, SMO, SOCS1, SOX10, SOX2, SPEN, SPOP, SRC, STAT4, SUFU , TET2, TGFBR2, TNFAIP3, TNFRSF14, TOPI, TP53, TSC1, TSC2, TSHR, VHL, WISP3, WT1, ZNF217, ZNF703, and combinations thereof.

**Amplification (e.g. *PCR)* Reaction Mixtures:**

Methods of the present invention, in certain embodiments, include forming an amplification reaction mixture. The reaction mixture typically is formed by combining a polymerase, deoxynucleoside triphosphates, nucleic acid fragments from a nucleic acid library generated from the sample, a series of forward target-specific outer primers and a plus strand reverse outer universal primer. Another illustrative embodiment is a reaction mixture that includes forward target-specific inner primers instead of the forward target-specific outer primers and amplicons from a first PCR reaction using the outer primers, instead of nucleic acid fragments from the nucleic acid library. The reaction mixtures provided herein, themselves forming in illustrative embodiments, a separate aspect of the invention. In illustrative embodiments, the reaction mixtures are PCR reaction mixtures. PCR reaction mixtures typically include magnesium.

In some embodiments, the reaction mixture includes ethylenediaminetetraacetic acid (EDTA), magnesium, tetramethyl ammonium chloride (TMAC), or any combination thereof. In some embodiments, the concentration of TMAC is between 20 and 70 mM, inclusive. While not meant to be bound to any particular theory, it is believed that TMAC binds to DNA, stabilizes duplexes, increases primer specificity, and/or equalizes the melting temperatures of different primers. In some embodiments, TMAC increases the uniformity in the amount of amplified products for the different targets. In some embodiments, the concentration of magnesium (such as magnesium from magnesium chloride) is between 1 and 8 mM.

The large number of primers used for multiplex PCR of a large number of targets may chelate a lot of the magnesium (2 phosphates in the primers chelate 1 magnesium). For example, if enough primers are used such that the concentration of phosphate from the primers is ~9 mM, then the primers may reduce the effective magnesium concentration by -4.5 mM. In some embodiments, EDTA is used to decrease the amount of magnesium available as a cofactor for the polymerase since high concentrations of magnesium can result in PCR errors, such as amplification of non-target loci. In some embodiments, the concentration of EDTA reduces the amount of available magnesium to between 1 and 5 mM (such as between 3 and 5 mM).

In some embodiments, the pH is between 7.5 and 8.5, such as between 7.5 and 8, 8 and 8.3, or 8.3 and 8.5, inclusive. In some embodiments, Tris is used at, for example, a concentration of between 10 and 100 mM, such as between 10 and 25 mM, 25 and 50 mM, 50 and 75 mM, or 25 and 75 mM, inclusive. In some embodiments, any of these concentrations of Tris are used at a pH between 7.5 and 8.5. In some embodiments, a combination of KCl and $(NH_4)_2SO_4$ is used, such as between 50 and 150 mM KCl and between 10 and 90 mM $(NH_4)_2SO_4$, inclusive. In some embodiments, the concentration of KCl is between 0 and 30 mM, between 50 and 100 mM, or between 100 and 150 mM, inclusive. In some embodiments, the concentration of $(NH_4)_2SO_4$ is between 10 and 50 mM,50 and 90 mM, 10 and 20 mM, 20 and 40 mM, 40 mM and 60, or 60 mM and 80 mM $(NH_4)_2SO_4$, inclusive. In some embodiments, the ammonium $[NH_4^+]$ concentration is between 0 and 160 mM, such as between 0 to 50, 50 to 100, or 100 to 160 mM, inclusive. In some embodiments, the sum of the potassium and ammonium concentration ($[K^+] + [NH_4^+]$) is between 0 and 160 mM, such as between 0 to 25, 25 to 50, 50 to 150, 50 to 75, 75 to 100, 100 to 125, or 125 to 160 mM, inclusive. An exemplary buffer with $[K^+] + [NH_4^+] = 120$ mM is 20 mM KCl and 50 mM $(NH_4)_2SO_4$. In some embodiments, the buffer includes 25 to 75 mM Tris, pH 7.2 to 8, 0 to 50 mM KCL, 10 to 80 mM ammonium sulfate, and 3 to 6 mM magnesium, inclusive. In some embodiments, the buffer includes 25 to 75 mM Tris pH 7 to 8.5, 3 to 6 mM $MgCl_2$, 10 to 50 mM KCl, and 20 to 80 mM $(NH_4)_2SO_4$, inclusive. In some embodiments, 100 to 200 Units/mL of polymerase are used. In some embodiments, 100 mM KCl, 50 mM $(NH_4)_2SO_4$, 3 mM $MgCl_2$, 7.5 nM of each primer in the library, 50 mM TMAC, and 7 ul DNA template in a 20 ul final volume at pH 8.1 is used.

In some embodiments, a crowding agent is used, such as polyethylene glycol (PEG, such as PEG 8,000) or glycerol. In some embodiments, the amount of PEG (such as PEG 8,000) is between 0.1 to 20%, such as between 0.5 to 15%, 1 to 10%, 2 to 8%, or 4 to 8%, inclusive. In some embodiments, the amount of glycerol is between 0.1 to 20%, such as between 0.5 to 15%, 1 to 10%, 2 to 8%, or 4 to 8%, inclusive. In some embodiments, a crowding agent allows either a low polymerase concentration and/or a shorter annealing time to be used. In some embodiments, a crowding agent improves the uniformity of the DOR and/or reduces dropouts (undetected alleles).

***Polymerases***

In some embodiments, a polymerase with proof-reading activity, a polymerase without (or with negligible) proof-reading activity, or a mixture of a polymerase with proof-reading activity and a polymerase without (or with negligible) proof-reading activity is used. In some embodiments, a hot start polymerase, a non-hot start polymerase, or a mixture of a hot start polymerase and a non-hot start polymerase is used. In some embodiments, a HotStarTaq DNA polymerase is used (see, for example, QIAGEN catalog No. 203203). In some embodiments, AmpliTaq Gold® DNA Polymerase is used. In some ebodiments a PrimeSTAR GXL DNA polymerase, a high fidelity polymerase that provides efficient PCR amplification when there is excess template in the reaction mixture, and when amplifying long products, is used (Takara Clontech, Mountain View, CA). In some embodiments, KAPA Taq DNA Polymerase or KAPA Taq HotStart DNA Polymerase is used; they are based on the single-subunit, wild-type *Taq* DNA polymerase of the thermophilic bacterium *Thermus aquaticus.* KAPA Taq and KAPA Taq HotStart DNA Polymerase have 5'-3' polymerase and 5'-3' exonuclease activities, but no 3' to 5' exonuclease (proofreading) activity (see, for example, KAPA BIOSYSTEMS catalog No. BK1000). In some embodiments, *Pfu* DNA polymerase is used; it is a highly thermostable DNA polymerase from the hyperthermophilic archaeum *Pyrococcus furiosus.* The enzyme catalyzes the template-dependent polymerization of nucleotides into duplex DNA in the 5'→3' direction. *Pfu* DNA Polymerase also exhibits 3'→5' exonuclease (proofreading) activity that enables the polymerase to correct nucleotide incorporation errors. It has no 5'→3' exonuclease activity (see, for example, Thermo Scientific catalog No. EP0501). In some embodiments Klentaql is used; it is a Klenow-fragment analog of Taq DNA polymerase, it has no exonuclease or endonuclease activity (see, for example, DNA POLYMERASE TECHNOLOGY, Inc, St. Louis, Missouri, catalog No. 100). In some embodiments, the polymerase is a PUSHION DNA polymerase, such as PHUSION High Fidelity DNA polymerase (M0530S, New England BioLabs, Inc.) or PHUSION Hot Start Flex DNA polymerase (M0535S, New England BioLabs, Inc.). In some embodiments, the polymerase is a Q5® DNA Polymerase, such as Q5® High-Fidelity DNA Polymerase (M0491S, New England BioLabs, Inc.) or Q5® Hot Start High-Fidelity DNA Polymerase (M0493S, New England BioLabs, Inc.). In some embodiments, the polymerase is a T4 DNA polymerase (M0203S, New England BioLabs, Inc.).

In some embodiment, between 5 and 600 Units/mL (Units per 1 mL of reaction volume) of polymerase is used, such as between 5 to 100, 100 to 200, 200 to 300, 300 to 400, 400 to 500, or 500 to 600 Units/mL, inclusive.

***PCR Methods***

In some embodiments, hot-start PCR is used to reduce or prevent polymerization prior to PCR thermocycling. Exemplary hot-start PCR methods include initial inhibition of the DNA polymerase, or physical separation of reaction components reaction until the reaction mixture reaches the higher temperatures. In some embodiments, slow release of magnesium is used. DNA polymerase requires magnesium ions for activity, so the magnesium is chemically separated from the reaction by binding to a chemical compound, and is released into the solution only at high temperature. In some embodiments, non-covalent binding of an inhibitor is used. In this method a peptide, antibody, or aptamer are non-covalently bound to the enzyme at low temperature and inhibit its activity. After incubation at elevated temperature, the inhibitor is released and the reaction starts. In some embodiments, a cold-sensitive Taq polymerase is used, such as a modified DNA polymerase with almost no activity at low temperature. In some embodiments, chemical modification is used. In this method, a molecule is covalently bound to the side chain of an amino acid in the active site of the DNA polymerase. The molecule is released from the enzyme by incubation of the reaction mixture at elevated temperature. Once the molecule is released, the enzyme is activated.

In some embodiments, the amount to template nucleic acids (such as an RNA or DNA sample) is between 20 and 5,000 ng, such as between 20 to 200, 200 to 400, 400 to 600, 600 to 1,000; 1,000 to 1,500; or 2,000 to 3,000 ng, inclusive.

In some embodiments a QIAGEN Multiplex PCR Kit is used (QIAGEN catalog No. 206143). For 100 x 50 µl multiplex PCR reactions, the kit includes 2x QIAGEN Multiplex PCR Master Mix (providing a final concentration of 3 mM $MgCl_2$, 3 x 0.85 ml), 5x Q-Solution (1 x 2.0 ml), and RNase-Free Water (2 x 1.7 ml). The QIAGEN Multiplex PCR Master Mix (MM) contains a combination of KCl and $(NH_4)_2SO_4$ as well as the PCR additive, Factor MP, which increases the local concentration of primers at the template. Factor MP stabilizes specifically bound primers, allowing efficient primer extension by HotStarTaq DNA Polymerase. HotStarTaq DNA Polymerase is a modified form of *Taq* DNA polymerase and has no polymerase activity at ambient temperatures. In some embodiments, HotStarTaq DNA Polymerase is activated by a 15-minute incubation at 95°C which can be incorporated into any existing thermal-cycler program.

In some embodiments, 1x QIAGEN MM final concentration (the recommended concentration), 7.5 nM of each primer in the library, 50 mM TMAC, and 7 ul DNA template in a 20 ul final volume is used. In some embodiments, the PCR thermocycling conditions include 95°C for 10 minutes (hot start); 20 cycles of 96°C for 30 seconds; 65°C for 15 minutes; and 72°C for 30 seconds; followed by 72°C for 2 minutes (final extension); and then a 4°C hold.

In some embodiments, 2x QIAGEN MM final concentration (twice the recommended concentration), 2 nM of each primer in the library, 70 mM TMAC, and 7 ul DNA template in a 20 ul total volume is used. In some embodiments, up to 4 mM EDTA is also included. In some embodiments, the PCR thermocycling conditions include 95°C for 10 minutes (hot start); 25 cycles of 96°C for 30 seconds; 65°C for 20, 25, 30, 45, 60, 120, or 180 minutes; and optionally 72°C for 30 seconds); followed by 72°C for 2 minutes (final extension); and then a 4°C hold.

Another exemplary set of conditions includes a semi-nested PCR approach. The first PCR reaction uses 20 ul a reaction volume with 2x QIAGEN MM final concentration, 1.875 nM of each primer in the library (outer forward and reverse primers), and DNA template. Thermocycling parameters include 95°C for 10 minutes; 25 cycles of 96°C for 30 seconds, 65°C for 1 minute, 58°C for 6 minutes, 60°C for 8 minutes, 65°C for 4 minutes, and 72°C for 30 seconds; and then 72°C for 2 minutes, and then a 4°C hold. Next, 2 ul of the resulting product, diluted 1:200, is as input in a second PCR reaction. This reaction uses a 10 ul reaction volume with 1x QIAGEN MM final concentration, 20 nM of each inner forward primer, and 1 uM of reverse primer tag. Thermocycling parameters include 95°C for 10 minutes; 15 cycles of 95C for 30 seconds, 65°C for 1 minute, 60°C for 5 minutes, 65°C for 5 minutes, and 72°C for 30 seconds; and then 72°C for 2 minutes, and then a 4°C hold. The annealing temperature can optionally be higher than the melting temperatures of some or all of the primers, as discussed herein (see U.S. Patent Application No. 14/918,544, filed Oct. 20, 2015, which is herein incorporated by reference in its entirety).

The melting temperature ($T_m$) is the temperature at which one-half (50%) of a DNA duplex of an oligonucleotide (such as a primer) and its perfect complement dissociates and becomes single strand DNA. The annealing temperature ($T_A$) is the temperature one runs the PCR protocol at. For prior methods, it is usually 5 C below the lowest $T_m$ of the

primers used, thus close to all possible duplexes are formed (such that essentially all the primer molecules bind the template nucleic acid). While this is highly efficient, at lower temperatures there are more unspecific reactions bound to occur. One consequence of having too low a $T_A$ is that primers may anneal to sequences other than the true target, as internal single-base mismatches or partial annealing may be tolerated. In some embodiments of the present inventions, the $T_A$ is higher than ($T_m$), where at a given moment only a small fraction of the targets have a primer annealed (such as only ~1-5%). If these get extended, they are removed from the equilibrium of annealing and dissociating primers and target (as extension increases $T_m$ quickly to above 70 C), and a new ~1-5% of targets has primers. Thus, by giving the reaction long time for annealing, one can get -100% of the targets copied per cycle.

In various embodiments, the annealing temperature is between 1, 2, 3, 4, 5, 6, 7, 8, 9, 10, 11, 12, 13 °C and 2, 3, 4, 5, 6, 7, 8, 9, 10, 11, 12, 13, or 15 °C on the high end of the range, greater than the melting temperature (such as the empirically measured or calculated $T_m$) of at least 25, 50, 60, 70, 75, 80, 90, 95, or 100% of the non-identical primers. In various embodiments, the annealing temperature is between 1 and 15 °C (such as between 1 to 10, 1 to 5, 1 to 3, 3 to 5, 5 to 10, 5 to 8, 8 to 10, 10 to 12, or 12 to 15 °C, inclusive) greater than the melting temperature (such as the empirically measured or calculated $T_m$) of at least 25; 50; 75; 100; 300; 500; 750; 1,000; 2,000; 5,000; 7,500; 10,000; 15,000; 19,000; 20,000; 25,000; 27,000; 28,000; 30,000; 40,000; 50,000; 75,000; 100,000; or all of the non-identical primers. In various embodiments, the annealing temperature is between 1 and 15 °C (such as between 1 to 10, 1 to 5, 1 to 3, 3 to 5, 3 to 8, 5 to 10, 5 to 8, 8 to 10, 10 to 12, or 12 to 15 °C, inclusive) greater than the melting temperature (such as the empirically measured or calculated $T_m$) of at least 25%, 50%, 60%, 70%, 75%, 80%, 90%, 95%, or all of the non-identical primers, and the length of the annealing step (per PCR cycle) is between 5 and 180 minutes, such as 15 and 120 minutes, 15 and 60 minutes, 15 and 45 minutes, or 20 and 60 minutes, inclusive.

As discussed herein, methods of the present invention in illustrative embodiments, are One-Sided nested multiplex PCR methods that use tiled primers (i.e. primers that bind a series of tiled primer binding sites on a target region of a target gene). In such methods, target DNA (for example nucleic acid fragments from a nucleic acid library made from ctDNA) that has an adaptor at the fragment ends can be used. Specific target amplification ("STA") can be performed with a multiplex set of nested Forward primers and using the ligation adapter tag as a binding site for a universal reverse primer. A second STA may then be performed using a set of nested Forward primers and a universal reverse primer that can be the same or different than the universal primer used for the first PCR reaction.

A skilled artisan will recognize that other amplification (e.g. PCR) variations can be used to carry out methods of the present invention, with illustrative embodiments including a series of tiled primers. For example, PCR variations can include the following:

Semi-nested PCR: After STA 1 a second STA can be performed that includes a multiplex set of internal nested Forward primers and one (or few) tag-specific Reverse primers.

Fully nested PCR: After STA step 1, it is possible to perform a second multiplex PCR (or parallel multiplex PCRs of reduced complexity) with two nested primers carrying tags (A, a, B, b).

Hemi-nested PCR: It is possible to use target DNA that has adaptors at the fragment ends. STA is performed comprising a multiplex set of Forward primers (B) and one (or few) tag-specific Reverse primers (A). A second STA can be performed using a universal tag-specific Forward primer and target specific Reverse primer.

Triply hemi-nested PCR: It is possible to use target DNA that has and adaptor at the fragment ends. STA is performed comprising a multiplex set of Forward primers (B) and one (or few) tag-specific Reverse primers (A) and (a). A second STA can be performed using a universal tag-specific Forward primer and target specific Reverse primers.

One-sided PCR: It is possible to use target DNA that has an adaptor at the fragment ends. STA may be performed with a multiplex set of Forward primers and one (or few) tag-specific Reverse primer.

Reverse semi-nested PCR: It is possible to use target DNA that has an adaptor at the fragment ends. STA may be performed with a multiplex set of Forward primers and one (or few) tag-specific Reverse primer.

There also may be more variants that are simply iterations or combinations of the above methods such as doubly nested PCR, where three sets of primers are used. Another variant is one-and-a-half sided nested mini-PCR, where STA may also be performed with a multiplex set of nested Forward primers and one (or few) tag-specific Reverse primer.

Note that in all of these variants, the identity of the Forward primer and the Reverse primer may be interchanged. Note that in some embodiments, the nested variant can equally well be run without the initial library preparation that comprises appending the adapter tags, and a universal amplification step. Note that in some embodiments, additional rounds of PCR may be included, with additional Forward and/or Reverse primers and amplification steps; these additional steps can be particularly useful if it is desirable to further increase the percent of DNA molecules that correspond to target regions of target genes from circulating tumor DNA.

### *Exemplary Multiplex PCR Methods*

The tiling PCR methods provided herein are multiplex PCR methods. Accordingly, in one aspect, the invention features methods of amplifying target overlapping segments of target regions of target genes in samples of nucleic acid fragments from a nucleic acid library. The method can include (i) contacting the nucleic acid sample with a library of primers that simultaneously hybridize to between 50, 100, 250, 500, 1,000; 2,000; 5,000; 7,500; 10,000; 15,000; 19,000; 20,000; 25,000; 27,000; 28,000; 30,000; 40,000; 50,000; and 75,000 primer binding sites (e.g. inner or outer primer binding sites) and 100, 250, 500, 1,000; 2,000; 5,000; 7,500; 10,000; 15,000; 19,000; 20,000; 25,000; 27,000; 28,000; 30,000; 40,000; 50,000; 75,000 and 100,000 primer binding sites, wherein the primer binding sites are typically a tiled series of primer binding sites. As discussed herein, groups of the primer binding sites are typically spaced apart on target region(s) of target gene(s) by a distance that can be equal to or less than the average amplicon size of the amplification reaction using the primers. In some embodiments, at least 50, 60, 70, 80, 90, 95, 96, 97, 98, 99, 99.5, or 100% of the target loci are amplified at least 5, 10, 20, 40, 50, 60, 80, 100, 120, 150, 200, 300, or 400-fold. In various embodiments, less than 60, 50, 40, 30, 20, 10, 5, 4, 3, 2, 1, 0.5, 0.25, 0.1, or 0.05% of the amplified products are primer dimers. In some embodiments, the method involves multiplex PCR followed by sequencing (such as high throughput sequencing) the multiplex amplicons to determine a mutation, such as a gene fusion in the target gene(s).

In various embodiments, long annealing times (as discussed herein and exemplified in Example 12) and/or low primer concentrations are used. In various embodiments, the length of the annealing step is between 15, 20, 25, 30, 35, 40, 45, or 60 minutes on the low end of the range and 20, 25, 30, 35, 40, 45, 60, 120, or 180 minutes on the high end of the range. In various embodiments, the length of the annealing step (per PCR cycle) is between 30 and 180 minutes. For example, the annealing step can be between 30 and 60 minutes and the concentration of each primer can be less than 20, 15, 10, or 5 nM

At high level of multiplexing, the solution may become viscous due to the large amount of primers in solution. If the solution is too viscous, one can reduce the primer concentration to an amount that is still sufficient for the primers to bind the template DNA. In various embodiments, between 1,000 and 100,000 different primers are used and the concentration of each primer is less than 20 nM, such as less than 10 nM or between 1 and 10 nM, inclusive.

The following examples are put forth so as to provide those of ordinary skill in the art with a complete disclosure and description of how to use the embodiments provided herein, and are not intended to limit the scope of the disclosure nor are they intended to represent that the Examples below are all or the only experiments performed. Efforts have been made to ensure accuracy with respect to numbers used (e.g. amounts, temperature, etc.) but some experimental errors and deviations should be accounted for. Unless indicated otherwise, parts are parts by volume, and temperature is in degrees Centigrade. It should be understood that variations in the methods as described can be made without changing the fundamental aspects that the Examples are meant to illustrate.

## EXAMPLES

### EXAMPLE 1. Identifying Fusion Gene Breakpoints for Tiling Analysis

Provided herein is an example of how a series of tiled primers can be designed and selected for use in methods of the present invention, especially methods for detecting a gene fusion using a one-side nested PCR reaction. The design of tiled primers for detection of gene fusions began with mapping COSMIC fusion transcripts to genomic coordinates (i.e., translocations). However, use of transcript-level information was found to induce uncertainty in breakpoint location because rearrangements were largely intronic (and so spliced out of the transcripts). Therefore, it was necessary to cover a range of sequence for each reported fusion based on exon boundaries. Identification of molecular signatures can assist in the development of a cancer detection panel for identifying gene fusions and can be applied beyond lung cancer to other cancers and diseases, e.g., ALK haemopoetic and lymphoid tissue, RET in thyroid cancer.

The evaluated target genes are known to have several fusion partners. However, gene expression of the target breakpoint is consistent because the fusion products are *Gain of Function* events and so the consistency of the breakpoint in the target gene was used for incorporation into tiling strategies. For example, targeted primers were designed to the genomic DNA of the target gene alone. This has elegantly accounted for the multiple fusion partners and the observation that the fusion breakpoints are larger for partner genes. This would require tiling < 3.6 kb of sequence for each of the three targets: ALK, ROS1 and RET.

Alternatively, both the target and the partner genes were also targeted which increased the required tiling substantially (see Table 1). Table 1 has a summary of breakpoints for target gene and their common partner genes (frequency > 1 %) and summarizes tiling requirements used to capture the reported fusion events. Genomic coordinates for Table 1 were used to define the tiling coordinates for translocation assays.

**Table 1:**

| Gene | ALK | ROS1 | RET |
|---|---|---|---|
| Reported prevalence in NSCLC* | 3-7% | 1% | 1% |
| Target gene rearrangement length (bases) | 3393 | 2937 | 3520 |
| Partner gene rearrangement length (bases) | 110928 | 3238 | 78849 |
| Total sequence length (bases) | 114321 | 6175 | 82369 |
| Number of distinct fusion events with at least 1% frequency of the gene's fusions | 44 | 2 | 11 |
| Proportion of all the gene's reported fusions within these coordinates | 0.961 | 1.000 | 0.983 |
| Total number of fusion events | 1331 | 12 | 1921 |
| *non-small cell lung cancer (NSCLC) | | | |

The domain breakpoints for each of the three target genes, and all partner genes with a contribution of more than 1% to that gene's reported fusion transcripts in COSMIC (there is a long tail of rare partners for ALK for example) were determined as shown in Table 2. Genomic coordinates are from human GRCh37.

Table 2:

| Target -Hugo | Partner -Hugo | Chr. Tar get | Start Target | End Target | Chr. Part ner | Start Partner | End Partner | Freq | Count |
|---|---|---|---|---|---|---|---|---|---|
| ALK | NPM1 | 2 | 29446394 | 29448326 | 5 | 170818803 | 170819713 | 0.45 | 625 |
| ALK | EML4 | 2 | 29446394 | 29449787 | 2 | 42472827 | 42553293 | 0.41 | 572 |
| ALK | TPM3 | 2 | 29446394 | 29448326 | 1 | 154130197 | 154142875 | 0.03 | 40 |
| ALK | RANBP2 | 2 | 29446394 | 29448326 | 2 | 109375004 | 109378556 | 0.03 | 36 |
| ALK | CLTC | 2 | 29446394 | 29448326 | 17 | 57763169 | 57771088 | 0.02 | 34 |
| ALK | ATIC | 2 | 29446394 | 29448326 | 2 | 216191701 | 216197104 | 0.02 | 24 |
| ROS1 | CD74 | 6 | 117642557 | 117645494 | 5 | 149782875 | 149784242 | 0.67 | 8 |
| ROS1 | LRIG3 | 6 | 117642557 | 117645494 | 12 | 59268355 | 59270226 | 0.33 | 4 |
| RET | CCDC6 | 10 | 43610184 | 43612838 | 10 | 61592411 | 61666990 | 0.59 | 1155 |
| RET | NCOA4 | 10 | 43610184 | 43613704 | 10 | 51582272 | 51584615 | 0.36 | 706 |
| RET | PRKAR 1A | 10 | 43610184 | 43612031 | 17 | 66522053 | 66523980 | 0.03 | 60 |

COSMIC fusions are annotated at the level of RNA transcripts; consequently, the underlying genomic fusion breakpoint is unknown most of the time. Therefore, a range for the breakpoint given the transcript information was inferred. Analysis of COSMIC v70 fusion database identified 54,290 recorded fusions. Fusion events were filtered such that i) Fusions are annotated with respect to the Ensembl transcript annotation with inferred transcript-level fusion coordinates (15,440 passed), ii) Fusions involved one and only one partner (54,063 passed), iii) Fusions that did not include insertions of novel sequence (54,236 passed), and iv) no restriction was applied to lung-cancer specific samples. After filtering 15,182 fusion remained.

Next, for each target gene, partners were identified that contributed at least 1% to the total number of observed fusions for that gene. Then for each fusion partner, the maximum genomic range of the breakpoint from the fusions between the target and its partner were recorded using the exonic coordinates of the gene. It is noted that accounting for strand (plus or minus) was also done. If the transcript coordinates reported in COSMIC did not match with the Ensembl coordinates, the inconsistency was noted and no range for that transcript was reported.

As a result of the filtering criteria ninety percent of the 122 reported ROS1 fusions failed filters (largely resulting from inconsistent transcript labeling). CD74 was identified as the most prevalent partner. Filtering removed SCL34A2, EZR, and GOPC. It can be possible to recover additional transcripts with further filtering refinements.

**EXAMPLE 2A. Development of Synthetic Fusion Standards**

**Design of a Gene Fusion Spike**

A fusion spike, as used herein, refers to an artificially synthesized gene fusion, e.g., CD74:Rosl, NMP1:Alk1 (x2) and TPM4:Alkl. The first gene is the Partner (e.g., CD74, NMP1 and TPM4) and the later the Target (Ros1, ALK1 (two sets) and Alk1). The fusion spikes were designed to correspond to the average length of cfDNA, selecting 160 bp in length. The design makes use of nine primers tiled across the 160 fusion spike "target" as illustrated in FIG. 1.

Fusion spikes were designed to span the ’junction’, as used herein, can refer to the fusion breakpoint between the two fusion partners. To illustrate, consider the following example, there are two genes A and B composed of sequence {a_i} and {b_i}, fusions occur between these two genes. To generate a fusion spike, we first identified the location of the breakpoint in each gene and then construct the spike S:

$$S = a_\{i-m\},...,a_i,b_j,...,b\{j+n\}$$

where the total length of S is 160 bases. Values were then specified for m and n such that different proportions of gene A and gene B are represented in the spike. The disclosed method is able to detect fusions in blood as it relies on DNA as the sample material which is usually fragmented at an approximate average length of about 50 bp, about 60 bp, about 70 bp, about 80 bp, about 90 bp, about 100 bp, about 110 bp, about 120 bp, about 130 bp, about 140 bp, about 150 bp, and at least about 160 bp.

**EXAMPLE 2B. Development of Synthetic Fusion Standards**

The design of synthetic fusion spikes was done in order to develop a system that allowed detecting of gene fusion profiles. Identification of a gene fusion profile can assist to identify the fused genomic sequence for rearrangements following sequencing of the fused genomic DNA. The genomic sequence (suspected of having a gene fusion) was used to construct tiled primer template synthetic oligonucleotides that tiled across each target sequence containing the breakpoint as tiled fusion spikes, each of 160 bp in length. FIG. 1 illustrates the tiling of these synthetic oligonucleotides to construct fusion spikes.

A review of the literature for published genome sequences of translocations was conducted to identify gene fusion products. This resulted in the selection of six regions containing gene fusions (5 ALK, 1 ROS) followed by bioinformatics computations to identify the corresponding genome location to unify the results.

Following genomic location identification of each of the six fusion regions, 160 bp in length double-stranded synthetic oligonucleotide fusion spikes were designed across each of the fusion breakpoints by tiling the spike across the fusion breakpoint in 8 base intervals. The range of the tiling started with 152 bases of gene A and 8 bases of gene B, ending with 8 bases of gene A and 152 bases of gene B as shown in FIGS. 2-3.

Tables 3A and 3B provide the synthetically designed gene fusion spikes for the selected regions. Column headings are as follows: Table 3A: SEQ ID NO (Corresponds to sequence in Sequence Listing); ID (reference to primary source of the reported rearrangement); and Sequence (Reported genomic sequence padded out to a uniform length for spike design). The sequence listing nucleotide symbols in Table 3A are in upper case if the specific sequence is found in the gene exon region, and lower case if found in the gene intron region. Table 3B: Gene1 (HUGO gene name of first gene involved in the fusion); Gene2 (HUGO gene name of second gene involved in fusion); SEQ ID NO.; g1 (Genomic coordinates corresponding to first gene within reported sequence) / g2 (Genomic coordinates corresponding to second gene within reported sequence); Start- cStart1/ cStart2 ("cStartl" - Start coordinate corresponding to first gene in reported sequence, "cStart2" - Start coordinate corresponding to second gene in reported sequence); End- cEnd1/ cEnd2 ("cEnd1"- End coordinate corresponding to first gene in reported sequence, "cEnd2"- End coordinate corresponding to second gene in reported sequence); Strand - Strand1/Strand21 (Strand relative to reference sequence (minus indicates reverse complement strand); Gap (Distance between cEnd1 and cStart2 (values > 0 indicate novel sequence, values <0 indicate microhomology); Identity- Identity1/Identiy2 (Percent identity when mapped to human reference); Resulting transcript (Prediction of whether the resulting translocation resulted in a transcript with oncogenic activity (both versions can be present for balanced translocations)); Plus (Prediction of whether the plus strand primer design will capture the translocation (significant because the one-sided design is strand specific)).

**Table 3A**

| SEQ ID NO: | ID | Sequence |
|---|---|---|
| 299 | GenBank: AF032882 | TGGTTAGGGAAACAGGGCAGGAGTTACCATCCCTGCCTAC AGAGAGGGAAACTGCAGTCCAAAGAGGTCCTGTGACCTGG TCCTCATGGCTCAGCTTGTAAGTAACAAGAGGCGGAATTAG AGCACAGATCCCCAGACACCAATTCAGATCCTAGGAAGTCT CAGTTTTTAGAGTATTTACTATCAGTGTTCTTTTTTTTTCTGA CTTCTTGCTGCTTGAGTTTTATAATGTCTAATAAATTGTATT TTAGCTGTGGAGGAAGATGCAGAGTCAGAAGATGAAGAGG AGGAGGATGTGAAACTC |
| 300 | GenBank: S82725 | AAAGTTCCTTTTCCCATGTGCTCTTTTTTTTTTTTTTTTAAA TAGAATAGAAGTCTCAGTTTTTAGAGTATTTACTATCAGTG TTCTTTTTTTTTCTGACTCTCAGTTTTTAGAGTCATTTACTAT CAGTGTTCTTTTTTTTCTGACCCCTGGGCCAGCTGCACCCTC AAATCCACTGCTGTGATTGCACTGAAGCTGCCCTACCCAAT GGCTGAGCACAGCAGAAATACTAAGGCAGGCCCAATTCCT GGGAGTCATGGGACTCCTCTGATGACTGACTTTGGCTCCAG AACCCCTTAGGGC |
| 301 | GenBank: AF186110 | AGTGTTTTGGTTTCTCCCACAGTATTCTGAAAAGGAGGACA AATATGAAGAAAGAAATTAAACTTCTGTCTGACAAACTGA AAGAGGCTGAGACCCGTGCTGAATTTGCAGAGAGAACGGT TGCAAAACTGGAAAAGACAATTGATGACCTGGAAGTGTAC CGCCGGAAGCACCAGGAGCTGCAAGCCATGCAGATGGAGC TGCAGAGCCCTGAGTACAAGCTGAGCAAGCTCCGCACCTC GACCATCATGACCGACTACAAACCCCAACTACTGCTTTGCT GGCAAGACCTCCTCCATCAGTG |

(continued)

| SEQ ID NO: | ID | Sequence |
|---|---|---|
| 302 | PMID: 18083107 | AAGCCAGGCAGTGTAGGGGCTTGGTGGTGGCCATCGAACCTGACCTCCACCTCTATCCGTATTAGGTCTTTGAGAGCTGGATGCACCATTGGCTCCTGTTTGAAATGAGCAGGCACTCCTTGGAGCAAAAGCCCACTGACGCTCCACCGAAAGATGATTTTTGGATACCAGAAACAAGTTTCATACTTTACTATTATAGTTGGAATATTTCTGGTTGTTACAATCCCACTGACCTTTGGTAAGTATAATAGAATTTTTAAAATAGGCAACAAACTGTTTACTTAATCATACCTGATTGATTTAT |
| 303 | PMID:18593 892 Variant 3a | ctgcagacaagcataaagatgtcatcatcaaccaagTgtaccgccggaagcaccaggagctg |
| 304 | PMID:18593 892 Variant 3b | atgtcaactcgcgaaaaaaacagccaagTgtaccgccggaagcaccaggagctg |

**Table 3B**

| Gene 1 | Gene 2 | SEQ ID: | gl/g2 | Start | End | Gap | Strand | Identity | Resulting transcript | Plus |
|---|---|---|---|---|---|---|---|---|---|---|
| ALK | NP M1 | 299 | gl: chr2:29447105-29447253 | 1 | 149 | | | 100 | non-functional | Not capt ured |
| | | | g2: chr5:170819618-170819766 | 156 | 304 | 7 | + | 100 | | |
| NPM 1 | AL K | 300 | **gl:** chr5:170819567-170819622 | 1 | 148 | | + | 97.9 | functional | Capt ured |
| | | | **g2:** chr2:29446876-29447024 | 156 | 304 | 8 | | 97.9 | | |
| TPM 4 | AL K | 301 | **gl:** chr19:16204323-16204563 | 1 | 156 | | + | 100 | functional | Capt ured |
| | | | **g2:** chr2:29446247-29446402 | 148 | 304 | -8 | | 97.4 | | |
| CD74 | RO S1 | 302 | **gl:** chr5:149784243-149784395 | 1 | 153 | | | 100 | functional | Capt ured |
| | | | **g2:** chr6:117645428-117645580 | 152 | 304 | -1 | | 100 | | |
| EML 4 | AL K | 303 | **gl:** chr2:42491846-42491871 | 1 | 36 | | + | 100 | functional | Capt ured |
| | | | **g2:** chr2:29446369-29446396 | 35 | 62 | -1 | | 100 | | |
| EML 4 | AL K | 304 | **gl:** chr2:42492064-42492091 | 1 | 28 | | + | 100 | functional | Capt ured |
| | | | **g2:** chr2:29446369-29446396 | 27 | 54 | -1 | | 100 | | |

**EXAMPLE 3. Exemplary Rules and Strategy for Primer Selection for Tiling Methods Provided Herein**

**Primer Design**

The following is an example of details of one approach for selecting primers for use in the one-sided nested PCR approach using primers that bind a tiled series of primer binding sites spaced across a target region of a target gene (i.e. gene of interest). Primers were designed for plus and minus strands of the target gene region with melting temperature (Tm) optimums of 58C and 61C (FIGS 4-6). Both relaxed (deltaG -6) verses strict (deltaG -3) primer sets were designed. The relaxed set had more windows covered with primers but can also contain potentially harmful primers that caused primer-dimers. Primers were ordered from IDT (Integrated DNA Technologies, Inc., San Diego, CA) with no tag on the Outer primers and a tag ACACGACGCTCTTCCGATCT (SEQ ID NO: 297) on the Inner primers.

Primer designs were generated with Primer3 (Untergrasser A, Cutcutache I, Koressaar T, Ye J, Faircloth BC, Remm M, Rozen SG (2012) "Primer3 - new capabilities and interfaces." Nucleic Acids Research 40(15):e115 and Koressaar T, Remm M (2007) "Enhancements and modifications of primer design program Primer3." Bioinformatics 23(10):1289-91) source code available at primer3.sourceforge.net). Primer specificity was evaluated by BLAST and added to the existing primer design pipeline criteria:

1. Plus (+) strand primers were generated for selected target regions. Target region sequences were targeted in windows every 20-50 bp. Each primer design window was 20-40 bp long from the window start. Primers were searched in two consecutive windows for pairing nested Outer and Inner primers. Outer primers were designed that targeted the right most, 5' (or leftmost on minus strand) coordinate of each region using Primer3. The rationale for windows was that an inner primer will be selected from every second window, and a matching outer primer (following rules described below) will be selected either from the same or previous (3') window but not farther away. Primers were generated using RunPrimer3.java with one_sided=true option. This mode of the program generates only one set of primers without generating a paired minus primer.

2. Primer specificities were determined using the BLASTn program from the ncbi-blast-2.2.29+ package. The task option "blastn-short" was used to map the primers against hg19 human genome. Primer designs were determined as "specific" if the primer has less than 100 hits to the genome and the top hit is the target complementary primer binding region of the genome and is at least two scores higher than other hits (score is defined by BLASTn program). This was done in order to have a unique hit to the genome and to not have many other hits throughout the genome.

3. Primers were grouped on each consecutive window to inner + outer pairs (see FIG. 5) with the following rules:

    a. There was an Outer/Inner primer pair every tiled window (30 bp window illustrated (FIG. 3))

    b. From every second window, a specific inner primer was tried based on output order by Primer3.

        i. A primer will be skipped if it overlaps >50% with any other inner primer that was already selected.

    c. An outer primer was attempted to be identified such that:

        i. Outer primers from the current and previous window (the one from inner primer) were tried to find a primer such that:

            1. The first base of the primer was before the first base of the inner primer (or after for minus primers)

            2. The part of the inner primer that doesn't overlap with the outer primer was between 5 and 20 bases

            3. The Outer primer was specific

            4. Primers were tested in the order given by Primer3 output

        ii. If (i) failed, try same as (i) except Outer primer was non-specific

        iii. If (ii) failed, try same as (i) except distance was 3 to 40 bases

        iv. If (iii) failed, try same as (i) except distance was 3 to 40 bases, and Outer primer was non-specific

v. If (iv) failed, try same as (i) except distance was 40 to 100 bases

vi. If (v) failed, try same as (i) except distance was 40 to 100 bases, and Outer primer was non-specific

d. None or minimal interactions with other primers (was tested separately for Inner and Outer primers)

e. Inner primers have no interactions with the plus strand tag sequence "ACACGACGCTCTTCCGATCT" (SEQ ID NO: 297)

f. Outer primers have no interactions with the minus strand tag sequence AGACGTGTGCTCTTCCGATCT (SEQ ID NO: 298)

g. The final selected primers were visualized in IGV (James T. Robinson, Helga Thorvaldsdóttir, Wendy Winckler, Mitchell Guttman, Eric S. Lander, Gad Getz, Jill P. Mesirov. Integrative Genomics Viewer. Nature Biotechnology 29, 24-26 (2011)) and UCSC browser (Kent WJ, Sugnet CW, Furey TS, Roskin KM, Pringle TH, Zahler AM, Haussler D. The human genome browser at UCSC. Genome Res. 2002 Jun;12(6):996-1006 ) using bed files and coverage maps for validation.

Primer sets with relaxed and strict deltaG thresholds (-6 vs -3) were designed for each of 58 and 61 Tm settings (including plus/minus strand and inner/outer primers, 4 pools per design). The final set of selected primers were assessed to see their coverage of each target region on each strand, and on the combination of each strand (termed as "both").

**EXAMPLE 4. Exemplary Method for Identifying Target Regions and Primers for Tiling Target Regions of TP53 in Ovarian Cancer**

The following provides an example of how primers can be designed for a method of the present invention for detecting a cancer gene mutation in a region of a cancer gene where various mutations are known to occur. In this embodiment, the mutation is typically not a gene fusion. Primers were designed as described above. Primer target regions included the following criteria: Included coding exons that contain 95% of the recurrent SNVs and small indels discovered in TCGA Ovarian study on the TP53 gene and the COSMIC database. The TCGA and COSMIC sequencing targeted only exonic regions of TP53. In TCGA, there were 316 patients, and in COSMIC 233 patients, where the number of patients with mutations (SNV+small indel) are shown in Table 4. 95.4% of patients have a mutation in these targets for the TCGA patient cohort.

**Table 4: Selected target regions for TP53**

| Chr | Start | End | Length | Features | Target-ID | TCGA Load (n=316) | COSMIC Load (n=233) |
|---|---|---|---|---|---|---|---|
| chr17 | 7,590,695 | 7,590,868 | 173 | 5’ | Target-1 | 0(0%) | 0(0%) |
| chr17 | 7,579,262 | 7,579,937 | 675 | 5’ and Exon-1 | Target-2 | 26 (8.2%) | 2 (0.9%) |
| chr17 | 7,578,127 | 7,578,861 | 734 | Exon-2 and Exon-3 | Target-3 | 129 (40.8%) | 60 (25.8%) |
| chr17 | 7,577,449 | 7,577,658 | 209 | Exon-4 | Target-4 | 66 (20.9%) | 39 (16.7%) |
| chr17 | 7,576,525 | 7,577,205 | 680 | Exon-5 and Exon-6 | Target-5 | 73 (23.1%) | 37 (15.9%) |
| chr17 | 7,573,877 | 7,574,083 | 206 | Exon-7 | Target-6 | 9 (2.9%) | 2 (0.9%) |
| chr17 | 7,571,720 | 7,573,058 | 1,338 | Exon-8 and 3’ | Target-7 | 0 (0%) | 0 (0%) |

UTR regions which were not tested in TCGA were also included in order to test whether there were additional mutations in the UTR regions even though they were not tested by exome panels. The literature has shown that there is potential diagnostic, microRNA altering mutations on the 3’ UTR (Li et al. "Single nucleotide variation in the TP53 3’ untranslated region in diffuse large B-cell lymphoma treated with rituximab-CHOP: a report from the International DLBCL Rituximab-CHOP Consortium Program", Blood 121(22):4529-40, 2013).

Primer target coverage was tested against target mutations on four additional genes, and exons 5 through 8 of TP53 that contain the majority of its mutations in ovarian cancer (Table 5). The coverage was tested with 75bp and 100bp read lengths excluding the primers (only the insert was counted towards the usable coverage). Table 6-8 provide coverage data. Tables 9-11 provide exemplary primer design criteria for Primer3.

**Table 5: Other Target Gene Regions**

| chr | start | end | gene_region |
|---|---|---|---|
| chr12 | 25,398,280 | 25,398,285 | KRAS_t1 |
| chr3 | 178,936,081 | 178,936,094 | PIK3CA_t1 |
| chr3 | 178,952,084 | 178,952,085 | PIK3CA_t2 |
| chr10 | 89,692,903 | 89,692,905 | PTEN_t1 |
| chr10 | 89,717,715 | 89,717,717 | PTEN_t2 |
| chr10 | 89,720,816 | 89,720,818 | PTEN_t3 |
| chr7 | 140,453,135 | 140,453,145 | BRAF_t1 |
| chr17 | 7,579,300 | 7,579,600 | TP53_t2s |
| chr17 | 7,578,170 | 7,578,560 | TP53_t3s |
| chr17 | 7,577,490 | 7,577,620 | TP53_t4s |
| chr17 | 7,576,840 | 7,577,160 | TP53_t5s |
| chr17 | 7,573,980 | 7,574,040 | TP53_t6s |

**Table 6: Plus Strand Design Coverage for Target Regions**

| Design | Plus Strand Primers | Positive Target Coverage 75bp | Positive Target Coverage 100bp |
|---|---|---|---|
| 58Tm Strict (-3) | 78 | 89% | 97% |
| 58Tm Relaxed (-6) | 90 | 90% | 98% |
| 61Tm Strict (-3) | 56 | 76% | 91% |
| 61Tm Relaxed (-6) | 59 | 76% | 87% |

**Table 7: Minus Strand Design Coverage for Target Regions**

| Design | Minus Strand Primers | Minus Target Coverage 75bp | Minus Target Coverage 100bp |
|---|---|---|---|
| 58Tm Strict (-3) | 88 | 88% | 93% |
| 58Tm Relaxed (-6) | 99 | 96% | 96% |
| 61Tm Strict (-3) | 79 | 86% | 90% |
| 61Tm Relaxed (-6) | 80 | 86% | 91% |

**Table 8: Combined Designs coverage on Target Regions**

| Design | Both Primers | Both Target Coverages 75bp | Both Target Coverages 100bp |
|---|---|---|---|
| 58Tm Strict (-3) | 166 | 100% | 100% |
| 58Tm Relaxed (-6) | 189 | 100% | 100% |
| 61Tm Strict (-3) | 135 | 97% | 99% |
| 61Tm Relaxed (-6) | 139 | 97% | 97% |

*Common Design Parameters*:

**Table 9: RunPrimer3.java ini file**

| Option | Value | Rationale |
|---|---|---|
| primer3 _path | /usr/local/bin/primer3_core | Version 2.3.6 |
| reference_genome_ path | /data/prod/share/bioinformatics/ References/hg19_snp138CommonMask | dbSNP masked reference |
| max_target_distance | 40 (Maximum bp length primer annealing to target) | Primers are selected at most 40 bp away from the provided target |
| One_sided | true | Only one primer generated |
| One_sided_left | True and false for plus strand versus minus strand designs | Two separate primer sets are generated, one for plus strand and one for minus strand. When set to true left primers. |

**Table 10: Primer-3 config file**

| Option | Value | Rationale |
|---|---|---|
| PRIMER_TASK | pick_ pcr_ primers | Regular task to pick primers |
| PRIMER_SALT_CORRECTIONS | 1 | Use SantaLucia JR (1998) |
| PRIMER_TM_FORMULA | 1 | Use SantaLucia JR (1998) |
| PRIMER_THERMODYNAMIC_OLIGO_ ALIGNMENT | 1 | Use thermodynamic models for hairpins and dimers |
| PRIMER_THERMODYNAMIC_TEMPLATE_ ALIGNMENT | 1 | use thermodynamic models for misannealing |
| PRIMER_MIN_SIZE | 15 | Minimum acceptable length for primer |
| PRIMER_OPT_SIZE | 23 | Optimal length for primer |
| PRIMER_MAX_SIZE | 35 | Maximum acceptable length for primer |
| PRIMER_WT_SIZE_GT | 0.03 | Very small penalty for longer primers |
| PRIMER_WT_SIZE_LT | 0.01 | No penalty for shorter primers |
| PRIMER_MIN_TM | OPT - 4 | Minimum acceptable melting temperature for primer oligo |
| PRIMER_OPT_TM | 58 or 61 | Optimal melting temperature for primer oligo |
| PRIMER_MAX_TM | OPT + 3 | Maximum acceptable melting temperature for primer oligo |
| PRIMER_WT_TM_LT | 1.5 | Penalty weight for primers with Tm lower than optimal. Lower Tm primers are penalized most. |
| PRIMER_WT_TM_GT | 0.5 | Penalty weight for primers with Tm over optimal. Higher Tm primers are penalized less compared to lower Tm. |
| PRIMER_MIN_GC | 20 | |
| PRIMER_OPT_GC_PERCENT | 50 | GC percent optimal is 50 percent and should be between 20 and 80 |
| PRIMER_MAX_GC | 80 | |

(continued)

| Option | Value | Rationale |
|---|---|---|
| PRIMER_WT_GC_PERCENT_LT | 0.02 | penalty for lower GC percent |
| PRIMER_WT_GC_PERCENT_GT | 0.02 | penalty for higher GC percent |
| PRIMER_MAX_END_GC | 3 | The maximum number of Gs or Cs allowed in the last five 3' bases of a left or right primer. Allow all. |
| PRIMER_MAX_POLY_X | 5 | Max 6 homopolymers allowed |

**Table 11: Other Design Parameters**

| Option | Value | Rationale |
|---|---|---|
| Min DeltaG Score | -3 or -6 | -3 designs have less interacting pairs. Primer pairs with extendable alignment scores between less than -3 are removed. For -6 designs, only those with score less than -6 are removed. We have also applied filters for non extendable alignment scores which may be removed in future versions for higher sensitivity. |
| chunkSize | 20 | Window size (in bps) for each design area. |
| numInteract | 500 | Maximum number of interactions for a primer with |
| | | less than -2 deltaG score. Primers with more interactions than this number usually interact with the tag sequence. |
| maxOverlap | 0.5 | Maximum fraction of overlap of a given primer with existing primers in the pool (inners and outers are tested separately) |
| blastScoreDiff | 2 | Minimum allowed difference between best blast alignment score and the second best score. If this score is less then specified, than the alignment is not considered specific. |
| blastMaxResults | 100 | If there are more than the specified number of blast alignments (above the minimum threshold) then the alignment is not considered specific. |
| primer_ concentration | 100 | For interaction.ini file |
| salt_concentration | 100 | For interaction.ini file |
| plus strand_tag | ACACGACGCTCTTC CGATCT (SEQ ID NO. 297) | For interaction.ini file |
| minus_tag | AGACGTGTGCTCTT CCGATCT (SEQ ID NO. 298) | For interaction.ini file |

**EXAMPLE 5. Exemplary One-Sided Nested Multiplex PCR Method with Target Specific Tiled Primers**

Multiplex, tiled primer pools (80-90 primers/pool (unidirectional plus strand primers without a paired minus strand primer)) were generated on the basis of in silico analysis of primer compatibility. Considerations included: partitioning overlapping amplicons into separate primer pools, minimizing the probability of primer-dimer formation and ensuring similarity of guanine, cytosine (GC) content within a single pool. Primers were pooled at equal molar quantities.

An outer plus strand primer pool and a pooled outer minus strand primer pool were separately amplified in a first amplification round. For amplification of the pooled plus or minus strand primers, the following PCR conditions were used in a 50uL reaction volume: 1.25 units of PrimeSTAR GXL DNA polymerase, 1-X PrimeSTAR GXL reaction buffer (both from Clonetech), 200uM of each dNTP, 25nM of each specific plus or minus strand primer, 2.5 uM of universal reverse primer and 1 ug of amplified library as a template. Alternatively, a non-amplified library can also be used. The library was doubled-stranded DNA with Adapters ligated to each end of the DNA strands. The first round of PCR amplification was performed under the following conditions: 98°C 1 min, 15x [98°C 10 sec, 63°C 15 min, 68°C 1 min], 68°C 2 min, 4°C hold (PCR No. 1). The amplification product was diluted 1:200 in water and 2 ul was added as a template into the second round of PCR amplification reaction (10 ul total volume). FIG. 6A illustrates the first round of the PCR amplification reaction with target specific primer(s) on one side and a universal reverse primer.

A second nested PCR amplification round was subsequently separately performed using a pooled inner plus strand primer pool and a pooled inner minus strand primer pool using the amplicons generated from the first round of amplification (PCR No. 1). The second PCR amplification round of pooled inner plus strand primers and pooled inner minus strand primer pools contained 0.25 units of PrimeSTAR GXL DNA polymerase, 1-X PrimeSTAR GXL reaction buffer, 200uM of each dNTPs, 10nM of each specific inner plus strand primer or 10 nM of each specific inner minus strand primer, 1 uM of universal reverse primer, and 2ul of diluted outer plus strand primer amplification product or minus strand amplification product from the first amplification round. The second round of PCR amplification was performed under the following conditions: 98°C 1 min, 15x [98°C 10 sec, 63°C 15 min, 68°C 1 min], 68°C 2 min, 4°C hold (PCR No. 2, Nested). FIG. 6B illustrates the second round of Nested PCR amplification reaction with target specific primer(s) on one side with a universal reverse primer. The workflow for Nested PCR with tiled target specific primers on one side is illustrated in FIGS. 7A-7B.

The amplified products were barcoded. One run of sequencing was performed with an approximately equal number of reads per sample.

Table 12 shows sequencing results from the analysis of simulated cfDNA sample using different library input concentrations. The Depth of Read (DOR) uniformity is shown for Plus strand design (FIG. 8A) and Minus strand design (FIG. 8B) with each pool having approximately 80-90 primers pooled and tiled across a genomic target region. FIG. 8C illustrates uniformity of coverage showing the combined coverage obtained with both Plus and Minus strand primer designs of the entire TP53 gene. Fig. 11 provides an exemplary analytic flow that can be used to detect SNVs in any gene, including the TP53 gene (See right side "SNV Detection") based on high throughput sequencing data, such as that generated in this example. Details regarding how this SNV detection analysis can be performed according to the method of FIG. 11 are provided in this specification.

**Table 12: Sequencing Results for plasma samples using different input amounts**

| Primer Cone, nM | Library Input in PCR No. 1, ng | Tiling Direction | total_reads | mapped_ fraction | on_ target | mapped*on_ target |
|---|---|---|---|---|---|---|
| 5 | 200 | + | 1,253,785 | 94.0% | 56.9% | 53.5% |
| 5 | 200 | + | 1,756,706 | 93.8% | 57.4% | 53.8% |
| 5 | 600 | + | 2,416,162 | 91.5% | 67.3% | 61.6% |
| 5 | 600 | + | 3,315,134 | 92.2% | 68.9% | 63.5% |
| 5 | 1000 | + | 3,259,584 | 90.5% | 67.5% | 61.1% |
| 5 | 1000 | + | 3,283,963 | 89.4% | 69.2% | 61.8% |
| 25 | 200 | + | 2,256,360 | 92.4% | 59.9% | 55.4% |
| 25 | 200 | + | 2,040,110 | 92.0% | 60.5% | 55.7% |
| 25 | 600 | + | 3,604,738 | 91.7% | 64.7% | 59.3% |
| 25 | 600 | + | 4,175,099 | 91.4% | 63.9% | 58.4% |
| 25 | 1000 | + | 4,346,821 | 89.7% | 64.8% | 58.2% |
| 25 | 1000 | + | 3,570,408 | 90.2% | 63.9% | 57.7% |
| 50 | 200 | + | 2,218,224 | 90.7% | 53.1% | 48.2% |
| 50 | 200 | + | 2,617,914 | 90.7% | 52.3% | 47.4% |

(continued)

| Primer Cone, nM | Library Input in PCR No. 1, ng | Tiling Direction | total_reads | mapped_ fraction | on_ target | mapped*on_ target |
|---|---|---|---|---|---|---|
| 50 | 600 | + | 3,731,977 | 88.6% | 56.3% | 49.9% |
| 50 | 600 | + | 3,273,555 | 88.6% | 54.6% | 48.4% |
| 50 | 1000 | + | 3,504,746 | 88.1% | 51.3% | 45.2% |
| 50 | 1000 | + | 3,951,828 | 88.2% | 54.0% | 47.6% |
| 5 | 200 | - | 1,755,569 | 91.6% | 57.9% | 53.0% |
| 5 | 200 | - | 2,449,005 | 92.5% | 57.0% | 52.8% |
| 5 | 600 | - | 2,871,767 | 91.9% | 68.7% | 63.2% |
| 5 | 600 | - | 2,590,101 | 91.8% | 69.0% | 63.3% |
| 5 | 1000 | - | 3,675,282 | 90.6% | 73.7% | 66.8% |
| 5 | 1000 | - | 3,818,799 | 91.0% | 73.6% | 66.9% |
| 5 | 200 | - | 4,611,083 | 87.2% | 48.7% | 42.5% |
| 25 | 200 | - | 4,526,120 | 88.1% | 54.3% | 47.8% |
| 25 | 600 | - | 5,794,201 | 86.9% | 57.8% | 50.2% |
| 25 | 600 | - | 5,041,755 | 87.7% | 56.7% | 49.7% |
| 25 | 1000 | - | 5,567,632 | 87.1% | 59.3% | 51.7% |
| 25 | 1000 | - | 4,860,506 | 86.9% | 60.2% | 52.3% |
| 25 | 200 | - | 5,202,605 | 82.6% | 40.3% | 33.3% |
| 50 | 200 | - | 5,711,641 | 84.3% | 41.2% | 34.8% |
| 50 | 600 | - | 5,810,409 | 85.1% | 47.3% | 40.2% |
| 50 | 600 | - | 5,813,149 | 84.1% | 52.9% | 44.5% |
| 50 | 1000 | - | 6,443,046 | 84.7% | 49.4% | 41.9% |
| 50 | 1000 | - | 6,472,887 | 85.2% | 51.8% | 44.1% |
| * total fraction of useful TP53 reads (e.g., 94.6 x 56.9 /100= 53.5%) | | | | | | |

**EXAMPLE 6. Exemplary One-Sided Nested One Step Multiplex PCR Method with Target Specific Tiled Primers**

Multiplex, tiled primer pools (plus strand primers without a paired minus strand primer) were generated on the basis of in silico analysis of primer compatibility. Considerations included: minimizing the probability of primer-dimer formation and ensuring similarity of guanine+cytosine (GC%) content within the pool. Primers were pooled at equimolar concentrations.

For amplification with the pooled primers, the following PCR conditions are used in a 10uL reaction volume: 0.25 units of PrimeSTAR GXL DNA polymerase, 1X PrimeSTAR GXL reaction buffer (both from Clonetech), 200uM of each dNTPs, 10nM of each primer, 1 uM of universal reverse primer, and 1 ug of amplified library as a template. Alternatively, a non-amplified library can also be used. The library is doubled-stranded DNA with Adapters ligated to each end of the DNA strands. The PCR amplification is performed under the following conditions: 98°C 1 min, 15x [98°C 10 sec, 63°C 15 min, 68°C 1 min], 68°C 2 min, 4°C hold. The amplification products are then barcoded in a subsequent PCR step and sequenced. One run of sequencing is performed with an approximately equal number of reads per sample.

**EXAMPLE 7. Exemplary PCR with Tiled Target Specific Inner Primer(s) on Two Sides**

Multiplex, tiled primer pools (80-90 primers/pool (unidirectional plus strand inner primers without a paired minus strand primer)) were generated on the basis of in silico analysis of primer compatibility. Considerations included: partitioning overlapping amplicons into separate primer pools, minimizing the probability of primer-dimer formation and ensuring similarity of guanine, cytosine (GC) content within a single pool. Primers were pooled at equal molar quantities.

Two PCR reactions containing inner Plus and inner Minus strand primer pools with each primer in the pools having a tag and a universal reverse primer present in each reaction were amplified individually. The following PCR conditions were used in a 50uL reaction volume: 1.25 units of PrimeSTAR GXL DNA polymerase, 1-X PrimeSTAR GXL reaction buffer (both from Clonetech), 200uM of each dNTP, 25nM of each specific plus or minus strand primer, 2.5 uM of universal minus strand primer and 1 ug of amplified library as a template. The library was doubled-stranded DNA with Adapters ligated to each end of the DNA strands. It is noted that if the quantity of starting DNA is relatively high, e.g. sheared genomic DNA, the starting DNA would not be in library format. The first round of PCR amplification was performed under the following conditions: 98°C 1 min, 15x [98°C 10 sec, 63°C 15 min, 68°C 1 min], 68°C 2 min, 4°C hold. The amplification product was diluted 1:200 in water. 2 ul of the diluted amplification product were added as the template into the second round PCR amplification reaction (10 ul total volume). FIG. 6A illustrates the first round of Nested PCR amplification reaction with target specific primer(s) on one side and a universal reverse primer on the other side. The amplified products were barcoded. One run of NGS sequencing was performed with an approximately equal number of reads per sample. Sequencing data was analyzed to identify determinative cancer mutations or fusions.

Assuming all primer designs worked, coverage of 100 bp can be calculated for the primer inserts to visualize them across the entire TP53 region and focus on specific exons

**EXAMPLE 8. Detection of Gene Fusions by PCR Using Tiled Gene Specific Primers**

Figures 9A-9B illustrate the disclosed three approaches for detecting gene fusions. In the One-Sided nested multiplex PCR tiling approach using target specific primers on one side, multiplex PCR pools of outer and inner primers with universal reverse primers are prepared to provide amplicons for sequencing across a chromosomal breakpoint and hence a gene fusion (FIG. 9A Top - Star1-Star2). If there is no breakpoint and thus no gene fusion, only the wildtype gene is read when sequenced. In the One-Sided multiplex PCR approach it too uses target specific primers on one side and multiplex PCR pools of DNA primers with universal reverse primers for sequencing across a chromosomal breakpoint and hence a gene fusion (FIG. 9B). Again, if there is no breakpoint and thus, no gene fusion, only the wildtype gene is read when sequenced. In the Two-Sided, one step multiplex PCR with target specific tiled primers approach (FIG. 9A Bottom - OneSTAR) if a gene fusion has occurred there will be an amplified PCR product spanning the breakpoint for reading by sequencing. But if there is no gene fusion, there is no sequencing read as there would be no amplified read in the region targeted by the left and right primers. The first and third methods were further tested using a 160 bp fusion spike.

A One-Sided nested multiplex PCR method with target specific tiled primers for detection of a gene fusion was performed as follows.

A pooled outer plus strand primer pool and a pooled outer minus strand primer pool were separately used for PCR amplification reactions as were pooled nested inner plus strand primers and pooled inner minus strand primers. For amplification of each of the two outer target specific primer pools, the following PCR conditions were used in a 20uL reaction volume: 1x Master Mix with 200uM of each dNTP, 1-X Master Mix reaction buffer, 25nM of each specific outer primer- in a pool of 60-90+ primers or 25 nM of each specific minus strand primer- in a pool of 60-90+ primers, 2.5 uM of universal reverse primer and 4 uL plateaued library as a template. The library was doubled-stranded DNA with Adapters ligated to each end of the DNA strands. The first round of PCR amplification was performed under the following conditions: 95°C 10 min, 15x [95°C 30 sec, 63°C 10 min, 72°C 2 min], 72°C 7 min, 4°C hold. The amplification product was diluted 1:20 in water and 2 ul was added as a template into the second round of nested PCR amplification reaction (10 ul total volume). 72°C?;

A pooled inner plus strand nested target specific primer pool and a pooled inner minus strand nested target specific primer pool were separately for PCR amplifications. The second PCR amplification round of pooled inner nested target specific primers contained 1x Master Mix with 200uM of each dNTP, 1x Master Mix reaction buffer, 40nM of each specific inner plus strand primer- in a pool of 60-90+ primers or 25 nM of each specific minus strand primer- in a pool of 60-90+ primers, 1 uM of universal reverse primer and 2ul of diluted outer plus strand primer amplification product or outer minus strand primer amplification product. The amplicons from the first PCR round using the outer plus strand primer pool is used with the inner plus strand nested target specific primer pool and the amplicons from the first PCR round using the outer minus strand primer pool is used with the inner minus strand nested target specific primer pool. The second round of PCR amplification was performed under the following conditions: 95°C 10 min, 15x [95°C 30 sec, 63°C 10 min, 72°C 2 min], 72°C 7 min, 4°C hold.

The amplified products from the second nested PCR amplification reactions were barcoded in a 10 uL reaction volume comprising 1x Qiagen Master Mix, 0.5 uM Plus Strand Barcode, 0.5 uM Minus strand Barcode, 1 uL amplification product from the second PCR amplification round of pooled inner primers diluted 1;20. The bar coding reaction was 95°C 10 min, 12x [95°C 30 sec, 62.5°C 3 min, 72°C 2 min], 72°C 7 min, 4°C hold. Following barcoding, the reactions were pooled, purified and one run of sequencing was performed with an approximately equal number of reads per sample.

Results for the TMP4-ALK visualization by One-Sided multiplex PCR are illustrated in FIG. 10A. The wildtype ALK One-Sided nested multiplex PCRis indicated on the top track sequencing read and the TPM4:ALK_9 breakpoint is shown on the lower track sequencing read. Readily apparent is that the fusion spike crosses the fusion boundary while the ALK wildtype amplification product does not cross the breakpoint (coverage at breakpoint is 33,855 reads vs. 34 for wildtype).

Results for the NPM1-ALK_9 visualization by One-Sided multiplex PCR are illustrated in FIG. 10B. The wildtype ALK One-Sided nested multiplex PCR is indicated on the top track sequencing read and the NPM1-ALK_9 breakpoint is shown on the lower track sequencing read. Readily apparent is that the fusion spike crosses the fusion boundary while the ALK wildtype amplification product does not cross the breakpoint (coverage at breakpoint is 12,437 reads vs. 33 for wildtype).

A Two-Sided, one step multiplex PCR method with target specific tiled primers for detection of a gene fusion was performed as follows:

A pooled inner plus strand target specific primer pool and a pooled inner minus strand target specific primer pool were combined and amplified for detection of a CD74_ROS1_13 fusion. The PCR amplification round of pooled inner plus strand and minus primers contained 1x Master Mix with 200uM of each dNTP, 1x Master Mix reaction buffer, 50nM of each specific inner plus strand and minus strand primer, and 4 uL plateaued library in a 10 uL total volume. PCR amplification was performed under the following conditions: 95°C 10 min, 30x [95°C 30 sec, 63°C 10 min, 72°C 30 sec], 72°C 2 min, 4°C hold.

The amplified products were barcoded in a 10 uL reaction volume comprising 1x Qiagen Master Mix, 0.5 uM Plus Strand Barcode, 0.5 uM Minus strand Barcode 1 uL OneSTAR amplification product diluted 1;20 in a 10 uL total volume. The bar coding reaction was 95°C 10 min, 12x [95°C 30 sec, 62.5°C 3 min, 72°C 2 min], 72°C 7 min, 4°C hold. Following barcoding, the reactions were pooled, purified and one run of sequencing was performed with an approximately equal number of reads per sample.

Results for the CD74_ROS1_13 visualization by Two-Sided, one step multiplex PCR with target specific tiled primers are illustrated in FIG. 10C. The wildtype CD74 by Two-Sided PCR with target specific tiled primers is indicated on the lower track sequencing read and the CD74_ROS1_13 breakpoint is shown on the upper track sequencing read. Readily apparent is that the fusion spike crosses the fusion boundary while the CD74 wildtype amplification product does not cross the breakpoint (coverage at breakpoint is 17,386 reads vs. 4 for wildtype).

**Data Analysis**

**Supplementary Read Analysis:**

For the above analysis, alignments were performed as follows: Sequencing reads from both strands (fast1 plus and fast1 minus) were assembled using a paired-end analysis. The assembled sequence was mapped to a publicly available reference genome without the on-test fusions using the BWA Aligner program. The BWA Aligner program reported supplementary alignments as alignments of reads that have a primary alignment that can explain the unmapped portion of the primary alignment. Sometimes there were multiple supplementary alignments for each primary alignment. By building a linkage map of the primary-supplementary alignment pairs, the breakpoints in the data were discovered. Breakpoints, as used herein, refers to the fusion of two sequences that would otherwise not be linked as they are too far apart from each other such that their fusion cannot be explained by a local mutation. The breakpoints identified in the mapped data, were either gene fusions or artifacts. Background noise was determined from negative samples and eliminated. Breakpoints were identified by determining whether the total number of breakpoint reads exceeded a cutoff.

Further analysis of the initial or seed fusion calls can be made by building a donor, acceptor fusion template based on the seeding fusion calls as indicated in Figure 11. The reads can then be remapped to the donor, acceptor fusion template in place of the publicly available reference genome without the fusion.

**Paired-End Bridge Analysis:**

Sequencing reads can be mapped in paired-end mode, where each sequenced strand is mapped separately, rather than after they are combined in paired end analysis. If sequencing reads are found to map on one fusion gene and its sequencing mate maps confidently on the fusion partner, then the sequence read can be counted as evidence of a detected fusion bridge. The bridge maps can be produced for the target regions and reported similar to supplementary read analysis. The counts of bridge reads versus breakpoint reads for one barcode can be analyzed and compared first and then metrics can be built to report them for all the barcodes. Thus, detection of breakpoints can be verified.

**EXAMPLE 9 One-Sided PCR Tiling Detection of Fusions to Assess De Novo Limit of Detection.**

A nested one-sided PCR tiling method was performed for the detection of gene fusions and to assess the limit of detection of the method. The experiment focused on EML4-ALK, TPM4-ALK and CD74-ROS 1 fusions and tested the detection of several specific rearrangements between those genes at low input percentages using a de-novo detection analysis algorithm. A titration series was performed on two independent gene fusion constructs generated by PCR amplification and on monosomal DNA generated from a fusion cell line, followed by measurement of the detected fusions using a nested one-sided tiling PCR embodiment of the present invention, and a de novo fusion detection algorithm.

**Methods**

A series of synthetic polynucleotides were created to mimic nucleic acid fragments that occur in circulating DNA in vivo, that include nucleic acid sequences from known fusion partner genes across a known genetic fusion breakpoint. To create the synthetic polynucleotides mimicking a TPM4:ALK and CD74:ROS1 fusion event, a synthetic oligonucleotide template with the indicated fusion sequence was PCR-amplified using primers shown in Table 13 under standard PCR conditions. The resulting amplified fragments were used for the titration experiment below, at each input percent.

**Table 13 Primers for Spike PCR.**

| Primer Name | Sequence | Primer Length | $T_m$ (°C) | SEQ ID NO. |
|---|---|---|---|---|
| F-CD74: ROS1_15 | CAAAAGCCCACTGACGCTC | 19 | 53.79 | 305 |
| R-CD74: ROS1_15 | TTAAGTAAACAGTTTGTTGCCTATTTTAAA AATT | 34 | 53.36 | 306 |
| F-TPM4:ALK_ 13 | GCAGAGAGAACGGTTGCAAA | 20 | 53.35 | 307 |
| R-TPM4:ALK_ 13 | GGGGTTTGTAGTCGGTCATGA | 21 | 53.85 | 308 |

The fusion spikes were quantified using the HS Qubit® nucleic acid quantitation kit (Thermo Fisher, Carlsbad, CA), and diluted in 1ng/µl wild type monosomal DNA. H2228 fusion cell line DNA, digested with micrococcal nuclease (MNase) was purified to monosomal DNA (AG16778 68ng/ul (B-Lymphocyte cell line), Coriell Institute for Medical Research, Camden, New Jersey, USA). H2228 fusion cell line genomic DNA (gDNA) was fragmented with the NEB Fragmentase kit (NEB, Ipswich, MA). A quality control assay (QC) was performed on both fragmented and monosomal H2228 Fusion cell line DNA on a Bioanalyzer (Agilent Technologies, Santa Clara, CA). The fragmented and monosomal H2228 fusion cell line DNA and wild type cell line monosomal DNA (AG16778 68ng/ul) were quantified using a Qubit® dsDNA BR Assay Kit for nucleic acid quantitation (Thermo Fisher, Carlsbad, CA). A total of 27 samples were prepared. As indicated above, two fusion spikes were made using amplicons generated by amplifying template DNA with the CD74:ROS1 primers in Table 13 above, and the other by amplifying template DNA with the TPM:ALK primers in Table 13. The two fusion spike amplicons were added individually at 10%, 1%, 0.5%, 0.1% and 0.05% input to 50,000 copies of wild type DNA (total of 10 samples with 5 samples/spike). Monosomal H2228 DNA was added at 100% (10,000 copies), 10%, 1%, 0.5%, 0.1% and 0.05% input to 50,000 copies of wild type DNA, in duplicate (forming a total of 12 samples). Three negative samples contained monosomal DNA, (50,000 copies). It is noteworthy that for H228, the cell line is assumed to be heterozygous for the gene fusion, which cuts the detectable percentages in half.

Briefly, nucleic acid libraries from the various DNA template samples disclosed above were prepared using the Natera Library prep kit (Natera, San Carlos, CA). The Library preparation reagents were used to transform cell-free DNA (cfDNA) fragments into an amplified library of DNA molecules, each consisting of the original cfDNA sequences flanked by a specific synthetic adapter DNA. The 3' or 5' overhangs of cfDNA fragments were converted to blunt ends using a polymerase followed by adding a single 3' A nucleotide to blunt-ended cfDNA fragments to enhance annealing and ligation of adapters. Synthetic adapter sequences were ligated with a single 3' T nucleotide at both ends of A-tailed cfDNA fragments. The adapter-ligated library generated with the library preparation reagents was then amplified by PCR using forward and reverse primers complementary to the adapters. The PCR amplification was performed under the following conditions: 95°C 2 min, 9x [95°C 20 sec, 55°C 20 sec, 68°C 20 sec], 68°C 2 min, 4°C hold. The PCR products were purified using Agencourt Ampure beads. The purified cfDNA library was stored at -10oC to -30oC in DNA suspension buffer (10mM Tris, pH 8.0, 0.1mM EDTA).

Quality control of the libraries was performed using a LabChip DNA analysis instrument (PerkinElmer, Waltham, MA). A multiplex, nested one-sided PCR reaction was performed by carrying out a first PCR, called "Starl," using a series of 148 forward target-specific outer primers and a reverse outer universal primer, to generate outer primer target amplicons. Next a second PCR, called "Star2," was performed by amplifying a portion of the outer primer target amplicons using a series of 148 forward target-specific inner primers and a reverse inner universal primer.

Barcodes were then added to the inner primer target amplicons by performing a barcoding PCR reaction on the 27 samples. A pooled sample for sequencing of the inner primer target amplicons was prepared by combining 2ul from each of the 27 samples. The sequencing sample was purified using Qiagen PCR purification kit and quantified using Qubit BR. The sample was sequenced (100 bp paired-end and single-index) using the HiSeq 2500 System and TruSeq Rapid SBS Kits (200 Cycle and 50 Cycle) (Illumina, San Diego, CA). The expected average DOR was calculated to be ~37,500 reads/assay based on 148 assays with 50% on target reads of a total of 300,000,000 reads, 150,000,000 on target reads and 5,500,000 reads/sample. The method is discussed in more detail below.

**Table 14 Spike sequences used for titration.**

| Spike ID | Sequence | SEQ ID NO. |
|---|---|---|
| CD74: ROS1 _15 | CAAAAGCCCACTGACGCTCCACCGAAAGATGATTTTTGGA TACCAGAAACAAGTTTCATACTTTACTATTATAGTTGGAAT ATTTCTGGTTGTTACAATCCCACTGACCTTTGGTAAGTATA ATAGAATTTTTAAAATAGGCAACAAACTGTTTACTTAA | 309 |
| TPM4: ALK_ 13 | GCAGAGAGAACGGTTGCAAAACTGGAAAAGACAATTGAT GACCTGGAAGTGTACCGCCGGAAGCACCAGGAGCTGCAA GCCATGCAGATGGAGCTGCAGAGCCCTGAGTACAAGCTGA GCAAGCTCCGCACCTCGACCATCATGACCGACTACAAACC CC | 310 |

STAR1 protocol: A one-sided outer PCR reaction mixture was formed that included the following: 25 nM of each fusion 1 outer pool primer (forward target-specific outer primers) (see FIGS. 13A-13H for list of target-specific tiled outer ALK and ROS primers used for this experiment), 2.5 uM RStar2_C3_Loop (outer universal primer), 4 ul plateaued library (nucleic acid library) were added into an in-house reaction mixture, sometimes referred to herein as the K23 master mix. The K23 master mix included the following concentrations within the final PCR reaction mixture: 75 mM Tris pH 8.0 (TekNova T1080); 5 mM $MgCl_2$ ; 30 mM KCl; 60 mM $(NH_4)_2SO_4$; 150 U/mL AmpliTaq Gold 360; 0.2 mM each dNTP (N0447S); and 3% Glycerol. The PCR amplification protocol followed was: 95 °C 10 min; 15x [95 °C for 30 sec, 63 °C for 10 min, 72 °C for 2 min]; 72 °C for 7 min, and a 4 °C hold.

STAR2 protocol: A one-sided inner PCR reaction mixture was formed that included the following: 40 nM of each fusion 1 Inner pool primer (forward, target-specific inner primers) (see FIGS. 13A-13H for list of target-specific tiled inner ROS and ALK primers used for this experiment), 1 uM RStar2 (inner universal primer), 2 ul Starl product (outer primer target amplicons) diluted 1:20. The PCR amplification protocol followed was: 95 °C 10 min; 15x [95 °C for 30 sec, 63 °C for 10 min, 72 °C for 2 min]; 72 °C for 7 min, and a 4 °C hold.

Barcoding protocol: The following, barcoding reaction mixture was formed: 1X Qiagen Master Mix (Qiagen, Germany), 0.5 uM F-BC-barcode, and 1 μl Star2 products (1:20 dilution inner primer target amplicons). The Bar Coding PCR amplification protocol followed was: 95 °C 10 min; 12x [95 °C for 30 sec, 62.5 °C for 3 min, 72 °C for 2 min]; 72 °C for 7 min, and a 4 °C hold.

Sample pooling and sequencing: A_sequencing pool was prepared by combining 2μl from each of the 27 samples. The pool was PCR purified using the Qiagen kit and quantified using BR Qubit. The pool was run on one HiSeq2500 100 bp paired-end, single index run. Pool concentration was determined using Qubit BR. The pool concentration was 377 nM.

Analysis was performed as set out in Example 8.

**Results**

Analysis of Primer counts using the inner and outer tiled ALK and ROS primers included in the one-sided nested multiplex tiled PCR methods herein generated the following reads: 198,695,383 total bamreads; 176,491,830 total mapped reads; 101,947,168 total mapped on target reads; ~89% mapped reads; ~51% mapped on target reads; and uniformity $90^{th}/10^{th}$ percentile of 61. Thus, about 50% of the total reads mapped as on target reads, which was consistent with other similar experiments. The same was true for uniformity, which was about 60% for this fusion pool.

Fusion percentages were calculated based on total fusion reads detected for individual primers. The sum of the fusion reads for one primer and multiple cigarstrings was calculated and divided by the total primer counts. This includes wild type reads and reads too short for alternative mapping (31bp minimum length after breakpoint), which might reduce the percent detected fusions for spikes with primer sites further away from the fusion breakpoint.

The EML4-ALK tiling assay was titrated using monosomal H2228 fusion cell line DNA. Noteworthy, the data was pre-selected for reads that map to EML4, all "false positive fusions" were excluded.

**Table 15: Fusion input, detected, non-assigned reads, fusion reads and total primer count for monosomal EML4_ALK.**

| Input % (heterozygous) | Detected % | Non Assigned Reads | Fusion Reads | Total Primer Count | No. # Cigarstrings |
|---|---|---|---|---|---|
| 50 | 70.31% | 5171 | 12245 | 17416 | 60 |
| 5 | 26.10% | 3548 | 1253 | 4801 | 31 |
| 0.5 | 2.20% | 4770 | 105 | 4875 | 7 |
| 0.25 | 1.35% | 4077 | 56 | 4133 | 5 |
| 0.05 | 0.31% | 3882 | 12 | 3894 | 1 |
| 0.025 | 0.20% | 4526 | 9 | 4535 | 1 |

As shown in Table 15, the pure monosomal DNA from the fusion cell line (100%) reached 70% fusion detection. There were ~5000 non assigned reads, which are reads that are too short to map to EML4 and wild type ALK reads. Noteworthy, 100% wildtype samples had an average of 4400 reads corresponding to the primer ALK_r201_i, which demonstrates the likelihood of the non-assigned reads to be wild type reads.

Not to be limited by theory, it is suspected that amplicon generation for the fusion reads had a higher efficiency (17,000 reads fusion vs 4400 reads wt) because no other primers were downstream for the fusion gene. On the other hand, for amplification of the wild type region the primer had to compete with downstream primers, see FIG. 12.

The EML4-ALK fusion was detected by primer ALK_r201_i and led to product sizes between 62bp and 155bp with 1-60 cigarstrings depending on the % input. The breakpoint was 11bp behind the primer end (total ALK amplicon length 31bp). The break was detected between EML4 Exon 6-Intron6 and ALK Intron19-Exon20, which fuses EML4-Exon6 with Exon20 creating Variant 3. This variant was previously reported for the H2228 cell line by Rikova et al. 2007.

Fragmented DNA from the H2228 cell line led to a detection of 20% fusion in a 100% fusion sample. This is a much lower percentage compared to the monosomal H2228 DNA, which yielded 70% fusion reads. The number of reads for primer ALK_r201_i for both 100% fragmented DNA samples tested were ~40,000, which is more than 2 times higher than the monosomal DNA sample (~17,500). Nevertheless, those samples fail to show similar performance.

Using the ROS tiled primers, the titrated ROS-CD74 spikes were not detected at the expected quantity, see Table 16. A very low percentage was detected in this experiment. It is believed that the reason for this is the long amplicon length necessary for successful mapping of this fusion. The ROS primers were placed at relatively large distances such that the only primer that bound within the spike sequence was 68bp away from the breakpoint. This means, a minimum amplicon length of about 120bp, a distance between the primer start site and the end of the synthetic spike, needed to be reached, considering the ROS primer was 24 nucleotides long and about 30 nucleotides of the CD74 gene need to be sequenced to identify the CD74 gene. This is double the amplicon length compared to the TPM4-ALK spike tested, which likely explains the discrepancy between input and detected percent for the ROS-CD74 spike. However, The DOR for the ROS primer was extremely high, which allowed a detection of 0.01% ROS-CD74 spike, see Table 16.

**Table 16 Fusion input, detected, non-assigned reads, fusion reads and total primer counts for spike ROS-CD74**

| Input % | Detected % | Non Assigned Reads | Fusion Reads | Total Primer Count | No. Cigarstrings |
|---|---|---|---|---|---|
| 10.00 | 0.89% | 76855 | 687 | 77542 | 9 |

(continued)

| Input % | Detected % | Non Assigned Reads | Fusion Reads | Total Primer Count | No. Cigarstrings |
|---|---|---|---|---|---|
| 1.00 | 0.07% | 87618 | 65 | 87683 | 2 |
| 0.50 | 0.05% | 75761 | 37 | 75798 | 1 |
| 0.10 | 0.01% | 62462 | 6 | 62468 | 1 |
| 0.05 | 0.01% | 82268 | 12 | 82280 | 1 |

The TPM4-ALK tiled PCR assay was titrated using a representative PCR amplified spike with a template having 48 nucleotides of TPM4 fused to 112 nucleotides of ALK. TPM4-ALK spikes were detected at expected percentages within the range of error, see Table 16. Low DOR for this primer did not allow detection below 0.1% input fusion DNA. Percentages seem accurate for this spike, because of the short amplicon length.

**Table 17. Fusion input, detected, wt reads, mutant reads and total reads for spike TMP4-ALK**

| Input % | Detected % | Non Assigned Reads | Fusion Reads | Total Primer Count | No. Cigarstrings |
|---|---|---|---|---|---|
| 10.00 | 9.62% | 3083 | 328 | 3411 | 1 |
| 1.00 | 1.40% | 2896 | 41 | 2937 | 1 |
| 0.50 | 0.59% | 2527 | 15 | 2542 | 1 |
| 0.10 | 0.30% | 3028 | 9 | 3037 | 1 |
| 0.05 | N/A | N/A | N/A | N/A | N/A |

**Conclusions**

De novo gene fusion detection was successful for EML4-ALK in monosomal DNA down to 0.05% input nucleic acids using the nested one-sided PCR approach provided herein. Furthermore, using this method a 0.1% TPM4-ALK spike (input) was detected in this experiment. The next lower titration point did not get any fusion reads, probably because the DOR was ~3,000 for this primer. The Ros-CD74 spike was detected down to 0.05% input using the nested one-sided PCR method. The Ros assay detected 6 fusion reads in 60,000 total reads (0.01 % quantified). A low number of fusion reads detected for ROS-CD74 was due to a relatively long amplicon length needed for mapping considering the PCR conditions under which the assay was performed. All ROS assays had on average a higher DOR (~58,000) and better uniformity compared to the ALK Assays (~41,000). The method of analysis used in this example does not allow quantification of wild type reads because only split reads are analyzed. Primer spacing, primer performance and amplicon length affected the sensitivity of the fusion calling.

**EXAMPLE 10 Gene Fusion Detection Method Analysis**

The experiments provided in this example illustrate details for successfully performing a method for detecting a fusion involving a target gene using a nested one-sided PCR tiling reaction followed by massively parallel, high throughput nucleic acid sequencing of the inner primer amplicons generated in the PCR tiling reaction. The experiments provided in this example illustrate how a skilled artisan can modify parameters of the nested one-sided PCR reaction in order to improve the sensitivity and/or specificity of the method. Reagents, instrumentation, and methods, unless otherwise specified, are as provided in Example 9 above.

A fusion template nucleic acid was used to test a nested one-sided tiling approach with three primer concentrations in the outer primer first PCR reaction (i.e. Star1) and three Starl cycle numbers, followed by three primer concentrations in the inner primer second PCR reaction (i.e. Star2) of the nested one-sided PCR step of the method, and three Star2 cycle numbers as well as three dilutions of Starl products input into the Star2 reaction. The samples were pooled and analyzed.

A mixture of TPM4:Alk1_7 template in wild type monosomal DNA was prepared from the 90% TPM4:Alk spike and 10% monosomonal DNA according using the primers and template provided in Example 9. The monosomal DNA was prepared from a wild type cell line (AG16778). The Starl reaction was performed with 148 assays (i.e.148 forward target-specific outer primers (see FIGS. 13A-13H for list of target-specific tiled outer ALK and ROS primers used for this experiment) and a reverse outer universal primer) for ROS 1 and ALK, with primer concentrations of 5nM, 10nM and 25nM, and three variation of cycle number, 15, 25 and 35 cycles. The Star2 reaction was performed with 148 assays (i.e. 148 forward target-specific inner primers, see FIGS. 13A-13H) for list of target-specific tiled inner ALK and ROS primers used for this experiment) and a reverse inner universal primer) for ROS1 and ALK, with three primer concentrations, 10nM, 20nM and 40nM, three different cycle numbers (15, 25, and 35 cycles), and three Starl dilutions 1:20, 1:200 and 1:2000. Except as indicated above, the Starl and Star2 reactions were performed in the K23 master mix and using the cycling conditions provided in Example 9. The 243 resulting samples that included the amplicon products of the Star2 reactions, were barcoded using the protocol in Example 9. A pool of all barcoded samples was prepared and sequenced as provided in Example 9. Data analysis and quality control was performed as provided in Example 9.

A list of all primer counts that mapped on target reads was supplied for each condition and primer. The uniformity was calculated using this data for the 90th and 10th percentile. A second separate analysis supplied information on total reads, % mapped reads and % on target reads for each condition tested. The data was paired and listed in Table 18. A total of 30 conditions were identified with Uniformities < 10. The original protocol resulted in a uniformity of 33 with 36.6% on target reads for the fusion pool tested in this experiment. Improved cycling condition selection shows that uniformity was improved to a value of ~5 (90th/10th percentile) with a percent on target of ~62%. Barcode 303 row in Table 18 is an especially noteworthy set of conditions tested with respect to good uniformity and % on target reads.

**Table 18**

| Barcode | Star1 Primer conc. nM | # cycles Star1 | Star1 dilution | Star2 Primer conc. nM | # cycles Star2 | Uniformity $90^{th}/10^{th}$ | % on target | 90th | 10th | Av DOR |
|---|---|---|---|---|---|---|---|---|---|---|
| 311 | 25 | 35 | 20 | 10 | 35 | 5.1 | 60.7 | 9,172 | 1,785 | 5,678 |
| 303 | 10 | 35 | 20 | 10 | 35 | 5.2 | 62.3 | 8,278 | 1,590 | 5,098 |
| 215 | 25 | 35 | 20 | 10 | 25 | 5.3 | 60.7 | 9,665 | 1,827 | 6,001 |
| 312 | 25 | 35 | 20 | 20 | 35 | 5.5 | 60.7 | 8,911 | 1,626 | 5,537 |
| 207 | 10 | 35 | 20 | 10 | 25 | 5.5 | 62.4 | 10,071 | 1,815 | 6,149 |
| 304 | 10 | 35 | 20 | 20 | 35 | 5.8 | 62.5 | 8,743 | 1,500 | 5,409 |
| 296 | 5 | 35 | 20 | 20 | 35 | 6.0 | 62.9 | 10,534 | 1,763 | 6,529 |
| 309 | 25 | 25 | 20 | 20 | 35 | 6.0 | 58.5 | 9,420 | 1,571 | 5,589 |
| 295 | 5 | 35 | 20 | 10 | 35 | 6.2 | 62.9 | 8,836 | 1,414 | 5,426 |
| 212 | 25 | 25 | 20 | 10 | 25 | 6.6 | 58.3 | 9,458 | 1,440 | 5,464 |
| 308 | 25 | 25 | 20 | 10 | 35 | 6.6 | 58.6 | 7,976 | 1,213 | 4,644 |
| 199 | 5 | 35 | 20 | 10 | 25 | 6.8 | 62.2 | 9,239 | 1,359 | 5,558 |
| 213 | 25 | 25 | 20 | 20 | 25 | 7.4 | 58.4 | 9,236 | 1,250 | 5,382 |
| 301 | 10 | 25 | 20 | 20 | 35 | 7.7 | 60.1 | 8,642 | 1,125 | 4,999 |
| 208 | 10 | 35 | 20 | 20 | 25 | 7.7 | 62.3 | 10,870 | 1,409 | 6,618 |
| 369 | 10 | 35 | 20 | 40 | 35 | 7.7 | 62.0 | 8,573 | 1,108 | 5,261 |
| 361 | 5 | 35 | 20 | 40 | 35 | 7.8 | 62.4 | 8,551 | 1,095 | 5,305 |
| 377 | 25 | 35 | 20 | 40 | 35 | 7.8 | 60.6 | 7,819 | 999 | 4,592 |
| 200 | 5 | 35 | 20 | 20 | 25 | 7.9 | 62.7 | 8,902 | 1,122 | 5,403 |
| 293 | 5 | 25 | 20 | 20 | 35 | 8.3 | 60.7 | 8,797 | 1,062 | 5,151 |
| 319 | 5 | 35 | 200 | 10 | 35 | 8.3 | 61.7 | 9,205 | 1,109 | 5,467 |
| 327 | 10 | 35 | 200 | 10 | 35 | 8.6 | 60.8 | 9,132 | 1,066 | 5,337 |
| 320 | 5 | 35 | 200 | 20 | 35 | 8.8 | 61.6 | 9,293 | 1,057 | 5,584 |
| 300 | 10 | 25 | 20 | 10 | 35 | 8.9 | 60.2 | 8,432 | 950 | 4,829 |
| 205 | 10 | 25 | 20 | 20 | 25 | 8.9 | 59.6 | 9,470 | 1,062 | 5,526 |

(continued)

| Barcode | Star1 Primer conc. nM | # cycles Star1 | Star1 dilution | Star2 Primer conc. nM | # cycles Star2 | Uniformity $90^{th}/10^{th}$ | % on target | 90th | 10th | Av DOR |
|---|---|---|---|---|---|---|---|---|---|---|
| 310 | 25 | 25 | 20 | 40 | 35 | 9.0 | 58.5 | 8,574 | 958 | 4,911 |
| 292 | 5 | 25 | 20 | 10 | 35 | 9.4 | 60.6 | 9,285 | 991 | 5,341 |
| 196 | 5 | 25 | 20 | 10 | 25 | 9.4 | 60.3 | 9,403 | 997 | 5,468 |
| 103 | 5 | 35 | 20 | 10 | 15 | 9.5 | 63.3 | 11,120 | 1,173 | 6,496 |
| 302 | 10 | 25 | 20 | 40 | 35 | 9.9 | 59.9 | 9,243 | 930 | 5,292 |

An improved exemplary protocol was identified, as follows:

Star1: 1x K23 (See Example 9), 10 nM outer pool, 2.5 μM RStar2_C3_Loop-Ryan, 4 μl plateau-ed library, 20 μl total volume.

PCR program: 95C 10 min; 35x [95C 30 sec, 63C 10 min, 72C 2 min]; 72C 7 min, 4C hold

Star2: 1x K23, 10nM inner pool, 1 μM RStar2, 2 μl Starl product diluted 1:20, 10 μl total volume

PCR program: 95C 10 min; 35x [95C 30 sec, 63C 10 min, 72C 2 min]; 72C 7 min, 4C hold

BC-reaction: 1x Qiagen MM, 0.5 μM R-BC-barcode, 0.5 μM F-BC-barcode, 1 μl Star2 product diluted 1:20, 10 μl total volume

BC-PCR program: 95C 10 min; 12x [95C, 30 sec, 62.5C 3 min, 72C 2 min]; 72C 7 min, 4C hold.

The protocol was improved in terms of uniformity and percent on target reads. Uniformity was reduced to 5 from the original 33 (90th/10th percentile) and a doubling of all on target reads was achieved from 36% to 63%. Thus, not only was the method for detecting fusions using a nested one-sided multiplex PCR reaction once again successfully demonstrated, methods for identifying improved PCR conditions were exemplified.

**EXAMPLE 11. Further Analysis of Tiling PCR Primer Locations for Detection of Fusions**

This example provides another proof of concept of the detection of gene fusions of cancer genes using a one-sided nested PCR tiling approach as well as two-sided across-the-breakpoint protocols.

The strategy that was tested in this set of experiments for performing a method of the present invention for fusion detection in blood or a fraction thereof, relied on multiplex PCR using tiled primer binding sites with primers whose amplicons were in a target region where a cancer-related target gene fusion is known to occur, followed by sequencing and bioinformatics analysis. The bioinformatics analysis identified fusions as sequence reads that mapped to two genes (the target gene and the fusion partner).

The one-sided nested tiling approach for fusion detection was tested using synthetic spikes as targets that mimicked circulating tumor DNA from fused genes. Four gene fusion pairs commonly found in lung cancer were selected for this experiment. For each fusion pair, 9 different spikes fragments were created with the same breakpoint but different proportion of target and partner genes. (see FIG. 14 and Table 19).

**Table 19**

| | **Fusion Pair 1** | **Fusion Pair 2** | **Fusion Pair 3** | **Fusion Pair 4** |
|---|---|---|---|---|
| **Spike 1** | CD74:ROS1_1 | NPM1:ALK_1 | NPM1:ALK_1 | TPM4:ALK_1 |
| **Spike 2** | CD74:ROS1_3 | NPM1:ALK_3 | NPM1:ALK_3 | TPM4:ALK_3 |
| **Spike 3** | CD74:ROS1_5 | NPM1:ALK_5 | NPM1:ALK_5 | TPM4:ALK_5 |
| **Spike 4** | CD74:ROS1_7 | NPM1:ALK_7 | NPM1:ALK_7 | TPM4:ALK_7 |

(continued)

| | Fusion Pair 1 | Fusion Pair 2 | Fusion Pair 3 | Fusion Pair 4 |
|---|---|---|---|---|
| **Spike 5** | CD74:ROS1_9 | NPM1:ALK_9 | NPM1:ALK_9 | TPM4:ALK_9 |
| **Spike 6** | CD74:R0S1_11 | NPM1:ALK_11 | NPM1:ALK_11 | TPM4:ALK_11 |
| **Spike 7** | CD74:ROS1_13 | NPM1:ALK_13 | NPM1:ALK_13 | TPM4:ALK_13 |
| **Spike 8** | CD74:ROS1_15 | NPM1:ALK_15 | NPM1:ALK_15 | TPM4:ALK_15 |
| **Spike 9** | CD74:ROS1_17 | NPM1:ALK_17 | NPM1:ALK_17 | TPM4:ALK_17 |

The fusion spikes and library preparation was performed according to Example 9. The input DNA was at 0% (10,000 copies of WT-DNA), 90% (10,000 copies of WT + 90,000 copies of spike), or spikes only (30ng of all 9 spikes).

A one-sided nested multiplex PCR amplification reaction was performed using the STAR1/STAR2 protocols as provided in Example 9 and a two-sided across-the-breakpoint protocol called the OneStar protocol provided herein. For the OneStar protocol a mixture of the Fusion 1 One-Star primer pool, 50 nM in 1x K23 reaction mixture, 4 μl StarI product diluted 1:20, 10 ul total volume was amplified using the following PCR program: 95C 10 min; 30x [95C 30 sec, 63C 10 min, 72C 30 sec]; 72C 2min, 4C hold.

The samples were barcoded using the barcoding protocol provided in Example 9 with the exceptions of using diluted STAR2/OneStar product instead of a Star 2 product. The samples were pooled into one pool, purified using Qubit, and sequenced with paired-end, single-index, 100 cycles reads. The 9 templates analyzed per fusion, with two barcodes per template, provided 18 barcode reads per analysis, e.g Tables 20 and 21. Data was analyzed as provided in Example 8.

**Detection of Fusion**

Two different approaches were used to detect fusions, called Star1/Star2 (One-Sided nested multiplex PCR) and OneStar (two sided nested tiling PCR). FIG. 15 Illustrates each method. It is noteworthy that the One-Sided nested multiplex PCR tiling approach (Star1-Star2 approach) does not require nearly as many primers since one side of the PCR reactions is a universal primer, and does not require prior knowledge of both fusion partners.

The sequencing data showed good coverage for the ALK and ROS primers used in this experiment, the majority with at least 1,000 reads. There were 2 assay dropouts out of 67 for ALK primers and 1 assay dropout out of 27 for ROS primers. Analysis of sequencing data indicated that fusions were successfully detected using both Star1/Star2 and OneStar approach (data not shown).

Analysis of sequencing data indicated that the percentage of on target reads was approximately 35% for the Star1/Star2 protocol and approximately 10% for OneStar protocol. However only about 1% of the on target reads for Star1/Star2 have fusions, whereas all reads in the OneStar protocol have fusions of ROS1:CD74.

The role of a target binding site location relative to a fusion breakpoint in detection of gene fusion using the one-sided nested PCR approach using a tiled series of inner and outer target primer binding sites, was analyzed. For the ROS1:CD74 fusion template amplified spike samples, three inner ROS1 primers for a one-sided PCR approach were designed to bind 506, 396, and 91 nucleotides from the breakpoint. Only PCR with the primer that bound a target primer binding site 91 nucleotides from the breakpoint yielded detectable fusion reads and only for the duplicate fusion spike nucleic acid library molecules that included the binding site 91 nucleotides from the break point (See samples 11-18 of Table 20; Samples 1-10 did not include the binding site 91 nucleotides from the break point). The highest fusion percent detected was slightly above 60%, which probably could have been higher but the binding site seems further from the breakpoint than ideal under these conditions with an average read length of about 80 base pairs.

**TABLE. 20 ROS1:CD74 fusion read, total read, and average read length data.**

| | FusionReads/TotalReads | TotalReads | AverageReadLength |
|---|---|---|---|
| **1** | 2% | 10550 | 63 |
| **2** | 3% | 8549 | 64 |
| **3** | 2% | 9064 | 63 |
| **4** | 3% | 11056 | 65 |
| **5** | 3% | 8260 | 64 |
| **6** | 4% | 9539 | 63 |

(continued)

| | FusionReads/TotalReads | TotalReads | AverageReadLength |
|---|---|---|---|
| **7** | 3% | 12787 | 63 |
| **8** | 4% | 9451 | 65 |
| **9** | 2% | 10455 | 62 |
| **10** | 2% | 8957 | 59 |
| **11** | 10% | 20185 | 68 |
| **12** | 12% | 19557 | 70 |
| **13** | 12% | 35972 | 71 |
| **14** | 13% | 24841 | 68 |
| **15** | 25% | 34637 | 75 |
| **16** | 28% | 38762 | 78 |
| **17** | 63% | 91163 | 86 |
| **18** | 61% | 90910 | 85 |

For the ALK:TPM4 fusion spike library, FIG. 16 shows the location of 4 of the forward inner primers tested, as well as their respective amplicons with respect to a breakpoint. FIG. 17 shows the relative location of inner primers 2, 3, and 4 with respect to the template fusion spike molecules, for these one-sided PCR amplifications. The last seven templates should be amplified and detected for P3 and the last three templates should be amplified for P4. P2 provided weaker results. Although not to be limited by theory, primer P2 at 22 nucleotides from the breakpoint appears to be at a distance that is too close to the breakpoint to be ideal. Data from the one-sided PCR amplifications is shown in Table 21. Primer 3 at 36 nucleotides from the breakpoint appeared to be at a particularly effective distance given these PCR conditions which yielded average amplicons of around 32 nucleotides in length, with over 85% fusion reads for some of the spike templates (Table 21). With respect to the P4 primer, which was 107 nucleotides from a breakpoint, the average read length for this primer was 50 bp. Therefore, fusion reads were not detected using the P4 primer. Under these conditions, where 49 bp read lengths were generated, 107 nucleotides were too far to detect the breakpoint/fusion.

**TABLE 21 ALK:TPM4 fusion read/total read, and total reads.**

| | FusionReads/TotalReads | TotalReads |
|---|---|---|
| 1 | 4% | 1233 |
| 2 | 5% | 1459 |
| 3 | 6% | 1310 |
| 4 | 6% | 1628 |
| 5 | 65% | 5381 |
| 6 | 65% | 6525 |
| 7 | 62% | 7096 |
| 8 | 56% | 6786 |
| 9 | 55% | 5058 |
| 10 | 49% | 5032 |
| 11 | 70% | 6038 |
| 12 | 70% | 6514 |
| 13 | 79% | 8615 |
| 14 | 77% | 8443 |
| 15 | 88% | 20363 |
| 16 | 88% | 19509 |
| 17 | 87% | 28463 |
| 18 | 87% | 33114 |

In another sample, an ALK:NPM1 fusion spike template library was analyzed with three inner primers (P1, P2, and P3) (for one-sided PCR), 21, 36, and 58 nucleotides from the breakpoint, with average amplicon length of 37, 41 and 38, respectively. In this experiment and under these conditions, P1 did not get amplified, as it appeared to be too close to the breakpoint. P3 was amplified but only provided 1% fusion detection. P2 (36 nucleotides from the breakpoint with an average amplicon length of 41 nucleotides) provided the highest detection of the fusion with some templates yielding over 70% fusion reads/total reads.

**EXAMPLE 12 One-Sided PCR Tiling Amplicon Length Vs. Annealing Time**

Reagents, instrumentation, and methods, unless otherwise specified, are defined in Example 9 above.

A one-sided PCR tiling method for the detection of gene fusions according to the present invention was tested to determine the effect of annealing/extension time on the yield and size of the longest amplicon product formed.

Templates were produced as follows: Templates, (T1 = 232bp (long control), T2 = 173 bp, T3 = 154 bp, T4 = 121 bp, T5 = 117 bp), were constructed by amplifying a 284 bp template (SEQ ID NO: 311) that included a portion of the human TP53 exon 4 in two consecutive singleplex reactions (i.e. amplicons from the first singleplex reaction were used as templates for the second reaction) using appropriate target specific primers as shown diagrammatically in FIG 18A. The templates were purified using Ampure 1.5X beads (Beckman Coulter) according to the standard manufacturer's protocol and diluted 1:5. The concentration of template DNA was determined using a BioAnalyzer 1K.

Five of the templates were used to analyze different time lengths for an annealing/extension step of a PCR reaction and to analyze a 1-stage versus a 2-stage PCR reaction to identify conditions that produce the largest amplicons in a reaction where primers bind to tiled primer binding sites. The reactions used the above templates (around 150 nucleotides in length) (FIG. 18A), to approximate circulating tumor DNA fragments. The PCR amplification mixture for the on-test conditions contained K23 buffer (see Example 9), AmpliTaq Gold 360 (Life Technologies, Carlsbad, CA) 30 units/200ul reaction mixture, 50nM of each target-specific primer, and 0.5 ng template. The primers were a series of 8 tagged primers that were complementary to a tiled series of primer binding sites on the initial 284 bp template (FIG. 18A). The 8 forward primers that bound the tiled series of primer binding sites were designed to generate amplicons of varying lengths to the 3' end of the appropriate template as follows: 8F8 (232bp), 8F3 (200bp), 8F9 (173bp), 8F10 (154bp), 5F1 (127bp), 5F8 (94bp), 5F9 (72bp), 5F3 (51bp). The reverse primers used to generate the amplicon sizes in parenthesis are indicated in FIG. 18A (e.g 5R3, 5R4, or 5R5). The PCR amplification protocols tested were a 1-stage and a 2-stage protocol as shown in Table 23. The sequence data of the forward and reverse primers are shown in Table 22. The percentage of a sample that is the longest available product was calculated by taking [nM conc. of long product]/sum ([nM conc of all products]).

**Table 22**

| Name | Sequence Name | Seq start | Seq end | Sequence | SEQ ID | bp |
|---|---|---|---|---|---|---|
| 8F8 | P_8_wt_ 8F8+tag | 7578222 | 7578246 | ACACGACGCTCTTCCGATtctgtcatccaaatact ccacacgc | 312 | 43 |
| 8F3 | P-D2_8_wt_ FW3+tag | 7578254 | 7578273 | ACACGACGCTCTTCCGATCTCTTCCACT CGGATAAGATGC | 313 | 40 |
| 8F9 | P_8_wt_ 8F9_tag | 7578281 | 7578298 | ACACGACGCTCTTCCGATgggccagacctaag agca | 314 | 36 |
| 8F10 | P_8_wt_ 8F10_tag | 7578300 | 7578321 | ACACGACGCTCTTCCGATtcagtgaggaatcag aggcctg | 315 | 40 |
| 5F1 | P-D2_5_wt_ FW1+tag | 7578327 | 7578345 | ACACGACGCTCTTCCGATCTCCTGGGC AACCAGCCCTGT | 316 | 39 |

(continued)

| Name | Sequence Name | Seq start | Seq end | Sequence | SEQ ID | bp |
|---|---|---|---|---|---|---|
| 5F8 | P_5_wt_ 5F8+tagC | 7578360 | 7578379 | ACACGACGCTCTTCCGATcagctgctcaccatc gctat | 317 | 38 |
| 5F9 | P_5_wt_ 5F9+tagC | 7578382 | 7578398 | ACACGACGCTCTTCCGATgagcagcgctcatg gtg | 318 | 35 |
| 5F3 | P-D2_5_wt_ FW3+tag | 7578403 | 7578421 | ACACGACGCTCTTCCGATCTCAGCGCC TCACAACCTCCG | 319 | 39 |
| 5R3 | P_5_wt_ 5R3+tagC | 7578453 | 7578430 | AGACGTGTGCTCTTCCGATCTcatggccatct acaagcagtcaca | 320 | 45 |
| 5R4 | P_5_wt_ 5R4+tagC | 7578397 | 7578376 | AGACGTGTGCTCTTCCGATCTaccatgagcg ctgctcagatag | 321 | 43 |
| 5R5 | P_5_wt_ 5R5+tagC | 7578372 | 7578355 | AGACGTGTGCTCTTCCGATCTgatggtgagc agctgggg | 322 | 39 |
| R-SQ | R-SQ | | | GTGACTGGAGTTCAGACGTGTGCTCTT CCGATC*T | 323 | 35 |

Table 23

| Stage & Cycle no. | Temp. °C | Time | Stage & Cycle no. | Temp. °C | Time |
|---|---|---|---|---|---|
| 1-Stage | 95 | 10 min | 2-Stage | 95 | 10 min |
| 10x | 95 | 20 sec | 3x | 95 | 20 sec |
| | 60 | 30/60/90 min | | 60 | 30/60/90 min |
| | 4 | Hold | 7x | 95 | 20 sec |
| | | | | 70 | 30/60/90 min |
| | | | | 4 | hold |

The percentages and absolute yields of amplicons obtained using the 1 stage and 2 stage annealing cycles with the 8F10+5R3 (154bp insert) template are listed in Table 24 and 25 respectively. The primer mix included the 8F10 (154bp), 5F1 (127bp), 5F8 (94bp), 5F9 (72bp), and 5F3 (51bp) forward primers and the 5R3 reverse primer. The 1 stage and 2 stage annealing cycles spectra of tagged primer fluorescence vs amplicon length are shown in FIGS. 18B-18C. The percent yield of the long amplicon, the 154 bp amplicon in this multiplex PCR reaction with tiled primer binding sites, increased with longer annealing time from 13% to 72 % using the 1 stage protocol and from 63% to 80% using the 2 stage protocol (see Tables 24 and 25). The selectivity for the long amplicon improved with the long annealing time of 90 min and with the 2 stage protocol as evident by a decrease in the percent yield of the short, 51bp primer amplified, amplicon from 70% to 20%.

**Table 24: 1 Stage Annealing**

| 1-stage | Annealing Time | 51bp | 72bp | 94bp | 127bp | 154bp |
|---|---|---|---|---|---|---|
| Relative Yield | 30m | 70% | | | | 13% |
| | 60m | 33% | | | | 67% |
| | 90m | 22% | | 6% | | 72% |
| | Annealing Time | 51bp (nM) | 72bp (nM) | 94bp (nM) | 127bp (nM) | 154bp (nM) |
| Absolute Yield | 30m | 14 | 0 | 0 | 0 | 3 |
| | 60m | 7 | 0 | 0 | 0 | 13 |
| | 90m | 6 | 0 | 2 | 0 | 20 |

**Table 25: 2 Stage Annealing**

| 2-stage | Annealing Time | 51bp | 72bp | 94bp | 127bp | 154bp |
|---|---|---|---|---|---|---|
| Relative | 30m | 37% | | | | 63% |
| Yield | 60m | 22% | | | | 78% |
| | 90m | 20% | | 6% | | 80% |
| | Annealing Time | 51bp (nM) | 72bp (nM) | 94bp (nM) | 127bp (nM) | 154bp (nM) |
| Absolute Yield | 30m | 8 | 0 | 0 | 0 | 13 |
| | 60m | 4 | 0 | 0 | 0 | 14 |
| | 90m | 4 | 0 | 0 | 0 | 15 |

The 2 stage protocol favored the longer amplicon over the 1 stage protocol as evident by the percent yield of the long amplicon using other starting templates (232 bp template, 121 bp template, and 117 bp template), of varying lengths (see FIGS. 19A- 19D). An increase in annealing/extension time increased the percent yield of the longer product of the 232 bp template and 121 bp templates while the 117 bp template remained consistent around 70%. The long 232 bp template amplification selectivity for the 51 bp amplicon was decreased by the longer 90 min annealing/extension time. The K23 Master Mix, ionic strength 300 nM, exhibited greater selectivity for the longer amplicon and produced fewer side products than the "Gold Master Mix," which had a lower ionic strength, 65nM, Taq Gold 0.3 U/μl, 2X Gold Buffer (Life Technologies, Carlsbad, CA, 3mM $MgCl_2$, and 0.4 mM dNTPs (see e.g. FIGS. 19B-19C).

Those skilled in the art can devise many modifications and other embodiments within the scope and spirit of the presently disclosed inventions. Indeed, variations in the materials, methods, drawings, experiments examples and embodiments described may be made by skilled artisans without changing the fundamental aspects of the disclosed inventions. Any of the disclosed embodiments can be used in combination with any other disclosed embodiment.

The disclosed embodiments, examples and experiments are not intended to limit the scope of the disclosure nor to represent that the experiments below are all or the only experiments performed. Efforts have been made to ensure accuracy with respect to numbers used (*e.g.,* amounts, temperature, *etc.*) but some experimental errors and deviations should be accounted for. It should be understood that variations in the methods as described may be made without changing the fundamental aspects that the experiments are meant to illustrate.

The disclosure is further explained by reference to the following numbered clauses:

1. A method for detecting a mutation in a target gene in a sample or a fraction thereof from a mammal, the method comprising:

    a) forming an initial reaction mixture by combining a polymerase, deoxynucleoside triphosphates, nucleic acid fragments from a nucleic acid library generated from the sample, a series of plus strand forward target-specific primers and a plus strand reverse universal primer, wherein the nucleic acid fragments comprise a reverse universal primer binding site, wherein the series of forward target-specific primers comprises 5 to 250 primers that bind to a tiled series of target-specific primer binding sites spaced apart on the target gene by between 10 and 100 nucleotides;

b) subjecting the initial reaction mixture to initial amplification conditions to generate target amplicons generated using primer pairs comprising one of the primers of the series of forward target-specific primers and the reverse universal primer; and
c) analyzing a nucleic acid sequence of at least a portion of the target amplicons, thereby detecting the mutation in the target gene.

2. The method of clause 1, wherein the analyzing comprises determining the nucleic acid sequence of at least a portion of the target amplicons using massively parallel sequencing.
3. The method of clause 1, wherein the plus strand forward target-specific primers are plus strand forward target-specific outer primers, and the plus strand reverse universal primer, is a plus strand reverse universal outer primer, and wherein the method further comprises before the analyzing:

a) forming an inner primer reaction mixture by combining an outer primer target amplicons, a polymerase, deoxynucleoside triphosphates, a reverse inner universal primer and a series of forward target-specific inner primers comprising 5 to 250 primers that bind to a tiled series of target-specific inner primer binding sites spaced apart on the target gene by between 10 and 100 nucleotides and each found on at least one outer primer target amplicon, configured to prime an extension reaction in the same direction as the series of target-specific outer primers; and
b) subjecting the inner primer reaction mixture to inner primer amplification conditions to generate inner primer target amplicons generated using primer pairs comprising one of the forward target-specific inner primers and the reverse inner universal primer, wherein the amplicons whose nucleic acid sequences are analyzed comprise the inner primer target amplicons, wherein the analyzed nucleic acid sequences are a portion of the outer primer target amplicons.

4. The method of clause 3, wherein the target-specific inner primer binding sites overlap the target-specific outer primer binding sites by between 0 and 25 nucleotides.
5. The method of clause 3, wherein the reverse inner universal primer comprises the same nucleotide sequence as the reverse outer universal primer.
6. The method of clause 3, wherein the tiled series of target-specific outer primer binding sites and the target-specific inner primer binding sites are found on a target region of each of 1 to 100 target genes.
7. The method of clause 6, wherein at least 50% or at least 75% of the outer primer target amplicons have overlapping sequences with at least one other of the outer primer target amplicon on each of 1 to 100 target genes, wherein each target region comprises between 500 and 10,000 nucleotides and wherein the target region comprises known mutations associated with a disease.
8. The method of clause 3, wherein at least 50% of the outer primer target amplicons and at least one of the inner primer target amplicons have overlapping sequences.
9. The method of clause 7, further comprising:

a) forming a minus strand outer primer reaction mixture by combining a polymerase, deoxynucleoside triphosphates, nucleic acid fragments from the nucleic acid library generated from the sample, a series of minus strand forward target-specific outer primers and minus strand reverse outer universal primer, wherein the nucleic acid fragments comprise a minus strand reverse outer universal primer binding site, wherein the series of minus strand forward target-specific outer primers comprises 5 to 250 primers that bind to a tiled series of minus strand forward target-specific outer primer binding sites spaced apart on the target gene by between 10 and 100 nucleotides, wherein the minus strand forward target-specific outer primer binding sites are located on the minus strand of the strand targeted by the target-specific outer primers;
b) subjecting the minus strand outer primer reaction mixture to amplification conditions to generate minus strand outer primer target amplicons generated using primer pairs comprising one of the primers of the series of minus strand, forward target-specific outer primers and the minus strand, reverse outer universal primer; and
c) analyzing the nucleic acid sequence of at least a portion of the minus strand, outer primer target amplicons, thereby detecting a mutation in the target gene.

10. The method of clause 9, wherein the method further comprises before the analyzing:

a) forming a minus strand, inner primer amplification reaction mixture by combining the minus strand, outer primer target amplicons, a polymerase, deoxynucleoside triphosphates, a minus strand, reverse inner universal primer and a series of forward minus strand, target-specific inner primers comprising 5 to 250 primers that bind to a tiled series of minus strand, target-specific inner primer binding sites spaced apart on the target gene by between 10 and 100 nucleotides and each found on at least one minus strand, outer primer target amplicon, configured to prime an extension reaction in the same direction as the series of minus strand, target-specific outer primers; and

b) subjecting the minus strand reaction mixture to minus strand, target-specific inner primer amplification conditions to form minus strand, inner primer target amplicons generated using primer pairs comprising one of the minus strand, forward target-specific inner primers and the minus strand, inner universal primer, wherein the amplicons whose nucleic acid sequences are analyzed comprise the minus strand, inner primer target amplicons.

11. The method of clause 9, wherein the minus strand, outer primer amplification conditions are identical to the outer primer amplification conditions.

12. The method of clause 10, wherein the minus strand, inner primer amplification conditions are identical to the inner primer amplification conditions.

13. The method according to any one of clauses 7-10, wherein the disease is cancer.

14. The method of clause 1 or 3, wherein the presence of at least 25 contiguous nucleotides from the target gene and at least 25 contiguous nucleotides from a region of a genome of the mammal not found on the target gene, on the outer primer target amplicon and/or the inner primer target amplicon is indicative of a gene fusion comprising the target gene.

15. The method of clause 1 or 3, wherein the series of plus strand target-specific outer primers comprises at least one primer that binds to a target primer binding site that is between 25 and 150 nucleotides from a known fusion breakpoint for the target gene, and wherein the outer primer target amplicons comprise amplicons that are at least 150 nucleotides long.

16. The method of clause 14, wherein the method detects a gene fusion from at least one fusion partner gene selected from the group consisting of AKT1, ALK, BRAF, EGFR, HER2, KRAS, MEK1, MET, NRAS, PIK3CA, RET, and ROS1.

17. The method of clause 16, wherein the gene fusion comprises a chromosomal translocation.

18. The method of clause 14, wherein the method detects the gene fusion from at least two fusion partner genes selected from the group consisting of AKT1, ALK, BRAF, EGFR, HER2, KRAS, MEK1, MET, NRAS, PIK3CA, RET, and ROS1, and wherein the series of target-specific outer primers comprises at least one primer that binds to a target primer binding site that is between 25 and 150 nucleotides from a known fusion breakpoint for each of the target genes, and wherein the outer primer target amplicons comprise amplicons that are at least 150 nucleotides long.

19. The method of clause 3, wherein the series of forward target-specific outer primers and the series of forward target-specific inner primers each comprise at least one primer that binds to a target primer binding site that is a target distance from a known fusion breakpoint for the target gene, and wherein the outer primer target amplicons comprise at least one amplicon that is as long as the target distance.

20. The method of clause 19, wherein the target gene is selected from AKT1, ALK, BRAF, EGFR, HER2, KRAS, MEK1, MET, NRAS, PIK3CA, RET, and ROS1.

21. The method of clause 19, wherein the target gene comprises at least two fusion partner genes selected from the group consisting of AKT1, ALK, BRAF, EGFR, HER2, KRAS, MEK1, MET, NRAS, PIK3CA, RET, and ROS1, and wherein the series of forward target-specific outer primers and the series of forward target-specific inner primers each comprise between 5 and 250 primers and each binds to at least one target region on one of the at least two fusion partner genes, and wherein at least one primer binds to a target primer binding site that is a target distance from a known fusion breakpoint for each of the at least two fusion partner genes, and wherein the outer primer target amplicons for each of the at least two fusion partner genes comprise at least one amplicon that is as long as the target distance.

22. The method of anyone of clauses 19-21, wherein the series of forward target-specific outer primers and the series of forward target-specific inner primers each comprise at least one primer that binds to a target primer binding site that is between 25 and 150 nucleotides or between 25 and 100 nucleotides or between 25 and 50 nucleotides from a known fusion breakpoint for each of the target genes, and wherein the outer primer target amplicons comprise amplicons that are at least 150 nucleotides long that span a known genetic fusion breakpoint.

23. The method of clause 22, wherein the primer amplification conditions comprise at least 5 PCR cycles having a target-specific outer primer annealing step of between 30 and 120 minutes at between 58C and 72C.

24. The method of clause 22, wherein the primer amplification conditions comprise a first set of between 2 and 10 PCR cycles with an outer primer annealing step of between 30 and 120 minutes at between 58C and 65C and a second set of between 5 and 50 PCR cycles with a target-specific outer primer annealing step of between 30 and 120 minutes at between 68C and 72C.

25. The method of clause 22, wherein the series of forward target-specific outer primers and the series of forward target-specific inner primers highest Tm is 2 to 10 degrees below the annealing temperature.

26. The method of clause 24, wherein the annealing is performed in a combined annealing/extension step.

27. The method of clause 1, wherein the amplification conditions comprise at least 5 PCR cycles having an annealing step of between 30 and 120 minutes at between 58C and 72C.

28. The method of clause 27, wherein the annealing step is between 60 and 90 minutes long.

29. The method of clause 27 or 28, wherein the highest Tm of at least 50% of the target-specific primers is between 1 and 10 degrees C below the annealing temperature used for PCR reaction.

30. The method of clause 27 or 28, wherein the highest Tm of the set of target-specific primers is 2 to 10 degrees below the annealing temperature and wherein the annealing is performed in a combined annealing/extension step.

31. The method of clause 27, wherein the series of target-specific primers comprises at least one primer that binds to a target primer binding site that is between 25 and 150 nucleotides from a known fusion breakpoint for the target gene.

32. A method for amplifying a target nucleic acid region *in vitro,* the method comprising:

a. forming a reaction mixture by combining a polymerase, deoxynucleoside triphosphates, nucleic acid fragments from a library, a first pool of a plurality of target-specific primers and a first reverse universal primer, wherein the nucleic acid fragments of the library comprise a universal reverse primer binding site, and wherein the plurality of target-specific primers comprises 5 to 250 primers that are capable of binding to a tiled series of primer binding sites that are spaced apart on the target region of the target gene by between 10 and 50 nucleotides; and

b. subjecting the reaction mixture to amplification conditions to form amplicons of 100 to 200 nucleotides in length, wherein the amplification conditions comprise an annealing step of between 30 and 120 minutes at between 58C and 72C, thereby amplifying the target nucleic acid region.

33. The method of clause 1 or 32, wherein the target-specific primer amplification conditions comprise at least 5 PCR cycles having a target-specific outer primer annealing step of between 60 and 90 minutes at between 58C and 72C.

34. The method of clause 1 or 32, wherein the target-specific primer amplification conditions comprise a first set of between 2 and 10 PCR cycles with a target-specific outer primer annealing step of between 30 and 120 minutes at between 58C and 65C and a second set of between 5 and 50 PCR cycles with a target-specific outer primer annealing step of between 30 and 120 minutes at between 68C and 72C.

35. The method according to clause 33 or 34, wherein the highest Tm of 50% of the target-specific outer primers is between 1 and 10 degrees C below the annealing temperature used for the PCR reaction.

36. The method according to clause 33 or 34, wherein the highest Tm of the set of target-specific primers is 2 to 10 degrees below the annealing temperature.

37. The method of cause 36, wherein the annealing is performed in a combined annealing/extension step.

38. A method for detecting a fusion involving a target gene in a sample or a fraction thereof from a mammal, the method comprising:

a. subjecting nucleic acids in the sample to a one-sided PCR tiling reaction across a target region of the target gene to generate outer primer target amplicons, wherein the tiling reaction is performed using a reverse outer universal primer and 5 to 250 forward outer target-specific primers that bind to a tiled series of outer target primer binding sites spaced apart on the target region of the target gene by between 10 and 100 nucleotides; and

b. analyzing the nucleic acid sequence of at least a portion of the target amplicons, thereby detecting a mutation in the target gene.

39. A method according to clause 38, further comprising performing a second one-sided PCR tiling reaction by amplifying the outer primer target amplicons using a reverse inner universal primer and a series of forward target-specific inner primers comprising 5 to 250 primers that bind to a tiled series of target inner primer binding sites spaced apart on the target region of the target gene by between 10 and 100 nucleotides and each found on at least one outer primer target amplicon, to generate forward inner primer target amplicons, wherein the forward target-specific inner primers are configured to prime an extension reaction in the same direction as the series of target-specific outer primers, and wherein the target amplicons whose nucleic acid sequences are analyzed comprise the forward inner primer target amplicons.

40. The method of clause 39, wherein the target-specific inner primer binding sites overlap the target-specific outer primer binding sites by between 5 and 20 nucleotides.

41. The method of clause 39, wherein the target region comprises a region of the target gene known to be involved in gene fusions.

42. The method of clause 1 or 38, wherein the tiled series of target-specific outer primer binding sites are spaced apart on the target region by between 10 and 75 nucleotides or between 15 and 50 nucleotides.

43. The method of clause 39, wherein the tiled series of target-specific outer primer binding sites and the target-specific inner primer binding sites is selected on a target region of each of 2 to 50 target genes.

**Claims**

1. A method for detecting a mutation in a target gene in a sample comprising circulating tumour DNA from a mammal, the method comprising:

    a) forming an initial reaction mixture by combining a polymerase, deoxynucleoside triphosphates, nucleic acid fragments from a nucleic acid library generated from the sample, a series of forward target-specific primers and a reverse universal primer, wherein the nucleic acid fragments comprise a reverse universal primer binding site, wherein the series of forward target-specific primers comprises 5 to 250 primers that bind to a tiled series of target-specific primer binding sites spaced apart on the target gene by between 10 and 100 nucleotides;
    b) subjecting the initial reaction mixture to initial amplification conditions to generate target amplicons generated using primer pairs comprising one of the primers of the series of forward target-specific primers and the reverse universal primer; and
    c) analyzing a nucleic acid sequence of at least a portion of the target amplicons, thereby detecting the mutation in the target gene.

2. The method of claim 1, wherein the analyzing comprises determining the nucleic acid sequence of at least a portion of the target amplicons using massively parallel sequencing.

3. The method of claim 1, wherein the forward target-specific primers are forward target-specific outer primers, and the reverse universal primer, is a reverse universal outer primer, and wherein the method further comprises before the analyzing:

    a) forming an inner primer reaction mixture by combining an outer primer target amplicons, a polymerase, deoxynucleoside triphosphates, a reverse inner universal primer and a series of forward target-specific inner primers comprising 5 to 250 primers that bind to a tiled series of target-specific inner primer binding sites spaced apart on the target gene by between 10 and 100 nucleotides and each found on at least one outer primer target amplicon, configured to prime an extension reaction in the same direction as the series of target-specific outer primers; and
    b) subjecting the inner primer reaction mixture to inner primer amplification conditions to generate inner primer target amplicons generated using primer pairs comprising one of the forward target-specific inner primers and the reverse inner universal primer, wherein the amplicons whose nucleic acid sequences are analyzed comprise the inner primer target amplicons, wherein the analyzed nucleic acid sequences are a portion of the outer primer target amplicons.

4. The method of claim 3, wherein the reverse inner universal primer comprises the same nucleotide sequence as the reverse outer universal primer.

5. The method of claim 3, wherein the tiled series of target-specific outer primer binding sites and the target-specific inner primer binding sites are found on a target region of each of 1 to 100 target genes.

6. The method of claim 5, wherein at least 50% or at least 75% of the outer primer target amplicons have overlapping sequences with at least one other of the outer primer target amplicon on each of 1 to 100 target genes, wherein each target region comprises between 500 and 10,000 nucleotides and wherein the target region comprises known mutations associated with a disease.

7. The method of claim 3, wherein at least 50% of the outer primer target amplicons and at least one of the inner primer target amplicons have overlapping sequences.

8. The method of claim 6, further comprising:

    a) forming a minus strand outer primer reaction mixture by combining a polymerase, deoxynucleoside triphosphates, nucleic acid fragments from the nucleic acid library generated from the sample, a series of minus strand forward target-specific outer primers and minus strand reverse outer universal primer, wherein the nucleic acid fragments comprise a minus strand reverse outer universal primer binding site, wherein the series of minus strand forward target-specific outer primers comprises 5 to 250 primers that bind to a tiled series of minus strand forward target-specific outer primer binding sites spaced apart on the target gene by between 10 and 100 nucleotides, wherein the minus strand forward target-specific outer primer binding sites are located on the minus strand of the strand targeted by the target-specific outer primers;
b) subjecting the minus strand outer primer reaction mixture to amplification conditions to generate minus strand outer primer target amplicons generated using primer pairs comprising one of the primers of the series of minus strand, forward target-specific outer primers and the minus strand, reverse outer universal primer; and
c) analyzing the nucleic acid sequence of at least a portion of the minus strand, outer primer target amplicons, thereby detecting a mutation in the target gene.

**9.** The method of claim 8, wherein the method further comprises before the analyzing:

a) forming a minus strand, inner primer amplification reaction mixture by combining the minus strand, outer primer target amplicons, a polymerase, deoxynucleoside triphosphates, a minus strand, reverse inner universal primer and a series of forward minus strand, target-specific inner primers comprising 5 to 250 primers that bind to a tiled series of minus strand, target-specific inner primer binding sites spaced apart on the target gene by between 10 and 100 nucleotides and each found on at least one minus strand, outer primer target amplicon, configured to prime an extension reaction in the same direction as the series of minus strand, target-specific outer primers; and
b) subjecting the minus strand reaction mixture to minus strand, target-specific inner primer amplification conditions to form minus strand, inner primer target amplicons generated using primer pairs comprising one of the minus strand, forward target-specific inner primers and the minus strand, inner universal primer, wherein the amplicons whose nucleic acid sequences are analyzed comprise the minus strand, inner primer target amplicons.

**10.** The method of claim 8, wherein the minus strand, outer primer amplification conditions are identical to the outer primer amplification conditions or wherein the minus strand, inner primer amplification conditions are identical to the inner primer amplification conditions.

**11.** The method according to any one of claims 8-9, wherein the disease is cancer.

**12.** The method of claim 1 or 3, wherein the presence of at least 25 contiguous nucleotides from the target gene and at least 25 contiguous nucleotides from a region of a genome of the mammal not found on the target gene, on the outer primer target amplicon and/or the inner primer target amplicon is indicative of a gene fusion comprising the target gene.

**13.** The method of claim 1 or 3, wherein the series of target-specific outer primers comprises at least one primer that binds to a target primer binding site that is between 25 and 150 nucleotides from a known fusion breakpoint for the target gene, and wherein the outer primer target amplicons comprise amplicons that are at least 150 nucleotides long.

**14.** The method of claim 12, wherein the method detects the gene fusion from at least two fusion partner genes selected from the group consisting of AKT1, ALK, BRAF, EGFR, HER2, KRAS, MEK1, MET, NRAS, PIK3CA, RET, and ROS1, and wherein the series of target-specific outer primers comprises at least one primer that binds to a target primer binding site that is between 25 and 150 nucleotides from a known fusion breakpoint for each of the target genes, and wherein the outer primer target amplicons comprise amplicons that are at least 150 nucleotides long.

**15.** The method of claim 3, wherein the series of forward target-specific outer primers and the series of forward target-specific inner primers each comprise at least one primer that binds to a target primer binding site that is a target distance from a known fusion breakpoint for the target gene, and wherein the outer primer target amplicons comprise at least one amplicon that is as long as the target distance.

FIG. 1

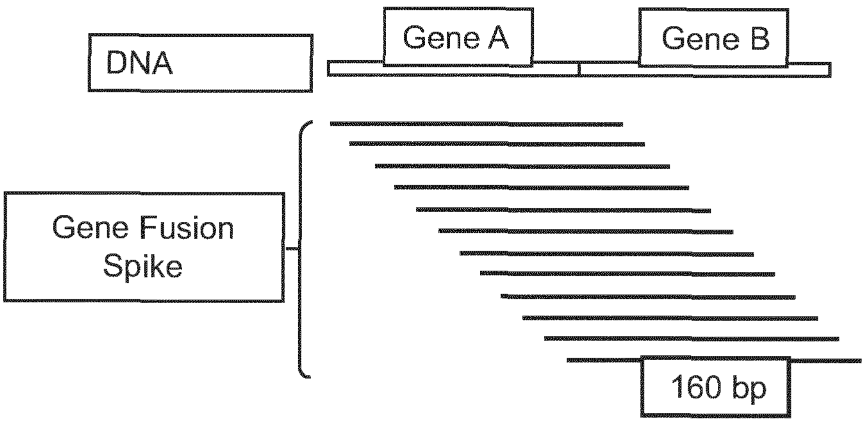
Gene A
Gene B
DNA
Gene Fusion Spike
160 bp

FIG. 2

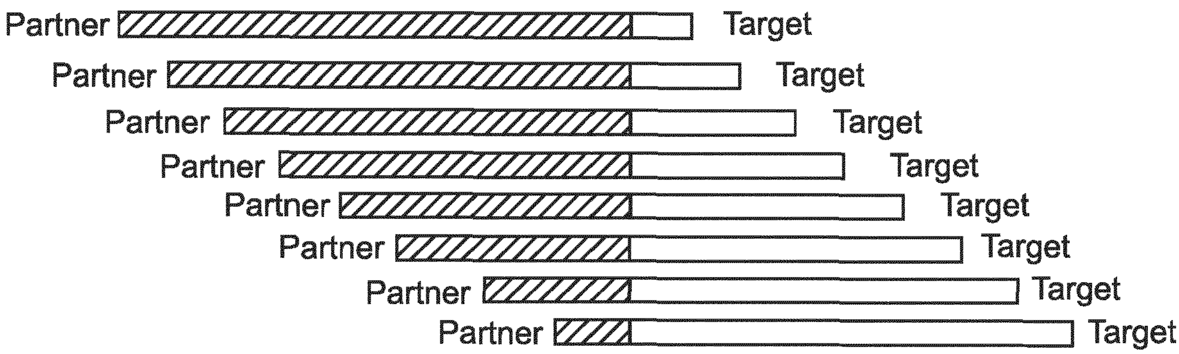
Partner
Target
Partner
Target
Partner
Target
Partner
Target
Partner
Target
Partner
Target
Partner
Target
Partner
Target

FIG. 3

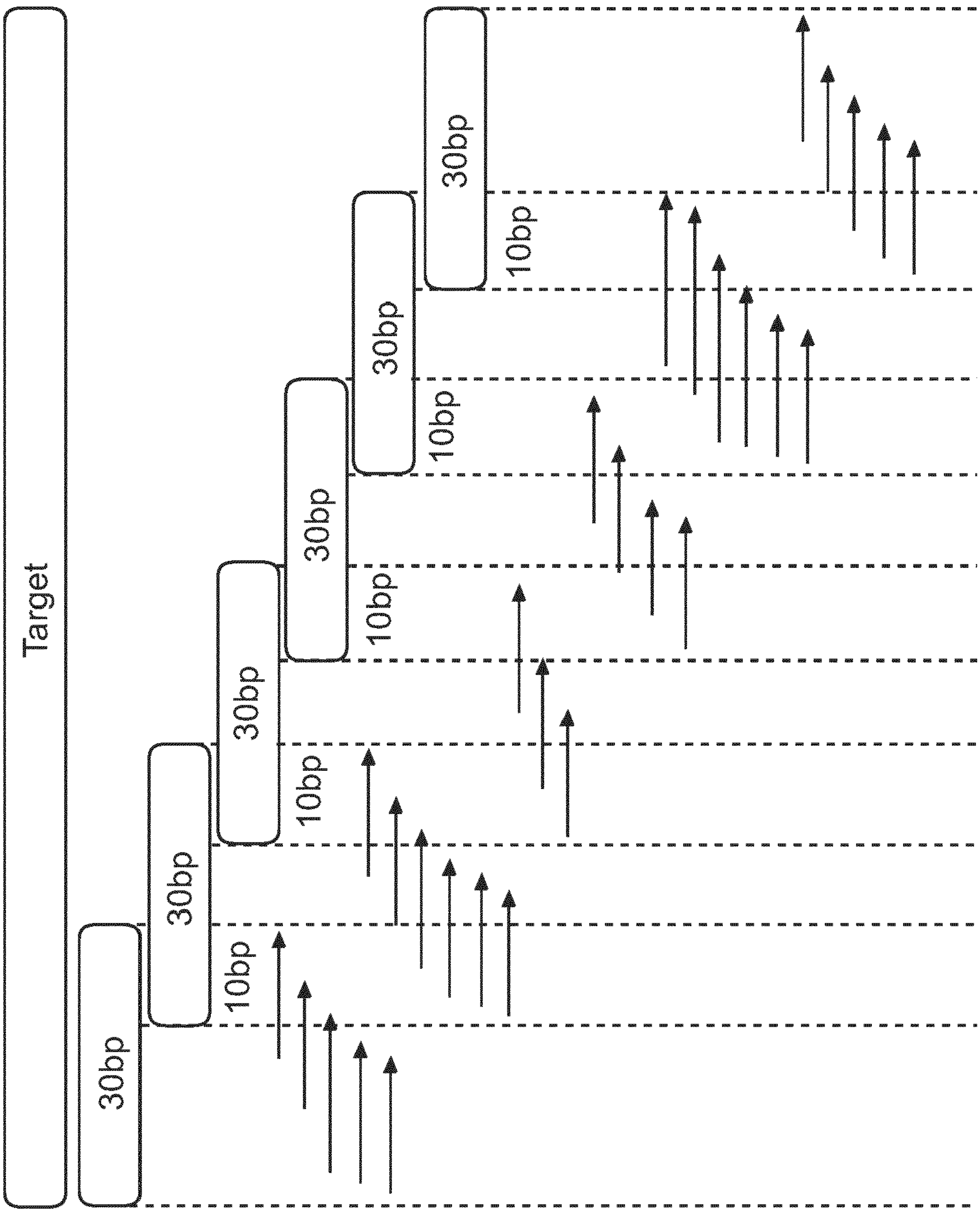
Target
30bp
30bp
30bp
30bp
30bp
30bp
10bp
10bp
10bp
10bp
10bp

FIG. 4

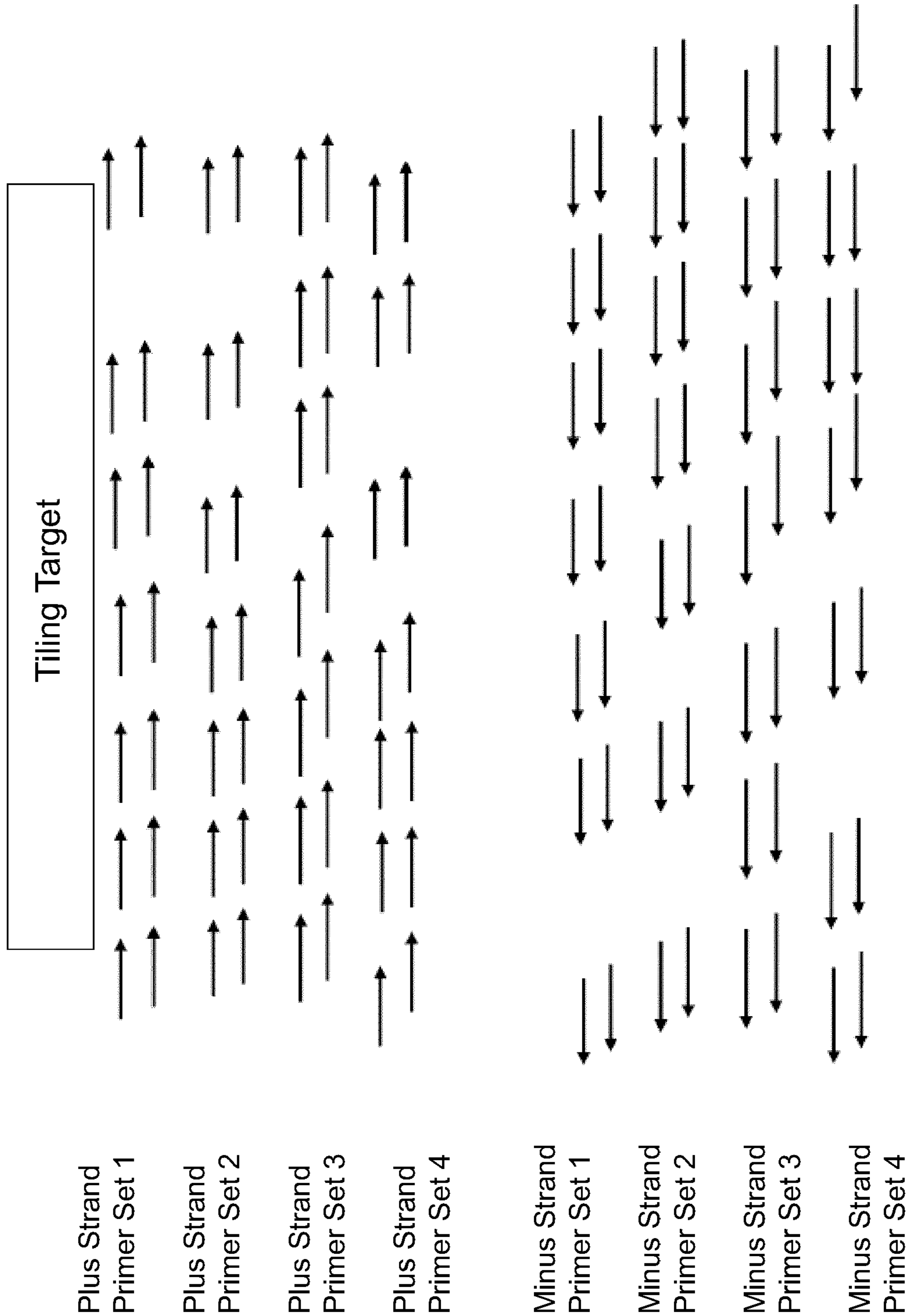
Tiling Target
Plus Strand Primer Set 1
Plus Strand Primer Set 2
Plus Strand Primer Set 3
Plus Strand Primer Set 4
Minus Strand Primer Set 1
Minus Strand Primer Set 2
Minus Strand Primer Set 3
Minus Strand Primer Set 4

FIG. 5

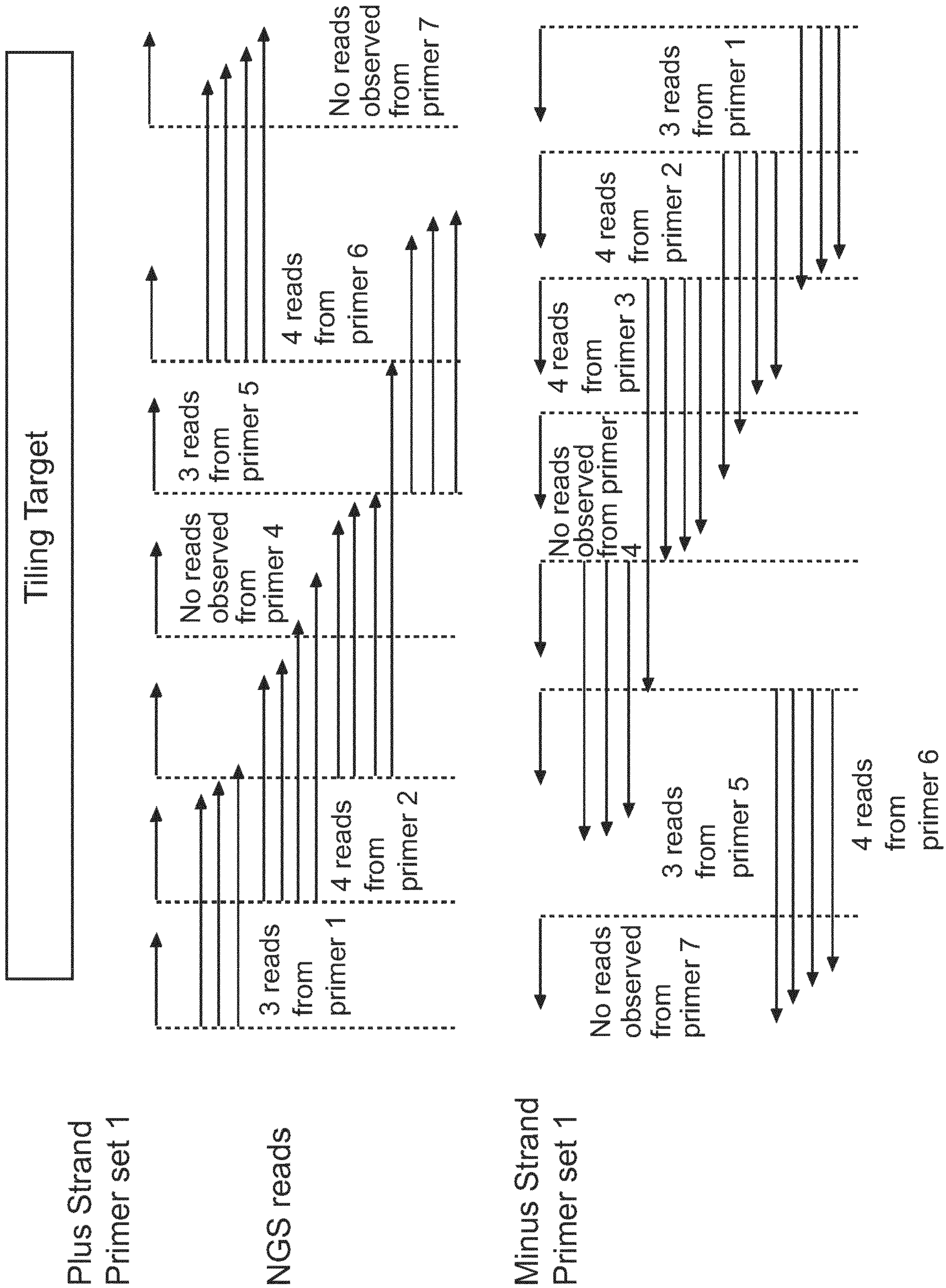
Tiling Target
Plus Strand Primer set 1
NGS reads
Minus Strand Primer set 1
3 reads from primer 1
4 reads from primer 2
No reads observed from primer 4
3 reads from primer 5
4 reads from primer 6
No reads observed from primer 7
3 reads from primer 1
4 reads from primer 2
4 reads from primer 3
No reads observed from primer 4
3 reads from primer 5
4 reads from primer 6
No reads observed from primer 7

FIG. 6A

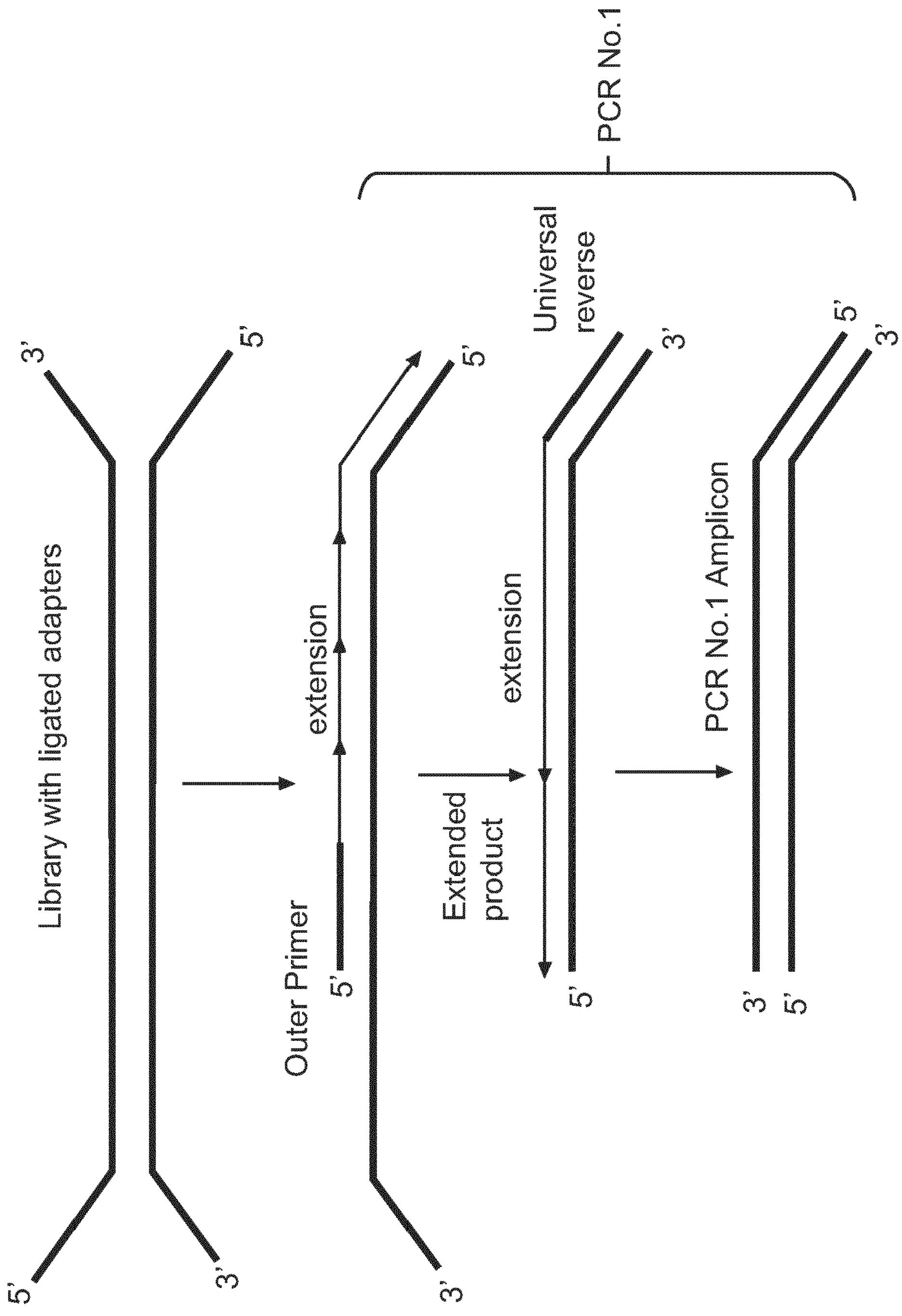
Library with ligated adapters
3'
5'
5'
3'
Outer Primer
5'
extension
5'
3'
Extended product
extension
Universal reverse
3'
5'
PCR No.1
PCR No.1 Amplicon
5'
3'
3'
5'

FIG. 6B

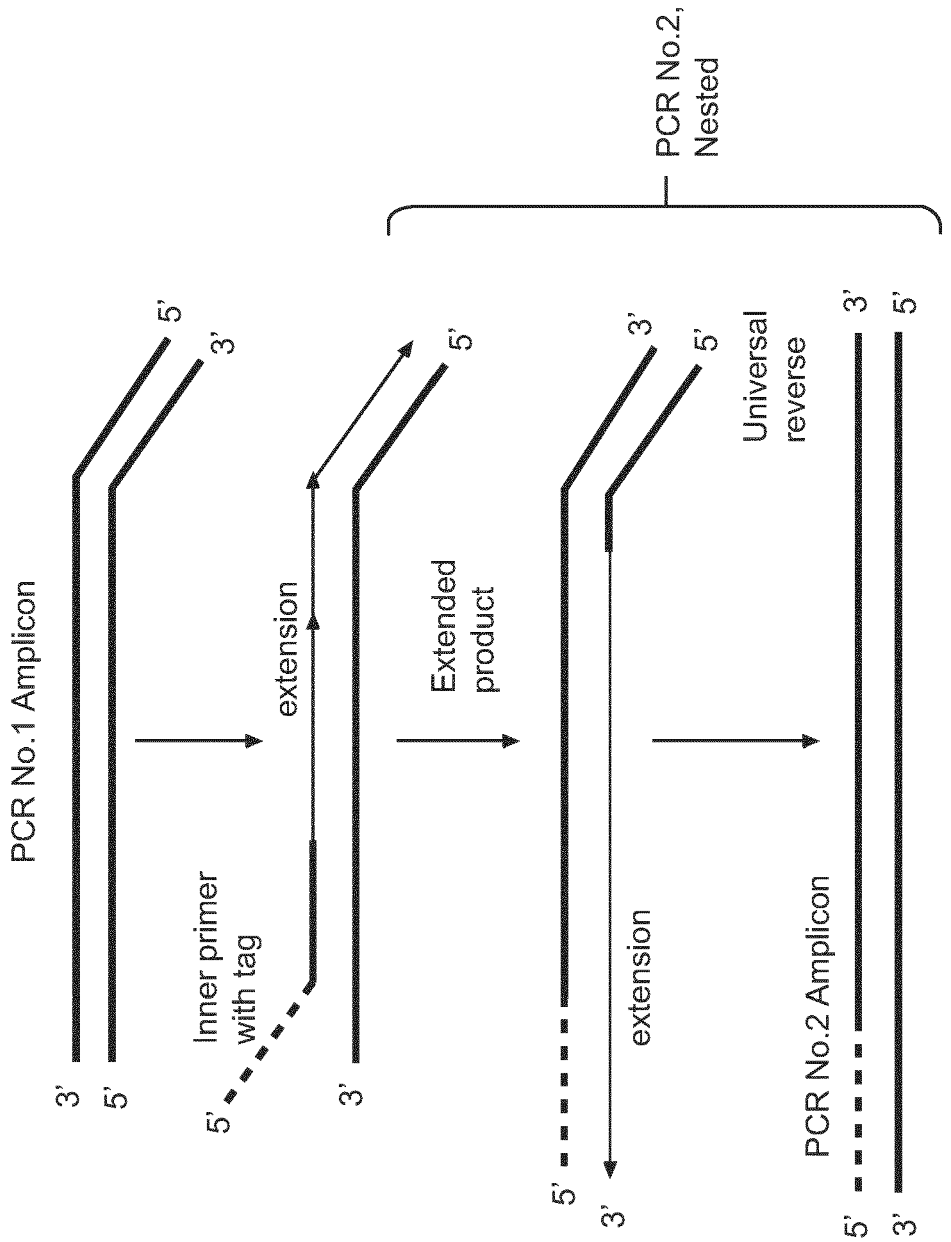
PCR No.1 Amplicon
3'
5'
5'
3'
Inner primer with tag
5'
3'
extension
5'
Extended product
5'
3'
3'
5'
extension
PCR No.2, Nested
Universal reverse
PCR No.2 Amplicon
5'
3'
3'
5'

FIG. 7A

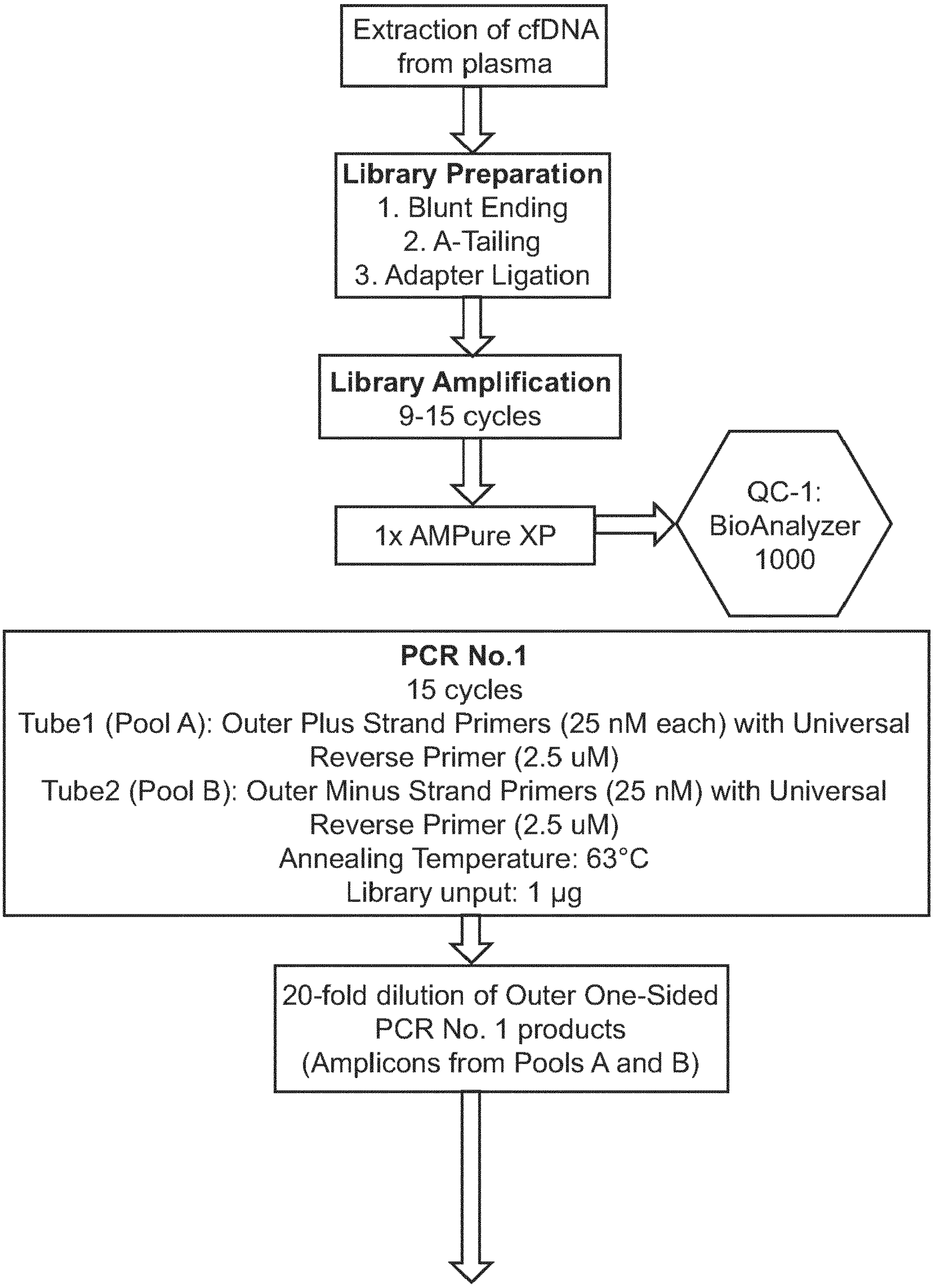
Extraction of cfDNA from plasma
Library Preparation
1. Blunt Ending
2. A-Tailing
3. Adapter Ligation
Library Amplification
9-15 cycles
1x AMPure XP
QC-1: BioAnalyzer 1000
PCR No.1
15 cycles
Tube1 (Pool A): Outer Plus Strand Primers (25 nM each) with Universal Reverse Primer (2.5 uM)
Tube2 (Pool B): Outer Minus Strand Primers (25 nM) with Universal Reverse Primer (2.5 uM)
Annealing Temperature: 63°C
Library unput: 1 µg
20-fold dilution of Outer One-Sided PCR No. 1 products
(Amplicons from Pools A and B)

FIG. 7B

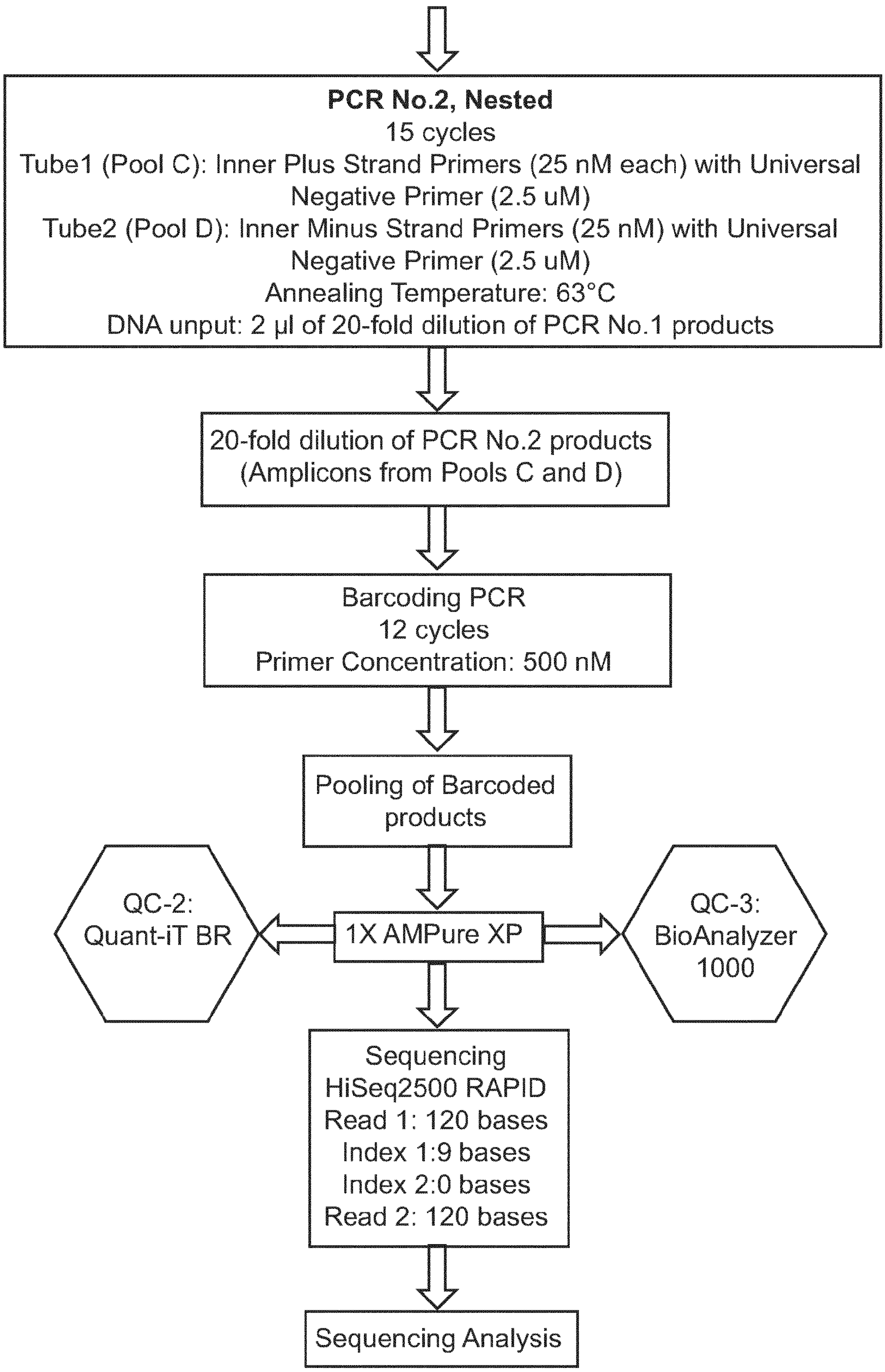
PCR No.2, Nested
15 cycles
Tube1 (Pool C): Inner Plus Strand Primers (25 nM each) with Universal Negative Primer (2.5 uM)
Tube2 (Pool D): Inner Minus Strand Primers (25 nM) with Universal Negative Primer (2.5 uM)
Annealing Temperature: 63°C
DNA unput: 2 µl of 20-fold dilution of PCR No.1 products
20-fold dilution of PCR No.2 products (Amplicons from Pools C and D)
Barcoding PCR
12 cycles
Primer Concentration: 500 nM
Pooling of Barcoded products
QC-2: Quant-iT BR
1X AMPure XP
QC-3: BioAnalyzer 1000
Sequencing
HiSeq2500 RAPID
Read 1: 120 bases
Index 1:9 bases
Index 2:0 bases
Read 2: 120 bases
Sequencing Analysis

FIG. 8A

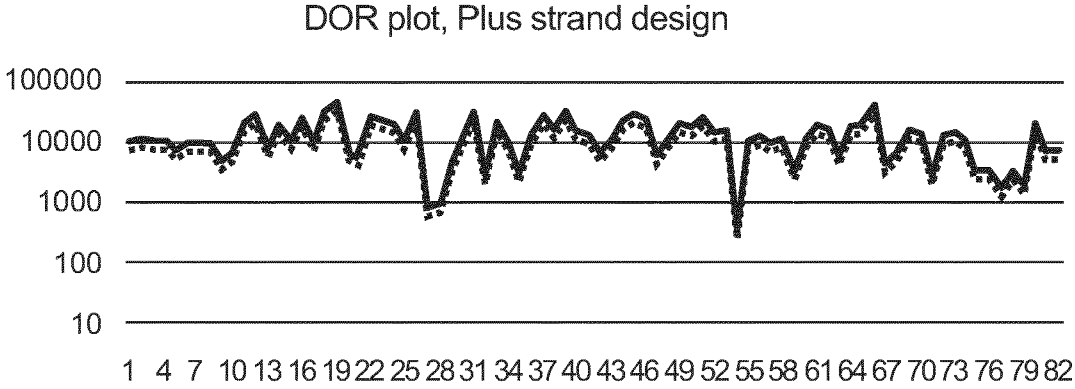


FIG. 8B

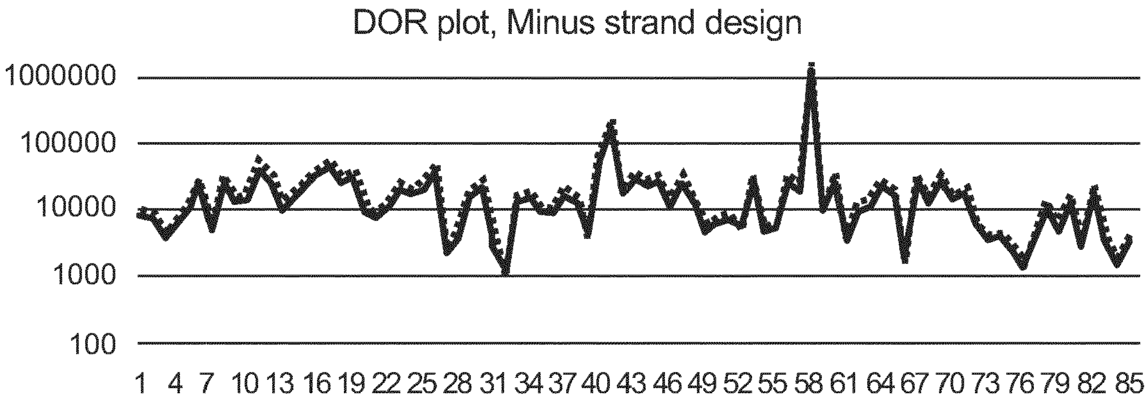


FIG. 8C

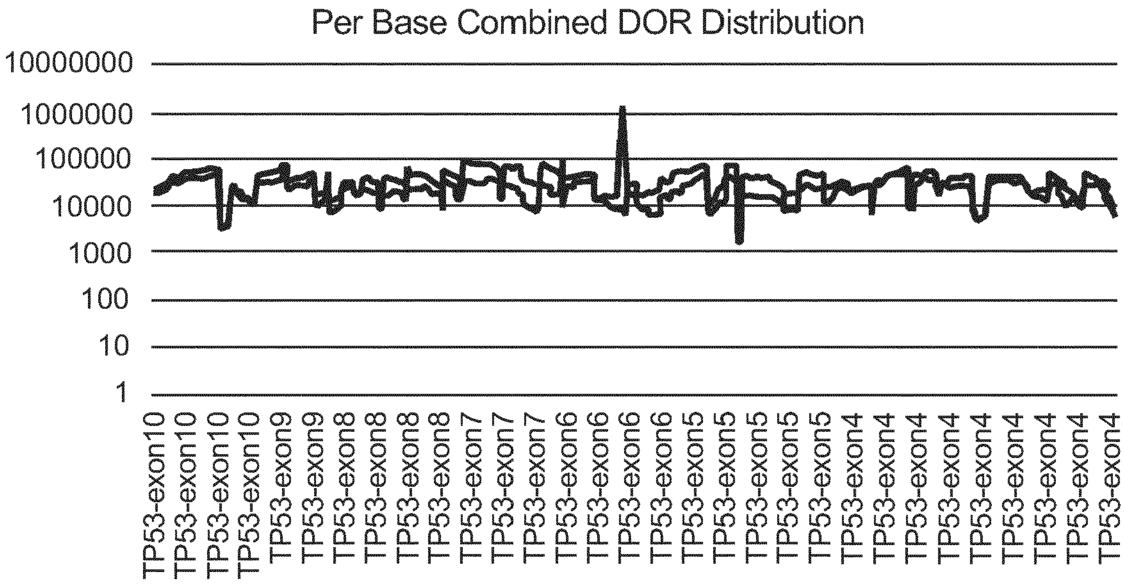

FIG. 9A

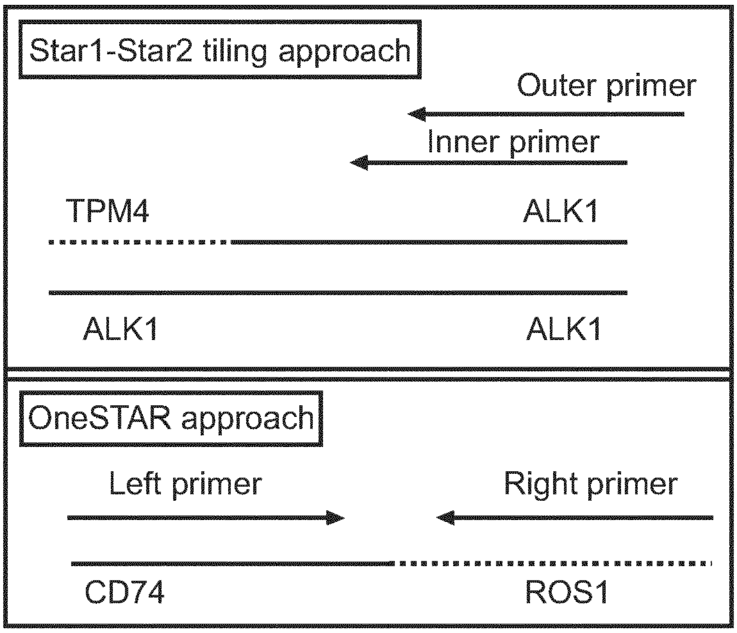
Star1-Star2 tiling approach
Outer primer
Inner primer
TPM4
ALK1
ALK1
ALK1
OneSTAR approach
Left primer
Right primer
CD74
ROS1

FIG. 9B

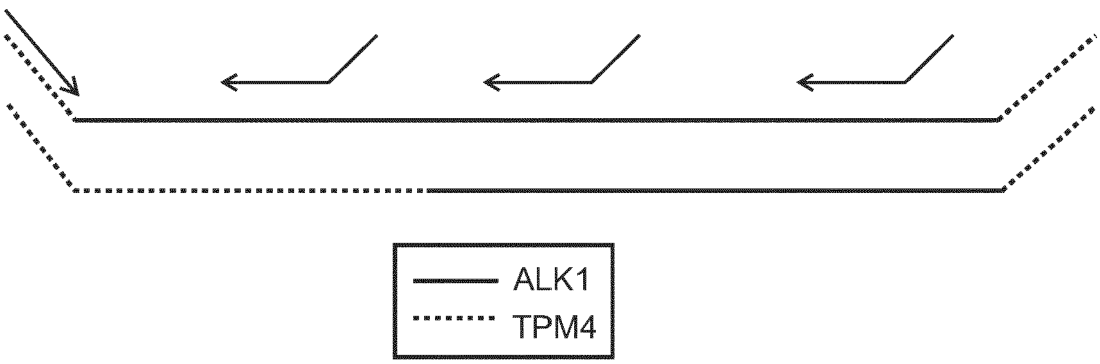
ALK1
TPM4

FIG. 10A

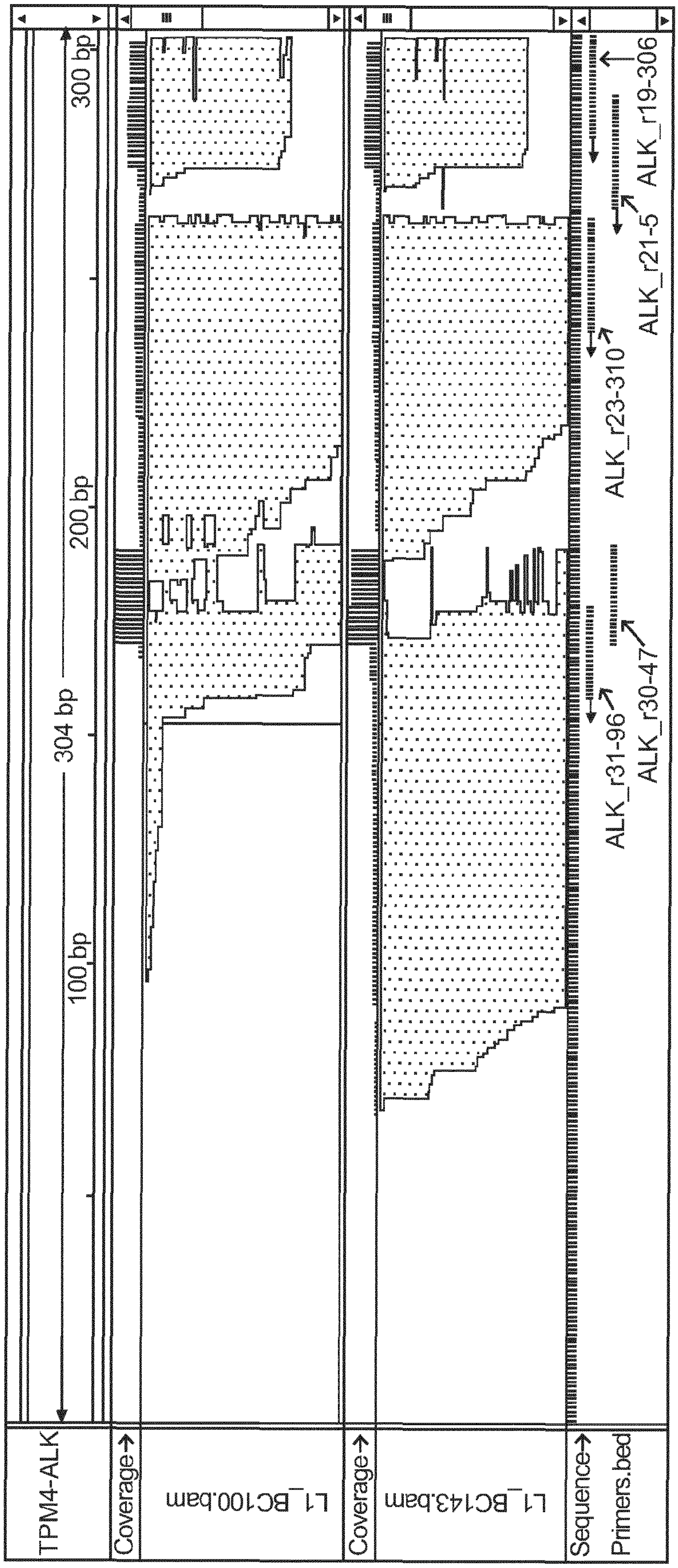
TPM4-ALK
300 bp
200 bp
304 bp
100 bp
Coverage→
L1_BC100.bam
Coverage→
L1_BC143.bam
Sequence→
Primers.bed
ALK_r19-306
ALK_r21-5
ALK_r23-310
ALK_r31-96
ALK_r30-47

FIG. 10B

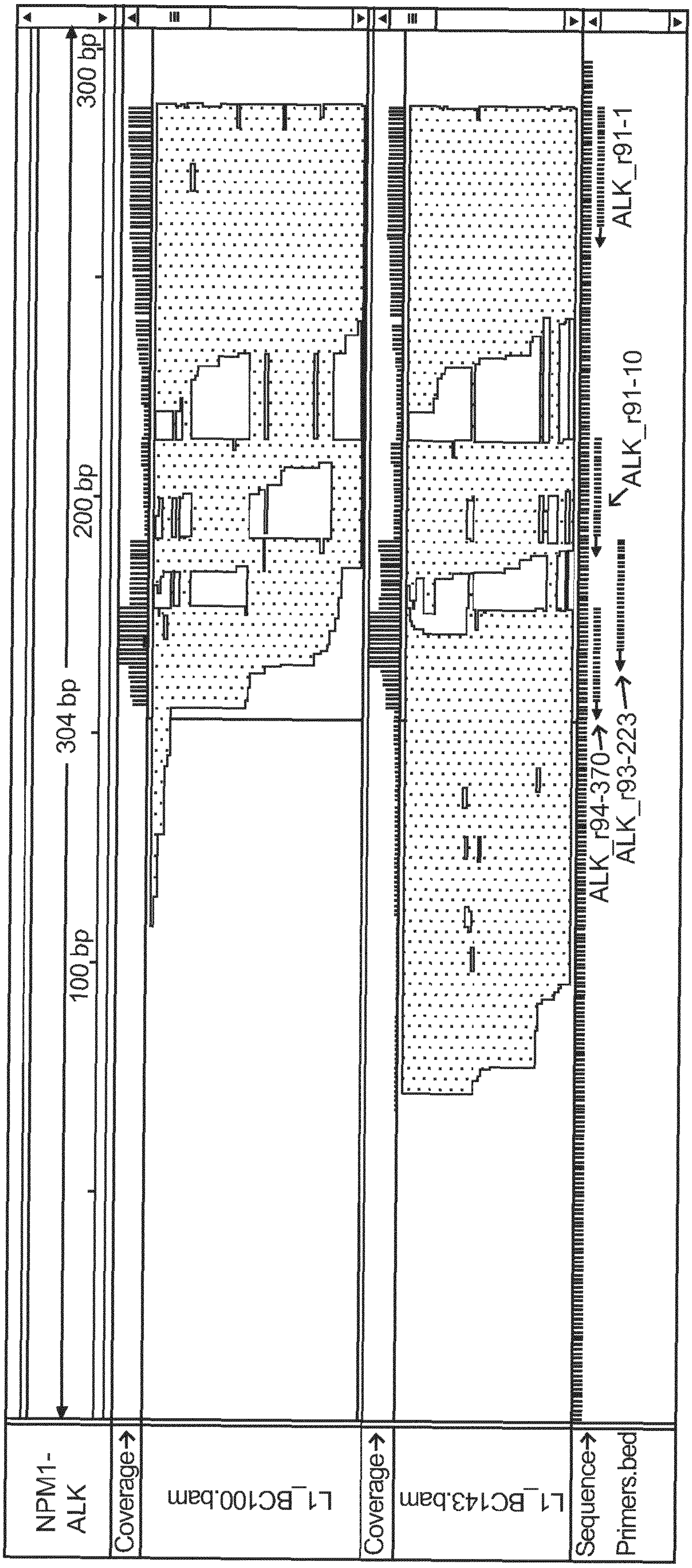
NPM1-ALK
Coverage
L1_BC100.bam
Coverage
L1_BC143.bam
Sequence
Primers.bed
100 bp
304 bp
200 bp
300 bp
ALK_r91-1
ALK_r91-10
ALK_r94-370
ALK_r93-223

FIG. 10C

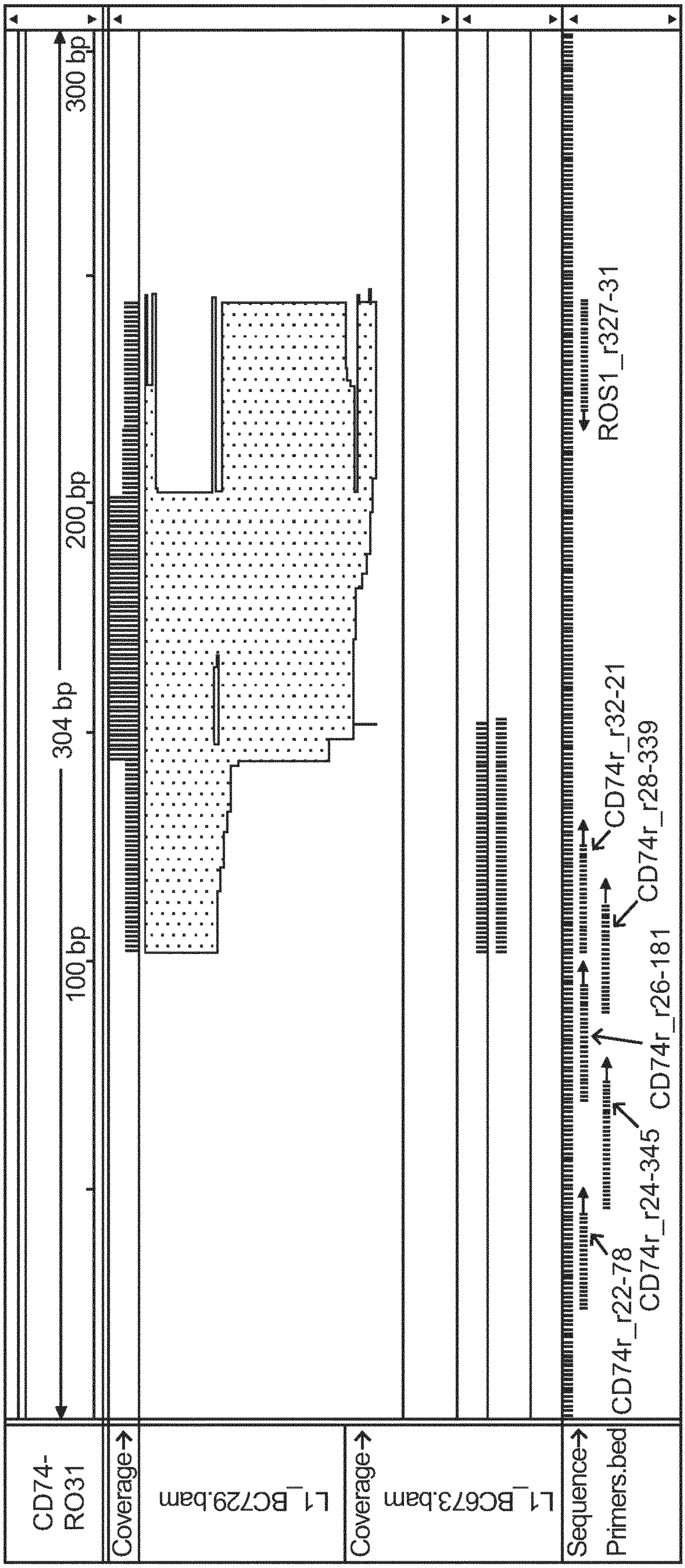
CD74-RO31
Coverage
L1_BC729.bam
Coverage
L1_BC673.bam
Sequence
Primers.bed
100 bp
304 bp
200 bp
300 bp
CD74r_r22-78
CD74r_r24-345
CD74r_r26-181
CD74r_r28-339
CD74r_r32-21
ROS1_r327-31

FIG. 11

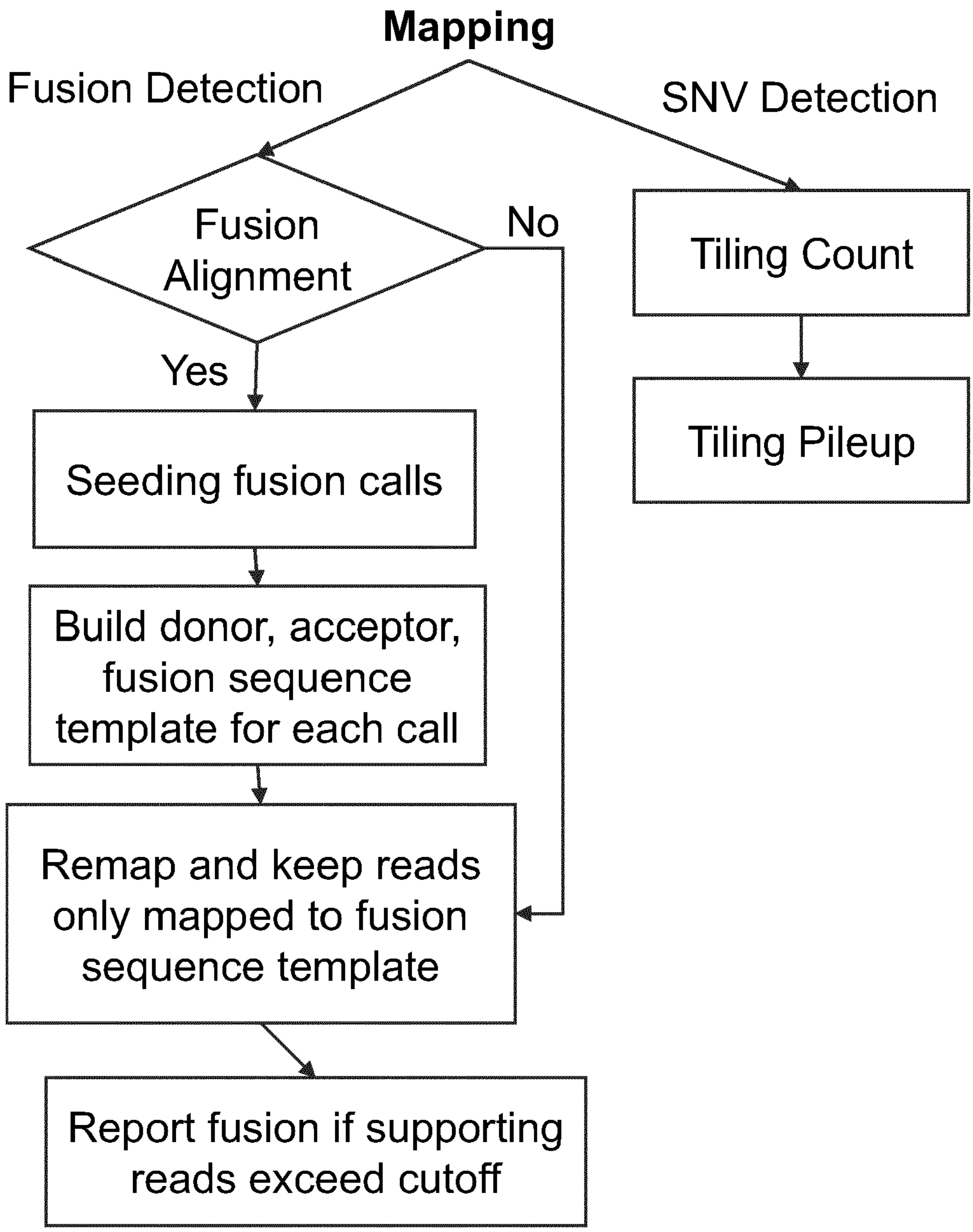
Mapping
Fusion Detection
SNV Detection
Fusion Alignment
No
Yes
Tiling Count
Tiling Pileup
Seeding fusion calls
Build donor, acceptor, fusion sequence template for each call
Remap and keep reads only mapped to fusion sequence template
Report fusion if supporting reads exceed cutoff

FIG. 12

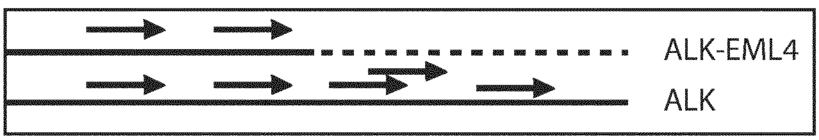
ALK-EML4
ALK

FIG. 13A

| Name | Specific | Start | bp | Tm | SEQ ID NO. | Name | Start | bp | Tm | SEQ ID NO. | Distance |
|---|---|---|---|---|---|---|---|---|---|---|---|
| ALK_r7-54 | TRUE | 29446124 | 22 | 58.5 | 1 | ALK_r7-22 | 29446130 | 23 | 57.8 | 2 | 7 |
| ALK_r9-290 | TRUE | 29446138 | 22 | 54.5 | 3 | ALK_r9-170 | 29446145 | 26 | 56.7 | 4 | 11 |
| ALK_r16-227 | TRUE | 29446207 | 24 | 56.6 | 5 | ALK_r16-183 | 29446223 | 20 | 57.1 | 6 | 12 |
| ALK_r19-341 | TRUE | 29446243 | 21 | 54.9 | 7 | ALK_r19-306 | 29446250 | 21 | 55.5 | 8 | 7 |
| ALK_r21-8 | TRUE | 29446256 | 22 | 58.1 | 9 | ALK_r21-5 | 29446263 | 24 | 57.9 | 10 | 9 |
| ALK_r23-371 | TRUE | 29446283 | 23 | 55.6 | 11 | ALK_r23-310 | 29446289 | 24 | 56.3 | 12 | 7 |
| ALK_r30-269 | TRUE | 29446348 | 21 | 56.1 | 13 | ALK_r30-47 | 29446360 | 21 | 58.3 | 14 | 12 |
| ALK_r30-185 | TRUE | 29446360 | 20 | 57.2 | 15 | ALK_r31-96 | 29446374 | 19 | 58.7 | 16 | 13 |
| ALK_r33-119 | TRUE | 29446384 | 19 | 59.0 | 17 | ALK_r33-43 | 29446390 | 22 | 58.4 | 18 | 9 |
| ALK_r35-362 | TRUE | 29446403 | 19 | 54.8 | 19 | ALK_r35-335 | 29446413 | 18 | 55.2 | 20 | 9 |
| ALK_r37-287 | FALSE | 29446427 | 16 | 54.7 | 21 | ALK_r38-45 | 29446434 | 23 | 58.8 | 22 | 14 |
| ALK_r39-318 | TRUE | 29446443 | 21 | 54.7 | 23 | ALK_r39-254 | 29446450 | 21 | 55.8 | 24 | 7 |
| ALK_r41-36 | FALSE | 29446462 | 24 | 58.7 | 25 | ALK_r42-0 | 29446468 | 25 | 58.0 | 26 | 7 |
| ALK_r44-0 | TRUE | 29446489 | 25 | 58.0 | 27 | ALK_r44-7 | 29446496 | 24 | 58.1 | 28 | 6 |
| ALK_r58-315 | TRUE | 29446625 | 32 | 55.0 | 29 | ALK_r58-148 | 29446636 | 26 | 57.1 | 30 | 5 |
| ALK_r61-83 | TRUE | 29446658 | 26 | 59.7 | 31 | ALK_r61-12 | 29446665 | 24 | 57.9 | 32 | 5 |

# FIG. 13B

| | | | | | | | | | | | |
|---|---|---|---|---|---|---|---|---|---|---|---|
| ALK_r67-201 | FALSE | 29446721 | 23 | 56.2 | 33 | ALK_r68-41 | 29446735 | 22 | 58.7 | 34 | 13 |
| ALK_r70-231 | TRUE | 29446746 | 18 | 55.2 | 35 | ALK_r70-58 | 29446754 | 21 | 59.0 | 36 | 11 |
| ALK_r71-237 | TRUE | 29446765 | 20 | 55.4 | 37 | ALK_r71-193 | 29446771 | 22 | 56.0 | 38 | 8 |
| ALK_r76-336 | FALSE | 29446813 | 19 | 55.3 | 39 | ALK_r76-111 | 29446820 | 21 | 57.6 | 40 | 9 |
| ALK_r78-427 | TRUE | 29446838 | 17 | 54.2 | 41 | ALK_r78-407 | 29446844 | 19 | 54.3 | 42 | 8 |
| ALK_r84-33 | TRUE | 29446885 | 24 | 58.6 | 43 | ALK_r84-1 | 29446894 | 26 | 58.1 | 44 | 11 |
| ALK_r90-372 | FALSE | 29446959 | 20 | 54.2 | 45 | ALK_r91-10 | 29446966 | 21 | 58.1 | 46 | 8 |
| ALK_r93-228 | TRUE | 29446976 | 22 | 56.3 | 47 | ALK_r93-223 | 29446988 | 24 | 56.4 | 48 | 14 |
| ALK_r94-48 | TRUE | 29446989 | 26 | 58.9 | 49 | ALK_r94-370 | 29447003 | 20 | 54.7 | 50 | 8 |
| ALK_r98-310 | TRUE | 29447025 | 29 | 55.3 | 51 | ALK_r98-208 | 29447031 | 29 | 56.8 | 52 | 6 |
| ALK_r101-414 | TRUE | 29447054 | 19 | 54.4 | 53 | ALK_r101-413 | 29447062 | 19 | 54.4 | 54 | 8 |
| ALK_r106-362 | TRUE | 29447105 | 24 | 55.2 | 55 | ALK_r106-20 | 29447112 | 22 | 58.0 | 56 | 5 |
| ALK_r111-249 | TRUE | 29447157 | 20 | 55.2 | 57 | ALK_r111-119 | 29447163 | 21 | 57.2 | 58 | 7 |
| ALK_r113-252 | TRUE | 29447183 | 21 | 55.0 | 59 | ALK_r113-221 | 29447190 | 23 | 55.5 | 60 | 9 |
| ALK_r117-291 | TRUE | 29447215 | 22 | 54.9 | 61 | ALK_r117-6 | 29447221 | 23 | 58.0 | 62 | 7 |
| ALK_r121-332 | TRUE | 29447256 | 18 | 54.0 | 63 | ALK_r121-260 | 29447267 | 26 | 55.0 | 64 | 19 |
| ALK_r136-534 | TRUE | 29447407 | 28 | 54.3 | 65 | ALK_r136-25 | 29447413 | 28 | 57.9 | 66 | 6 |
| ALK_r140-86 | TRUE | 29447455 | 20 | 57.7 | 67 | ALK_r140-24 | 29447462 | 21 | 58.3 | 68 | 8 |
| ALK_r143-418 | TRUE | 29447476 | 20 | 55.4 | 69 | ALK_r143-67 | 29447482 | 22 | 58.6 | 70 | 8 |
| ALK_r145-47 | TRUE | 29447500 | 24 | 58.7 | 71 | ALK_r145-67 | 29447508 | 25 | 58.9 | 72 | 9 |

FIG. 13C

| Name | Specific | Start | bp | Tm | SEQ ID NO. | Name | Start | bp | Tm | SEQ ID NO. | Distance |
|---|---|---|---|---|---|---|---|---|---|---|---|
| ALK_r147-459 | TRUE | 29447516 | 22 | 55.0 | 73 | ALK_r147-179 | 29447523 | 29 | 60.0 | 74 | 14 |
| ALK_r152-325 | TRUE | 29447568 | 26 | 57.0 | 75 | ALK_r152-286 | 29447578 | 22 | 57.1 | 76 | 6 |
| ALK_r154-503 | TRUE | 29447584 | 25 | 55.5 | 77 | ALK_r154-44 | 29447592 | 31 | 58.1 | 78 | 14 |
| ALK_r159-466 | FALSE | 29447647 | 20 | 55.7 | 79 | ALK_r160-10 | 29447657 | 22 | 58.2 | 80 | 12 |
| ALK_r162-390 | TRUE | 29447664 | 21 | 56.7 | 81 | ALK_r162-55 | 29447672 | 22 | 57.9 | 82 | 9 |
| ALK_r163-24 | TRUE | 29447681 | 20 | 58.0 | 83 | ALK_r164-296 | 29447703 | 17 | 60.2 | 84 | 19 |
| ALK_r166-341 | FALSE | 29447712 | 18 | 56.3 | 85 | ALK_r167-313 | 29447723 | 23 | 56.9 | 86 | 16 |
| ALK_r168-59 | TRUE | 29447727 | 23 | 58.6 | 87 | ALK_r168-576 | 29447737 | 20 | 54.4 | 88 | 7 |
| ALK_r171-328 | TRUE | 29447754 | 20 | 56.8 | 89 | ALK_r171-48 | 29447766 | 22 | 58.3 | 90 | 14 |
| ALK_r174-496 | TRUE | 29447789 | 21 | 55.1 | 91 | ALK_r174-59 | 29447798 | 22 | 58.4 | 92 | 10 |
| ALK_r178-425 | TRUE | 29447830 | 20 | 56.2 | 93 | ALK_r178-392 | 29447838 | 21 | 56.4 | 94 | 9 |
| ALK_r183-530 | TRUE | 29447878 | 26 | 54.9 | 95 | ALK_r183-5 | 29447888 | 25 | 58.2 | 96 | 9 |
| ALK_r186-378 | TRUE | 29447912 | 19 | 56.2 | 97 | ALK_r186-230 | 29447919 | 21 | 57.3 | 98 | 9 |
| ALK_r187-5 | TRUE | 29447923 | 23 | 58.0 | 99 | ALK_r187-3 | 29447929 | 24 | 58.0 | 100 | 7 |
| ALK_r188-191 | TRUE | 29447936 | 25 | 57.3 | 101 | ALK_r189-123 | 29447943 | 27 | 59.3 | 102 | 9 |
| ALK_r193-128 | TRUE | 29447977 | 19 | 58.9 | 103 | ALK_r193-66 | 29447983 | 22 | 58.6 | 104 | 9 |
| ALK_r194-506 | TRUE | 29447988 | 22 | 54.2 | 105 | ALK_r194-153 | 29447994 | 25 | 57.3 | 106 | 9 |

FIG. 13D

| | | | | | | | | | | | |
|---|---|---|---|---|---|---|---|---|---|---|---|
| ALK_r196-247 | TRUE | 29448015 | 19 | 59.7 | 107 | ALK_r196-119 | 29448021 | 19 | 57.8 | 108 | 6 |
| ALK_r201-323 | TRUE | 29448056 | 22 | 60.7 | 109 | ALK_r201-86 | 29448062 | 20 | 58.2 | 110 | 4 |
| ALK_r204-50 | TRUE | 29448087 | 21 | 58.0 | 111 | ALK_r204-32 | 29448095 | 20 | 58.0 | 112 | 7 |
| ALK_r206-208 | TRUE | 29448104 | 23 | 60.0 | 113 | ALK_r206-29 | 29448110 | 22 | 58.2 | 114 | 5 |
| ALK_r211-392 | TRUE | 29448154 | 22 | 55.4 | 115 | ALK_r211-9 | 29448160 | 22 | 57.9 | 116 | 6 |
| ALK_r213-226 | TRUE | 29448184 | 21 | 57.2 | 117 | ALK_r213-133 | 29448192 | 21 | 57.5 | 118 | 8 |
| ALK_r216-428 | TRUE | 29448211 | 25 | 55.0 | 119 | ALK_r216-390 | 29448218 | 24 | 55.4 | 120 | 6 |
| ALK_r221-33 | TRUE | 29448255 | 25 | 58.5 | 121 | ALK_r221-23 | 29448262 | 26 | 57.9 | 122 | 8 |
| ALK_r224-155 | TRUE | 29448296 | 21 | 59.3 | 123 | ALK_r224-66 | 29448302 | 19 | 57.9 | 124 | 4 |
| ALK_r228-418 | TRUE | 29448329 | 22 | 56.4 | 125 | ALK_r228-284 | 29448336 | 21 | 60.1 | 126 | 6 |
| ALK_r230-213 | TRUE | 29448352 | 17 | 58.9 | 127 | ALK_r230-172 | 29448363 | 20 | 58.8 | 128 | 14 |
| ALK_r232-445 | TRUE | 29448366 | 19 | 56.5 | 129 | ALK_r232-431 | 29448376 | 22 | 56.5 | 130 | 13 |
| ALK_r236-430 | TRUE | 29448410 | 18 | 60.8 | 131 | ALK_r236-415 | 29448418 | 23 | 61.0 | 132 | 13 |
| ALK_r240-267 | TRUE | 29448451 | 26 | 60.1 | 133 | ALK_r240-13 | 29448457 | 25 | 58.2 | 134 | 5 |
| ALK_r242-147 | TRUE | 29448464 | 25 | 59.2 | 135 | ALK_r242-6 | 29448470 | 24 | 58.0 | 136 | 5 |
| ALK_r245-468 | TRUE | 29448500 | 24 | 55.4 | 137 | ALK_r245-290 | 29448506 | 25 | 57.0 | 138 | 7 |
| ALK_r246-178 | TRUE | 29448512 | 28 | 57.5 | 139 | ALK_r247-148 | 29448518 | 29 | 59.1 | 140 | 7 |
| ALK_r248-250 | TRUE | 29448535 | 24 | 60.0 | 141 | ALK_r249-205 | 29448541 | 24 | 59.5 | 142 | 6 |
| ALK_r254-124 | TRUE | 29448586 | 23 | 58.9 | 143 | ALK_r254-25 | 29448592 | 25 | 58.3 | 144 | 8 |
| ALK_r258-405 | TRUE | 29448628 | 28 | 56.6 | 145 | ALK_r258-47 | 29448634 | 28 | 58.1 | 146 | 6 |

FIG. 13E

| Name | Specific | Start | bp | Tm | SEQ ID NO. | Name | Start | bp | Tm | SEQ ID NO. | Distance |
|---|---|---|---|---|---|---|---|---|---|---|---|
| ALK_r260-647 | FALSE | 29448652 | 26 | 54.6 | 147 | ALK_r262-592 | 29448679 | 23 | 54.9 | 148 | 24 |
| ALK_r270-438 | TRUE | 29448747 | 22 | 55.6 | 149 | ALK_r270-350 | 29448760 | 21 | 56.4 | 150 | 12 |
| ALK_r272-98 | TRUE | 29448768 | 23 | 59.3 | 151 | ALK_r272-178 | 29448774 | 26 | 60.2 | 152 | 9 |
| ALK_r273-349 | TRUE | 29448786 | 26 | 55.7 | 153 | ALK_r274-279 | 29448792 | 28 | 56.8 | 154 | 8 |
| ALK_r279-103 | TRUE | 29448842 | 22 | 58.9 | 155 | ALK_r279-61 | 29448853 | 20 | 58.1 | 156 | 9 |
| ALK_r282-236 | TRUE | 29448868 | 19 | 56.0 | 157 | ALK_r282-263 | 29448874 | 24 | 55.6 | 158 | 11 |
| ALK_r283-314 | TRUE | 29448880 | 25 | 55.2 | 159 | ALK_r283-169 | 29448888 | 23 | 56.9 | 160 | 6 |
| ALK_r290-171 | TRUE | 29448944 | 23 | 56.7 | 161 | ALK_r290-120 | 29448963 | 18 | 59.7 | 162 | 14 |
| ALK_r292-143 | TRUE | 29448967 | 20 | 60.2 | 163 | ALK_r292-237 | 29448973 | 22 | 55.5 | 164 | 8 |
| ALK_r296-224 | TRUE | 29449004 | 24 | 55.5 | 165 | ALK_r296-54 | 29449010 | 32 | 58.5 | 166 | 14 |
| ALK_r328-174 | FALSE | 29449324 | 23 | 56.9 | 167 | ALK_r331-191 | 29449370 | 22 | 56.7 | 168 | 45 |
| ALK_r333-336 | TRUE | 29449375 | 23 | 54.6 | 169 | ALK_r333-232 | 29449387 | 24 | 56.6 | 170 | 13 |
| ALK_r334-0 | TRUE | 29449392 | 24 | 58.0 | 171 | ALK_r335-179 | 29449405 | 25 | 57.2 | 172 | 14 |
| ALK_r338-333 | TRUE | 29449429 | 25 | 55.7 | 173 | ALK_r338-7 | 29449436 | 25 | 58.2 | 174 | 7 |
| ALK_r340-68 | TRUE | 29449446 | 23 | 58.8 | 175 | ALK_r340-59 | 29449452 | 21 | 58.4 | 176 | 4 |
| ALK_r341-77 | TRUE | 29449464 | 22 | 57.8 | 177 | ALK_r341-444 | 29449472 | 21 | 54.5 | 178 | 7 |
| ALK_r346-608 | TRUE | 29449508 | 22 | 54.1 | 179 | ALK_r346-597 | 29449515 | 24 | 54.2 | 180 | 9 |

FIG. 13F

| | | | | | | | | | | | |
|---|---|---|---|---|---|---|---|---|---|---|---|
| ALK_r347-148 | TRUE | 29449519 | 25 | 57.5 | 181 | ALK_r347-63 | 29449526 | 24 | 58.7 | 182 | 6 |
| ALK_r349-516 | TRUE | 29449542 | 18 | 54.2 | 183 | ALK_r349-384 | 29449552 | 20 | 55.6 | 184 | 12 |
| ALK_r356-192 | TRUE | 29449611 | 25 | 59.7 | 185 | ALK_r356-175 | 29449618 | 23 | 57.5 | 186 | 5 |
| ALK_r359-49 | TRUE | 29449634 | 22 | 58.3 | 187 | ALK_r359-28 | 29449641 | 20 | 58.1 | 188 | 5 |
| ALK_r361-385 | TRUE | 29449660 | 25 | 56.3 | 189 | ALK_r361-373 | 29449667 | 26 | 56.4 | 190 | 8 |
| ALK_r362-33 | TRUE | 29449674 | 29 | 58.0 | 191 | ALK_r363-81 | 29449684 | 25 | 57.7 | 192 | 6 |
| ALK_r366-12 | TRUE | 29449710 | 26 | 58.0 | 193 | ALK_r366-8 | 29449717 | 25 | 58.0 | 194 | 6 |
| ALK_r372-299 | TRUE | 29449769 | 18 | 57.4 | 195 | ALK_r372-251 | 29449775 | 21 | 57.4 | 196 | 9 |
| ALK_r373-24 | TRUE | 29449783 | 24 | 57.9 | 197 | ALK_r373-60 | 29449791 | 21 | 58.3 | 198 | 5 |
| ALK_r378-220 | TRUE | 29449824 | 26 | 59.8 | 199 | ALK_r378-155 | 29449830 | 26 | 59.5 | 200 | 6 |
| ALK_r380-444 | TRUE | 29449849 | 22 | 56.8 | 201 | ALK_r380-298 | 29449857 | 25 | 57.2 | 202 | 11 |
| ALK_r381-310 | TRUE | 29449862 | 29 | 60.0 | 203 | ALK_r382-221 | 29449871 | 25 | 57.5 | 204 | 5 |
| ALK_r386-358 | TRUE | 29449904 | 31 | 60.1 | 205 | ALK_r386-199 | 29449911 | 27 | 59.5 | 206 | 3 |
| ALK_r387-175 | TRUE | 29449925 | 24 | 59.1 | 207 | ALK_r388-583 | 29449934 | 24 | 56.2 | 208 | 9 |
| ALK_r390-186 | TRUE | 29449944 | 24 | 59.1 | 209 | ALK_r390-129 | 29449960 | 20 | 58.4 | 210 | 12 |
| ALK_r391-359 | FALSE | 29449968 | 16 | 57.5 | 211 | ALK_r391-637 | 29449974 | 18 | 56.0 | 212 | 8 |
| ALK_r394-51 | TRUE | 29449986 | 25 | 57.9 | 213 | ALK_r394-11 | 29449993 | 26 | 58.0 | 214 | 8 |
| ALK_r396-703 | TRUE | 29450012 | 19 | 55.5 | 215 | ALK_r396-369 | 29450019 | 21 | 57.3 | 216 | 9 |
| ALK_r396-95 | TRUE | 29450022 | 21 | 58.4 | 217 | ALK_r397-56 | 29450030 | 21 | 58.1 | 218 | 8 |
| ALK_r399-226 | TRUE | 29450040 | 25 | 59.6 | 219 | ALK_r399-132 | 29450047 | 26 | 59.1 | 220 | 8 |

FIG. 13G

| Name | Specific | Start | bp | Tm | SEQ ID NO. | Name | Start | bp | Tm | SEQ ID NO. | Distance |
|---|---|---|---|---|---|---|---|---|---|---|---|
| ROS1_r3-257 | TRUE | 117642245 | 26 | 56.6 | 221 | ROS1_r4-138 | 117642251 | 25 | 57.4 | 222 | 5 |
| ROS1_r10-259 | TRUE | 117642315 | 25 | 54.5 | 223 | ROS1_r10-179 | 117642321 | 24 | 56.1 | 224 | 5 |
| ROS1_r12-5 | TRUE | 117642329 | 24 | 58.2 | 225 | ROS1_r12-2 | 117642336 | 23 | 58.0 | 226 | 6 |
| ROS1_r16-156 | TRUE | 117642367 | 25 | 60.4 | 227 | ROS1_r16-129 | 117642376 | 25 | 60.0 | 228 | 9 |
| ROS1_r17-344 | TRUE | 117642384 | 23 | 54.3 | 229 | ROS1_r17-14 | 117642391 | 25 | 57.9 | 230 | 9 |
| ROS1_r25-163 | TRUE | 117642461 | 19 | 57.3 | 231 | ROS1_r25-66 | 117642467 | 21 | 58.9 | 232 | 8 |
| ROS1_r29-185 | TRUE | 117642499 | 24 | 55.2 | 233 | ROS1_r29-101 | 117642505 | 21 | 57.3 | 234 | 3 |
| ROS1_r86-36 | TRUE | 117643067 | 31 | 56.4 | 235 | ROS1_r86-22 | 117643077 | 27 | 56.6 | 236 | 6 |
| ROS1_r101-66 | TRUE | 117643217 | 31 | 55.7 | 237 | ROS1_r101-6 | 117643226 | 30 | 59.0 | 238 | 8 |
| ROS1_r122-135 | TRUE | 117643427 | 25 | 55.3 | 239 | ROS1_r122-0 | 117643435 | 25 | 58.0 | 240 | 8 |
| ROS1_r123-157 | TRUE | 117643445 | 26 | 54.8 | 241 | ROS1_r123-91 | 117643455 | 21 | 56.3 | 242 | 5 |
| ROS1_r131-244 | TRUE | 117643520 | 30 | 54.9 | 243 | ROS1_r131-34 | 117643526 | 29 | 58.7 | 244 | 5 |
| ROS1_r141-155 | TRUE | 117643617 | 31 | 55.6 | 245 | ROS1_r141-18 | 117643624 | 30 | 57.9 | 246 | 6 |
| ROS1_r147-1 | FALSE | 117643681 | 26 | 58.0 | 247 | ROS1_r147-73 | 117643687 | 28 | 57.1 | 248 | 8 |
| ROS1_r168-51 | TRUE | 117643896 | 26 | 56.2 | 249 | ROS1_r168-19 | 117643902 | 23 | 57.3 | 250 | 3 |
| ROS1_r172-9 | TRUE | 117643928 | 24 | 57.6 | 251 | ROS1_r172-1 | 117643934 | 24 | 58.7 | 252 | 6 |
| ROS1_r174-119 | TRUE | 117643949 | 26 | 54.9 | 253 | ROS1_r174-3 | 117643955 | 30 | 58.2 | 254 | 10 |

FIG. 13H

| | | | | | | | | | | | |
|---|---|---|---|---|---|---|---|---|---|---|---|
| ROS1_r197-343 | TRUE | 117644186 | 24 | 55.4 | 255 | ROS1_r197-215 | 117644192 | 22 | 56.8 | 256 | 4 |
| ROS1_r198-169 | TRUE | 117644197 | 25 | 57.2 | 257 | ROS1_r199-9 | 117644204 | 25 | 58.3 | 258 | 7 |
| ROS1_r200-147 | TRUE | 117644212 | 30 | 57.6 | 259 | ROS1_r200-379 | 117644219 | 26 | 55.9 | 260 | 3 |
| ROS1_r203-117 | TRUE | 117644249 | 21 | 57.6 | 261 | ROS1_r203-83 | 117644256 | 20 | 57.7 | 262 | 6 |
| ROS1_r205-482 | FALSE | 117644262 | 18 | 54.2 | 263 | ROS1_r206-4 | 117644270 | 24 | 58.2 | 264 | 14 |
| ROS1_r208-371 | TRUE | 117644295 | 26 | 55.2 | 265 | ROS1_r208-365 | 117644301 | 25 | 55.3 | 266 | 5 |
| ROS1_r210-200 | TRUE | 117644311 | 28 | 60.8 | 267 | ROS1_r210-120 | 117644319 | 26 | 59.8 | 268 | 6 |
| ROS1_r214-479 | TRUE | 117644350 | 26 | 55.5 | 269 | ROS1_r214-461 | 117644356 | 26 | 55.7 | 270 | 6 |
| ROS1_r228-175 | TRUE | 117644490 | 27 | 55.6 | 271 | ROS1_r228-148 | 117644496 | 26 | 56.1 | 272 | 5 |
| ROS1_r234-259 | TRUE | 117644552 | 26 | 54.4 | 273 | ROS1_r234-220 | 117644558 | 25 | 54.8 | 274 | 5 |
| ROS1_r246-87 | TRUE | 117644667 | 30 | 55.9 | 275 | ROS1_r246-16 | 117644673 | 33 | 58.7 | 276 | 9 |
| ROS1_r251-103 | TRUE | 117644720 | 22 | 54.2 | 277 | ROS1_r251-86 | 117644726 | 25 | 54.6 | 278 | 9 |
| ROS1_r277-220 | TRUE | 117644977 | 25 | 55.0 | 279 | ROS1_r277-109 | 117644983 | 24 | 57.0 | 280 | 5 |
| ROS1_r278-246 | TRUE | 117644994 | 20 | 55.0 | 281 | ROS1_r278-5 | 117645000 | 26 | 58.0 | 282 | 12 |
| ROS1_r284-65 | TRUE | 117645047 | 25 | 57.4 | 283 | ROS1_r284-47 | 117645056 | 29 | 57.7 | 284 | 13 |
| ROS1_r286-183 | TRUE | 117645068 | 31 | 55.9 | 285 | ROS1_r286-155 | 117645074 | 30 | 56.3 | 286 | 5 |
| ROS1_r297-141 | TRUE | 117645177 | 33 | 56.1 | 287 | ROS1_r297-136 | 117645184 | 31 | 56.1 | 288 | 5 |
| ROS1_r327-381 | TRUE | 117645482 | 28 | 54.1 | 289 | ROS1_r327-313 | 117645489 | 24 | 54.7 | 290 | 3 |
| ROS1_r340-227 | TRUE | 117645612 | 27 | 56.4 | 291 | ROS1_r340-34 | 117645618 | 26 | 59.0 | 292 | 5 |
| ROS1_r343-234 | TRUE | 117645637 | 30 | 55.9 | 293 | ROS1_r343-11 | 117645647 | 29 | 58.1 | 294 | 9 |
| ROS1_r345-156 | TRUE | 117645658 | 33 | 56.9 | 295 | ROS1_r345-13 | 117645667 | 29 | 58.3 | 296 | 5 |

FIG. 14

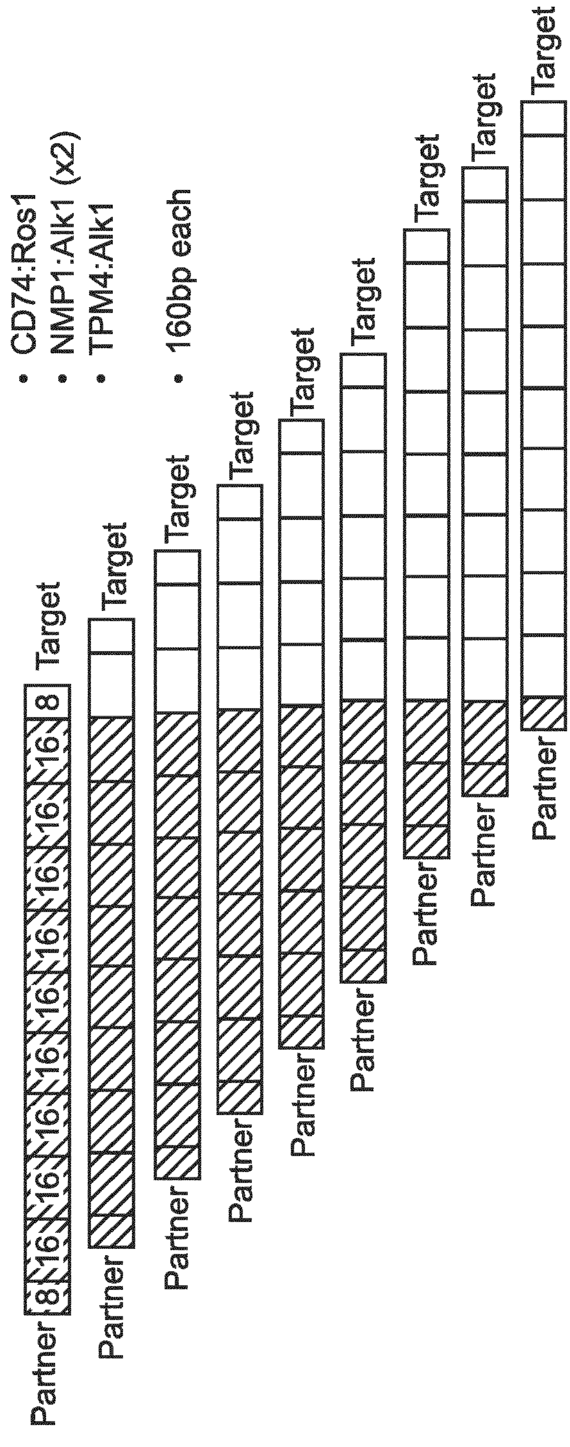

• CD74:Ros1
• NMP1:Alk1 (x2)
• TPM4:Alk1
• 160bp each
Partner
Target
8
16

FIG. 15

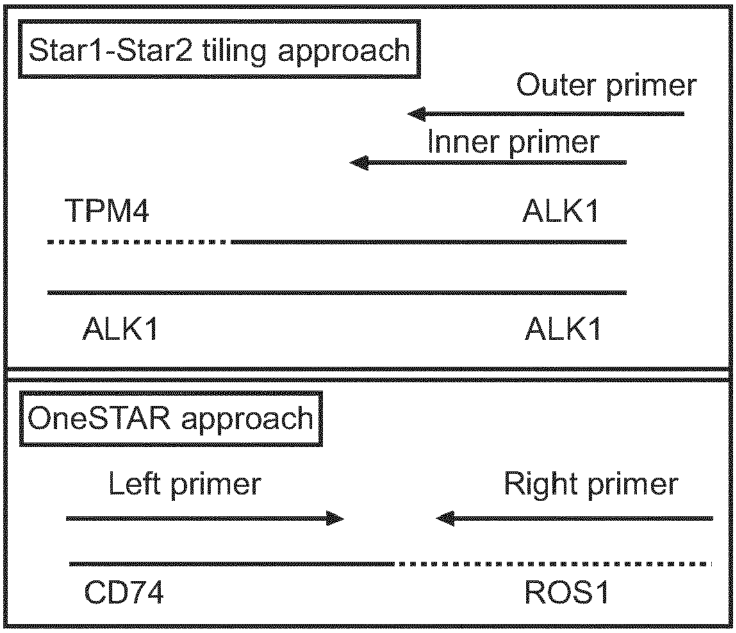
Star1-Star2 tiling approach
Outer primer
Inner primer
TPM4
ALK1
ALK1
ALK1
OneSTAR approach
Left primer
Right primer
CD74
ROS1

FIG. 16

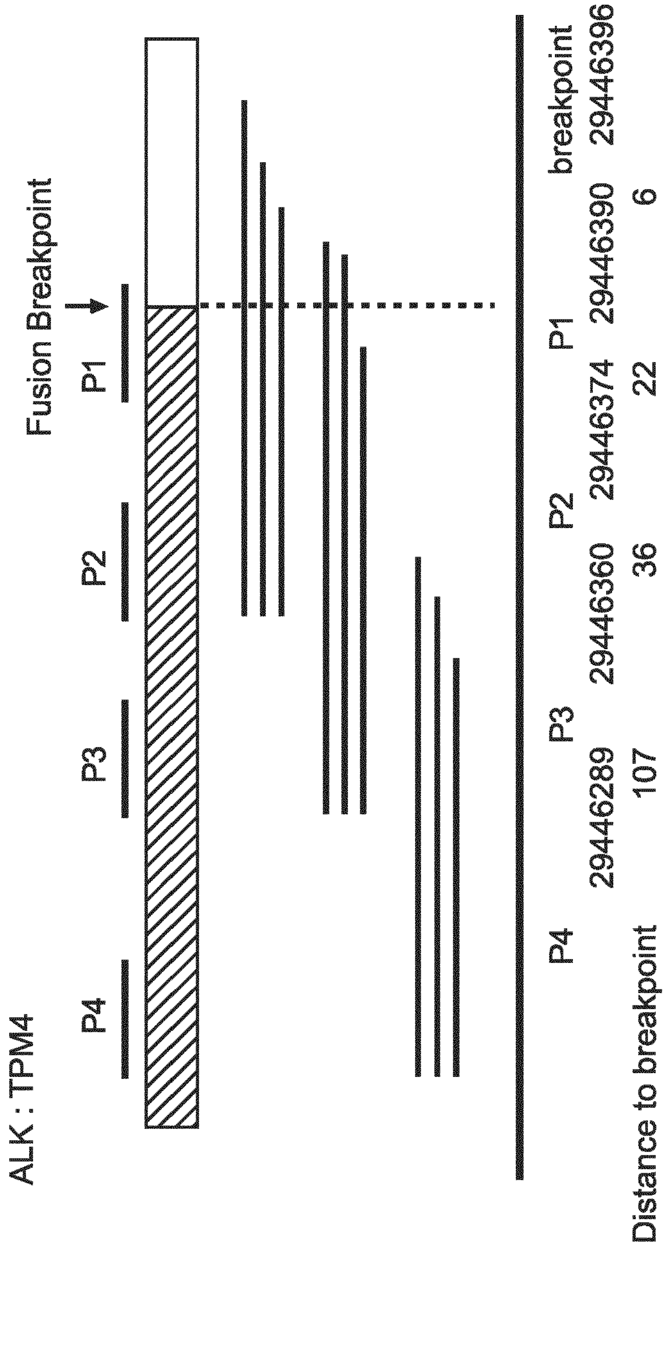
ALK : TPM4
Fusion Breakpoint
P4
P3
P2
P1
P4
P3
P2
P1
breakpoint
29446289
29446360
29446374
29446390
29446396
Distance to breakpoint
107
36
22
6

FIG. 17

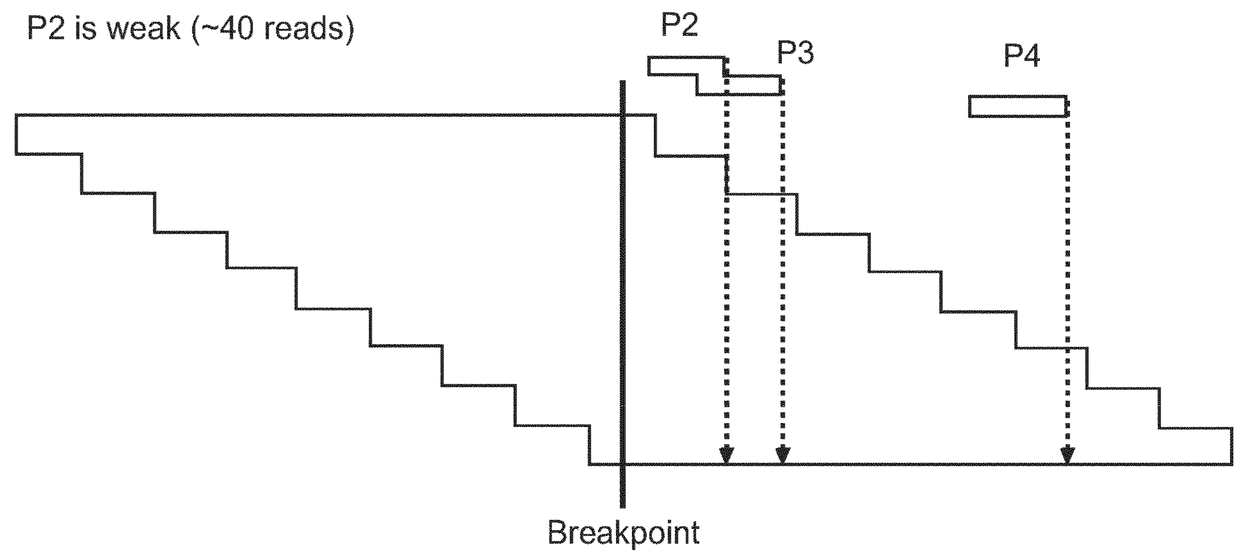


Last seven templates should be amplified/detected for P3
ALK : TPM4 Last three templates should be amplified for P4

FIG. 18A

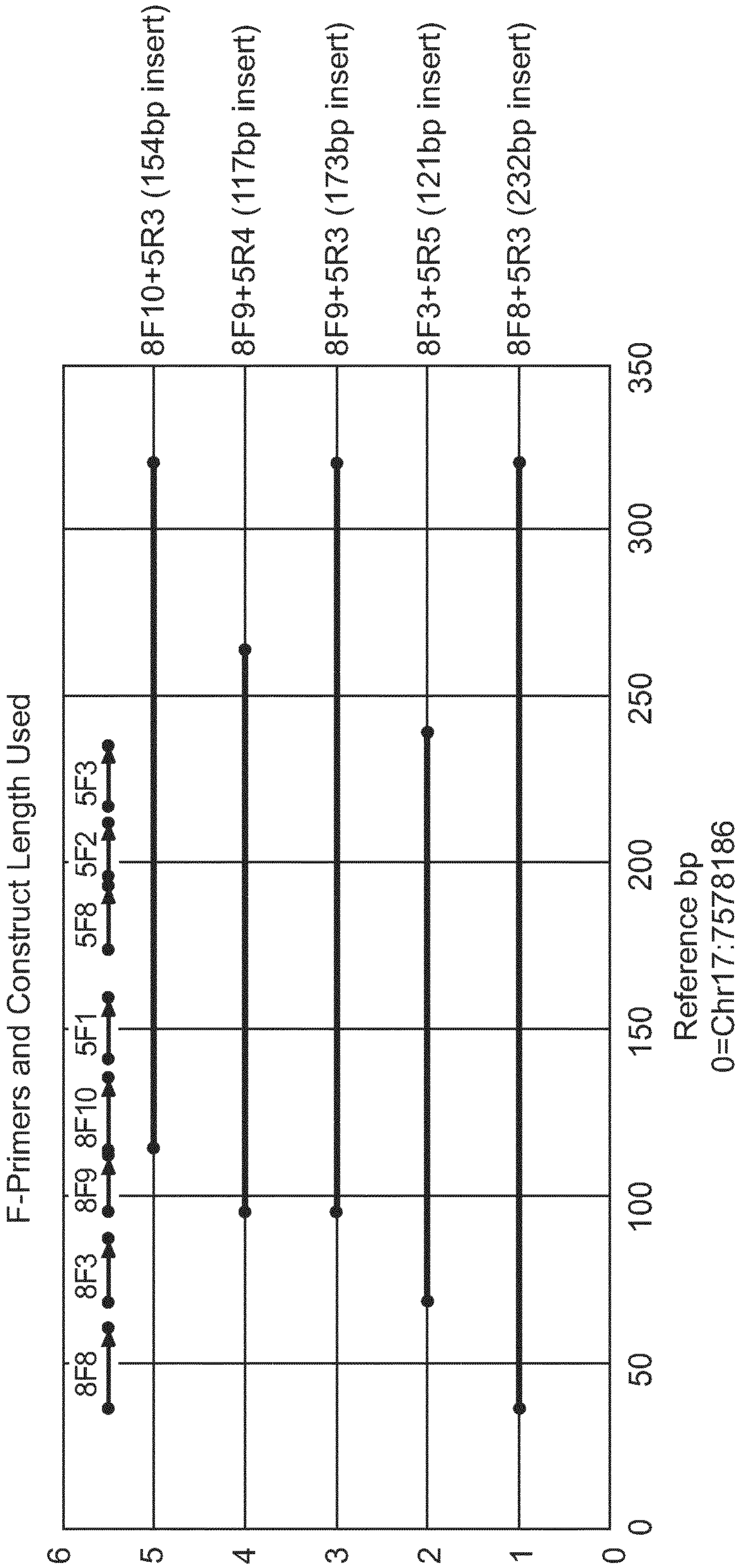
F-Primers and Construct Length Used
8F8
8F3
8F9
8F10
5F1
5F8
5F2
5F3
8F10+5R3 (154bp insert)
8F9+5R4 (117bp insert)
8F9+5R3 (173bp insert)
8F3+5R5 (121bp insert)
8F8+5R3 (232bp insert)
0
1
2
3
4
5
6
0
50
100
150
200
250
300
350
Reference bp
0=Chr17:7578186

FIG. 18B

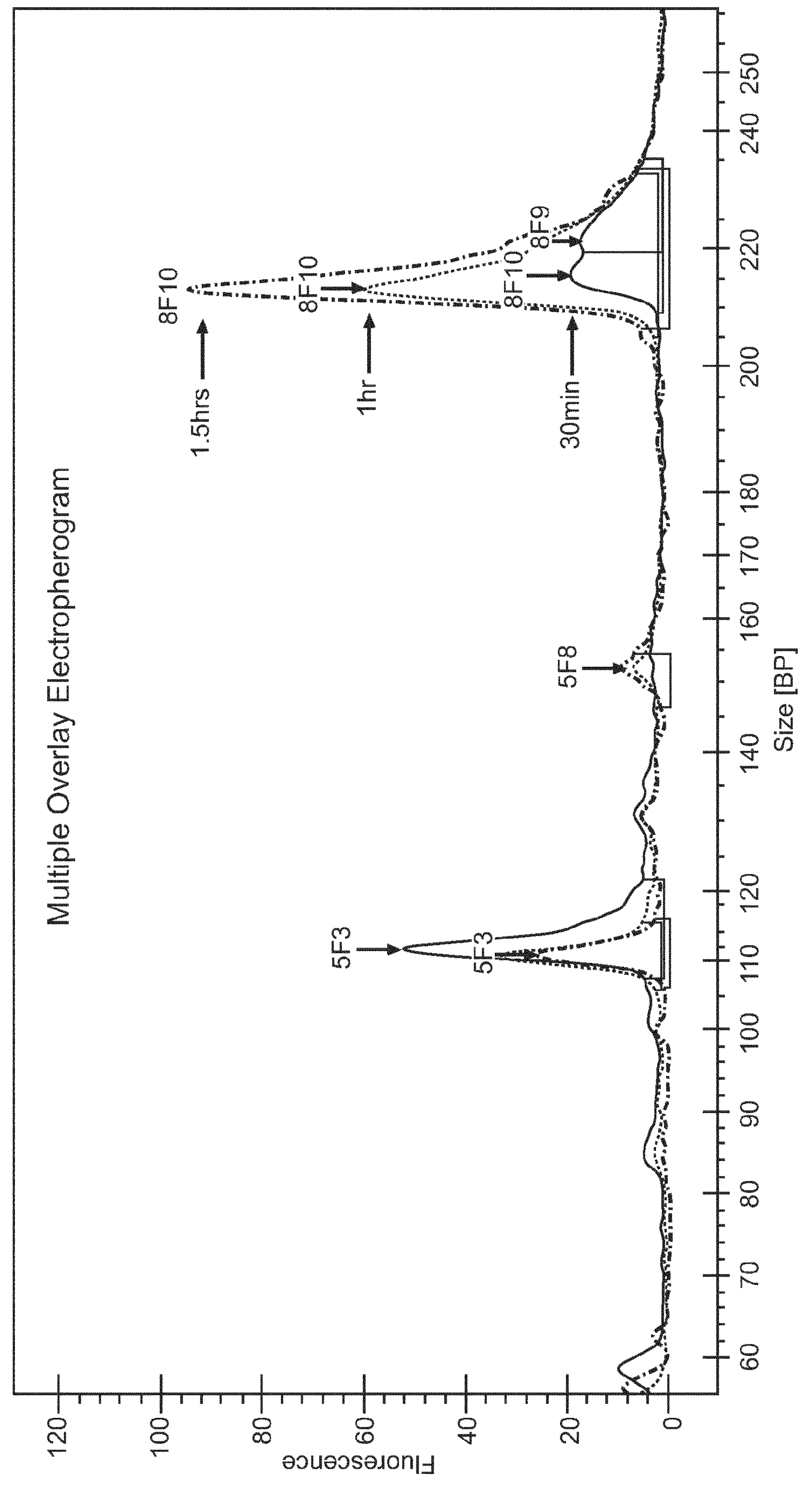
Multiple Overlay Electropherogram
1.5hrs
1hr
30min
8F10
8F10
8F10
8F9
5F3
5F3
5F8
Fluorescence
0
20
40
60
80
100
120
Size [BP]
60
70
80
90
100
110
120
140
160
170
180
200
220
240
250

FIG. 18C

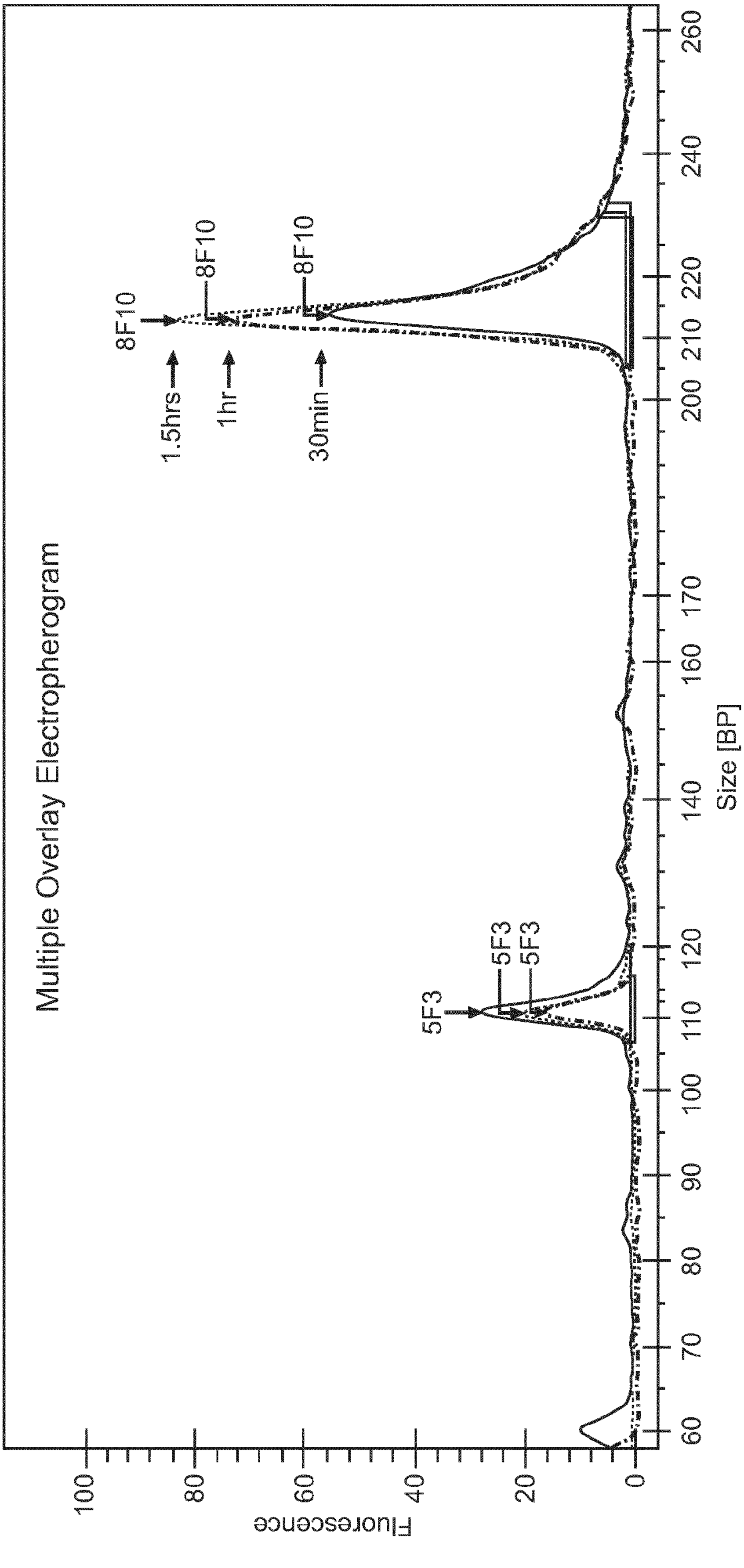
Multiple Overlay Electropherogram
1.5hrs
1hr
30min
8F10
8F10
8F10
5F3
5F3
5F3
Fluorescence
0
20
40
60
80
100
60
70
80
90
100
110
120
140
160
170
200
210
220
240
260
Size [BP]

FIG. 19A

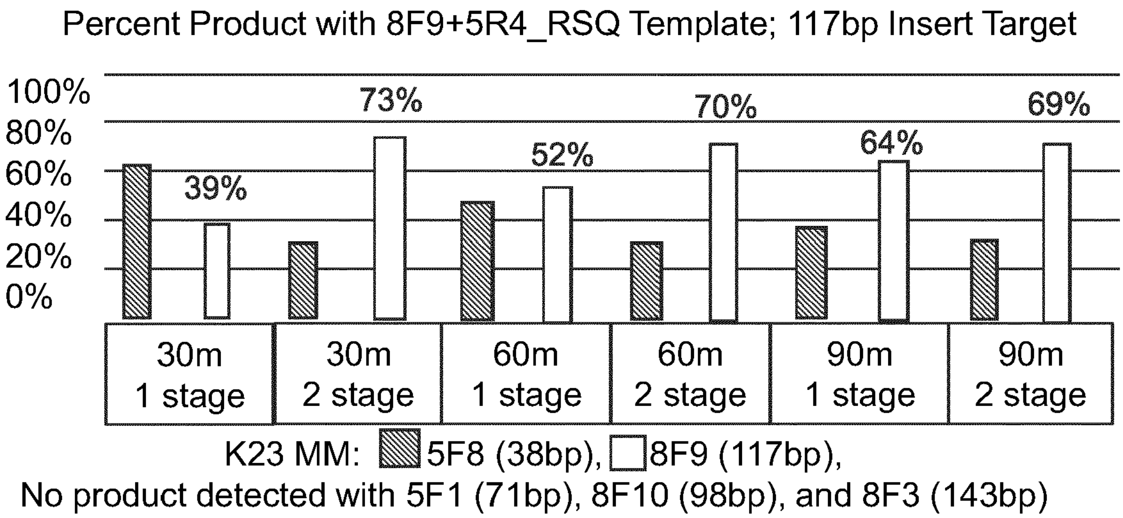


FIG. 19B

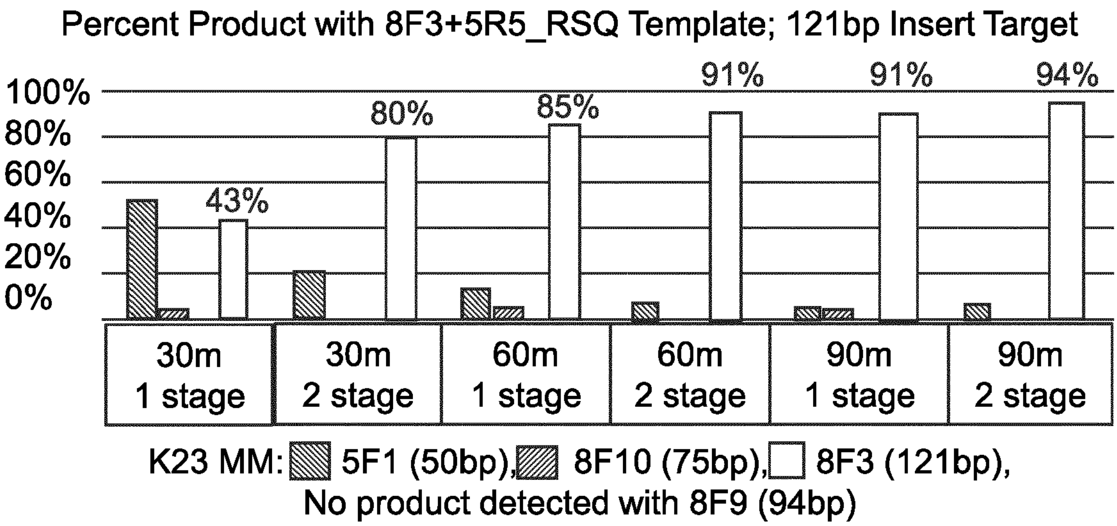

FIG. 19C

Percent Product with 8F3+5R5_RSQ Template; 121bp Insert Target

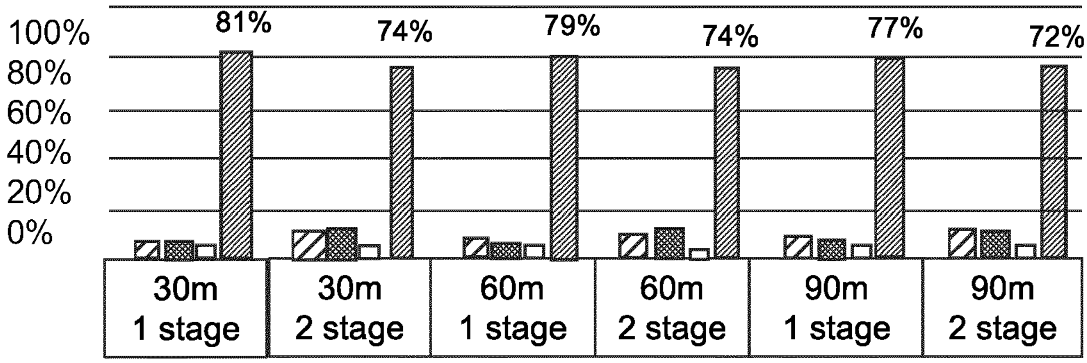


Gold MM: 5F1(50bp), 8F10(75bp), 8F9(94bp), and 8F3(121bp)

FIG. 19D

Percent Product with 8F8+5R3_RSQ Template; 232bp Insert Target

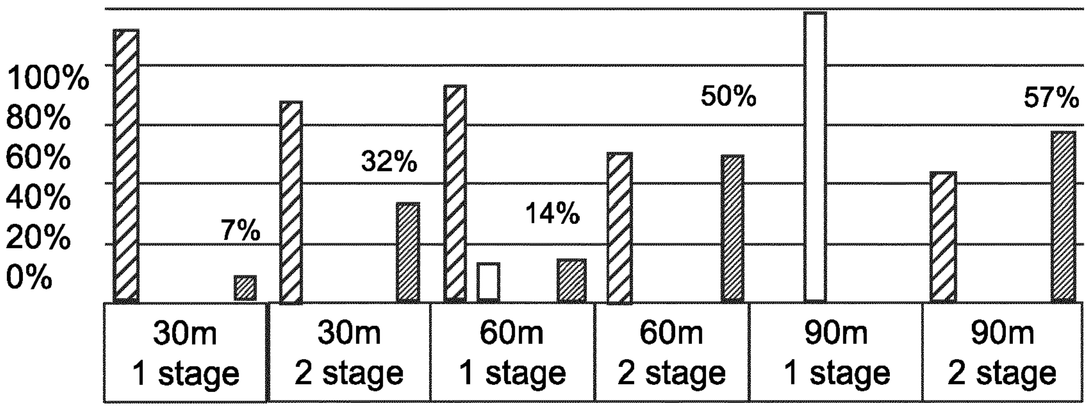


K23 MM: 5F3(51bp), 8F10(75bp), 5F8(94bp), and 8F8(232bp)
No product detected with SF9(72bp), SF1(127bp), 8F10(154bp), 8F9(173bp), and 8F3(200bp).

Europäisches Patentamt
European Patent Office
Office européen des brevets

# EUROPEAN SEARCH REPORT

Application Number
EP 20 20 3880

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2012/108920 A1 (NATERA INC [US]; RABINOWITZ MATTHEW [US] ET AL.) 16 August 2012 (2012-08-16) * p. 5 l. 20-21,p. 95 l. 8 to p. 96 l. 24 and Fig. 6-8 * | 1-15 | INV. C12Q1/68 C12Q1/6827 |
| A | WO 02/090505 A2 (UNIV VIRGINIA COMMONWEALTH [US]; CHEN XIANGNING [US]) 14 November 2002 (2002-11-14) * p. 4 l. 7-15, Fig. 2A, p. 7 and example 5 (p. 26),p. 21 l. 26-30 * | 1-15 | |
| Y | WO 2015/148494 A1 (QUEST DIAGNOSTICS INVEST INC [US]) 1 October 2015 (2015-10-01) * p. 3, 2d para * | 12 | |
| Y | A. NARAYAN ET AL: "Ultrasensitive Measurement of Hotspot Mutations in Tumor DNA in Blood Using Error-Suppressed Multiplexed Deep Sequencing", CANCER RESEARCH, vol. 72, no. 14, 15 July 2012 (2012-07-15), pages 3492-3498, XP055407873, US ISSN: 0008-5472, DOI: 10.1158/0008-5472.CAN-11-4037 | 1 | TECHNICAL FIELDS SEARCHED (IPC) |
| A | * abstract, and fig. 1 * | 2-15 | C12Q |
| X | WO 2013/177220 A1 (SCRIPPS RESEARCH INST [US]; HEAD STEVEN ROBERT [US] ET AL.) 28 November 2013 (2013-11-28) | 2-11, 13-15 | |
| Y | * [0111]-[0117] * | 1,12 | |

The present search report has been drawn up for all claims

1

| Place of search | Date of completion of the search | Examiner |
|---|---|---|
| Munich | 2 February 2021 | Lapopin, Laurence |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 20 3880

This annex lists the patent family members relating to the patent documents cited in the above-mentioned European search report. The members are as contained in the European Patent Office EDP file on
The European Patent Office is in no way liable for these particulars which are merely given for the purpose of information.

02-02-2021

| Patent document cited in search report | Publication date | Patent family member(s) | Publication date |
|---|---|---|---|
| WO 2012108920 A1 | 16-08-2012 | AU 2011358564 A1 | 05-09-2013 |
| | | BR 112013020220 A2 | 09-10-2018 |
| | | CA 2824387 A1 | 16-08-2012 |
| | | CN 103608818 A | 26-02-2014 |
| | | EP 2673729 A1 | 18-12-2013 |
| | | JP 6153874 B2 | 28-06-2017 |
| | | JP 2014507141 A | 27-03-2014 |
| | | RU 2013141237 A | 20-03-2015 |
| | | US 2012270212 A1 | 25-10-2012 |
| | | US 2013178373 A1 | 11-07-2013 |
| | | US 2015051087 A1 | 19-02-2015 |
| | | US 2017242960 A1 | 24-08-2017 |
| | | US 2018025109 A1 | 25-01-2018 |
| | | US 2018201995 A1 | 19-07-2018 |
| | | US 2019203290 A1 | 04-07-2019 |
| | | US 2019211391 A1 | 11-07-2019 |
| | | US 2019211392 A1 | 11-07-2019 |
| | | US 2019211393 A1 | 11-07-2019 |
| | | US 2019249241 A1 | 15-08-2019 |
| | | US 2019256906 A1 | 22-08-2019 |
| | | US 2019256908 A1 | 22-08-2019 |
| | | US 2019256909 A1 | 22-08-2019 |
| | | US 2019360036 A1 | 28-11-2019 |
| | | US 2020181697 A1 | 11-06-2020 |
| | | US 2020190573 A1 | 18-06-2020 |
| | | WO 2012108920 A1 | 16-08-2012 |
| WO 02090505 A2 | 14-11-2002 | AU 2002305436 A1 | 18-11-2002 |
| | | US 2005175996 A1 | 11-08-2005 |
| | | WO 02090505 A2 | 14-11-2002 |
| WO 2015148494 A1 | 01-10-2015 | BR 112016021878 A2 | 24-10-2017 |
| | | CA 2943636 A1 | 01-10-2015 |
| | | CN 106462669 A | 22-02-2017 |
| | | EP 3123380 A1 | 01-02-2017 |
| | | US 2015275277 A1 | 01-10-2015 |
| | | US 2018274040 A1 | 27-09-2018 |
| | | US 2020157641 A1 | 21-05-2020 |
| | | WO 2015148494 A1 | 01-10-2015 |
| WO 2013177220 A1 | 28-11-2013 | AU 2013266394 A1 | 26-02-2015 |
| | | AU 2019204045 A1 | 27-06-2019 |
| | | CA 2874413 A1 | 28-11-2013 |
| | | CN 104736722 A | 24-06-2015 |
| | | CN 109082462 A | 25-12-2018 |
| | | EP 2852687 A1 | 01-04-2015 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO. EP 20 20 3880

This annex lists the patent family members relating to the patent documents cited in the above-mentioned European search report. The members are as contained in the European Patent Office EDP file on
The European Patent Office is in no way liable for these particulars which are merely given for the purpose of information.

02-02-2021

| Patent document cited in search report | Publication date | Patent family member(s) | Publication date |
|---|---|---|---|
| | | EP 3514243 A1 | 24-07-2019 |
| | | US 2015265995 A1 | 24-09-2015 |
| | | US 2019076813 A1 | 14-03-2019 |
| | | WO 2013177220 A1 | 28-11-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62357847
- US 91854415


### Non-patent literature cited in the description


- **KALNINA et al.** *World J Gastroenterol.,* 07 November 2015, vol. 21 (41), 11636-11653
- **JIANG et al.** *Proc Natl Acad Sci USA,* vol. 112, E1317-E1325
- **HAMAKAWA et al.** *Br J Cancer.,* 2015, vol. 112, 352-356
- **UNTERGRASSER A ; CUTCUTACHE I ; KORESSAAR T ; YE J ; FAIRCLOTH BC ; REMM M ; ROZEN SG.** Primer3 - new capabilities and interfaces. *Nucleic Acids Research,* 2012, vol. 40 (15), e115
- **KORESSAAR T ; REMM M.** Enhancements and modifications of primer design program Primer3. *Bioinformatics,* 2007, vol. 23 (10), 1289-91
- **ROBINSON et al.** Integrative Genomics Viewer. *Nature Biotechnology,* 2011, vol. 29, 24-26
- **SUGNET et al.** The human genome browser at UCSC. *Genome Res.,* June 2002, vol. 12 (6), 996-1006
- **LI H. ; DURBIN R.** Fast and accurate long-read alignment with Burrows-Wheeler Transform. *Bioinformatics,* 2010
- **JAMES T. ROBINSON ; HELGA THORVALDSDÓTTIR ; WENDY WINCKLER ; MITCHELL GUTTMAN ; ERIC S. LANDER ; GAD GETZ ; JILL P. MESIROV.** Integrative Genomics Viewer. *Nature Biotechnology,* 2011, vol. 29, 24-26
- **KENT WJ ; SUGNET CW ; FUREY TS ; ROSKIN KM ; PRINGLE TH ; ZAHLER AM ; HAUSSLER D.** The human genome browser at UCSC. *Genome Res.,* June 2002, vol. 12 (6), 996-1006
- **LI et al.** Single nucleotide variation in the TP53 3' untranslated region in diffuse large B-cell lymphoma treated with rituximab-CHOP: a report from the International DLBCL Rituximab-CHOP Consortium Program. *Blood,* 2013, vol. 121 (22), 4529-40